# EUROPEAN PATENT APPLICATION

(11) **EP 4 825 870 A1**
(43) Date of publication of application: **21.10.2026**
(21) Application number: 24904198.9
(22) Date of filing: 09.12.2024
(51) Int. Cl.: C07K 16/00

(54) **CYTOSOL-PENETRATING ANTIBODIES WITH HIGHLY EFFICIENT CYTOSOL PENETRATING ABILITY AND USES THEREOF**

(30) Priority: 14.12.2023 KR 20230181932; 06.12.2024 KR 20240180719
(71) Applicant: AJOU UNIVERSITY INDUSTRY-ACADEMIC COOPERATION FOUNDATION, Gyeonggi-do 16499 (KR)
(72) Inventor: KIM, Yong Sung, Suwon-si, Gyeonggi-do 16504 (KR); KIM, Dae-Seong, Seoul 08346 (KR); BYEON, Jeongseon, Bucheon-si, Gyeonggi-do 14787 (KR); KIM, Seong-Eun, Suwon-si, Gyeonggi-do 16497 (KR)
(74) Representative: Mewburn Ellis LLP
(86) International application number: PCT/KR2024/020015
(87) International publication number: WO 2025/127633

(57) **Abstract**

The present invention relates to: an antibody constant region in which an endosomal escape motif having activity under endosomal slightly acidic conditions is introduced into the constant region of an antibody to impart the ability of an immunoglobulin G (IgG) antibody to actively translocate from outside a cell into the cytoplasm; an antibody comprising same; a method for producing same; and use thereof. More specifically, the present invention provides a high-efficiency cytosol-penetrating antibody (cytotransmab) having an improved endosomal escape ability by introducing an endosomal escape motif into a CH3 domain, which is a heavy chain constant region of an IgG antibody, and/or a CL domain, which is a light chain constant region thereof, and relates to a pharmaceutical composition for the treatment, prevention or diagnosis of cancer, comprising the antibody.

## Description

### Technical Field

The present invention relates to an antibody constant region wherein an endosomal escape motif is introduced into the constant region of an immunoglobulin G (IgG) antibody to confer the ability to actively move from outside a cell into the cytosol to the antibody, an antibody comprising the same, a method for producing the same, and the use thereof. More specifically, the present invention provides a high-efficiency cytosol-penetrating antibody (cytotransmab) with enhanced endosomal escape ability wherein an endosomal escape motif is introduced into the heavy chain constant region CH3 domain of an IgG antibody and/or the light chain constant region CL domain, and relates to a pharmaceutical composition for the treatment, prevention, or diagnosis of cancer comprising the antibody.

### Background Art

Antibodies are immune proteins that bind to antigens and interfere with the action of the antigen or eliminate the antigen. There are five types of antibodies: immunoglobulin (Ig) M, IgD, IgG, IgA, and IgE. A full-length immunoglobulin G (IgG) antibody has a very stable Y-shaped structure (molecular weight, 150 kDa) made up of two heavy chain proteins (each 50 kDa) and two light chain proteins (each 25 kDa). The heavy and light chains of antibodies are divided into variable regions whose amino acid sequences differ between antibodies, and constant regions whose amino acid sequence is the same between antibodies. The heavy chain constant region contains CH1, H (hinge), CH2, and CH3 domains. Each domain consists of two beta sheets (β-sheets) linked by a disulfide bond.

The variable regions of the heavy and light chains combine to form an antigen binding site, which is present on each of the two arms of the Y-shaped structure. This portion that can bind to an antigen is called an antibody binding fragment (Fab), and a portion that cannot bind to an antigen is called a crystallizable fragment (Fc). The Fab and Fc are linked by a flexible hinge region.

Within the heavy chain variable region (VH) and the light chain variable region (VL) of antibodies, there are portions whose amino acid sequences are particularly different between antibodies, which are also called complementarity determining regions (CDRs) because they constitute sites that bind to antigens. Looking at the three-dimensional structure of an antibody, these CDRs form loops on the surface of the antibody, and below the loops, there is a framework region (FR) that provides structural support. There are three loop structures in each of VH and VL, and these six loop structures combine together and come into direct contact with an antigen.

The CH2-CH3 region of the antibody's heavy chain constant region (Fc) contains an FcRn (neonatal Fc receptor) binding site that extends the antibody's serum half-life. Additionally, the hinge-CH2 region contains binding sites for FcR (Fc receptor) and complement, which mediate antibody-dependent cellular cytotoxicity and complement-dependent cellular cytotoxicity.

A number of therapeutic antibodies that target cell membrane proteins or extracellularly secreted proteins due to their property of binding to target proteins with high specificity and high affinity have been developed. All antibodies approved for therapeutic use to date specifically target extracellularly secreted proteins or cell membrane proteins. On the other hand, conventional antibodies are large in molecular size and hydrophilic in nature, so they cannot directly penetrate living cells because they cannot pass through hydrophobic plasma membranes or endosomal membranes composed of lipid bilayers. Therefore, conventional antibodies have limitations in that they cannot target various disease-related proteins in cells.

Antibodies that target cell membrane receptor proteins can enter the cell via the endosomal pathway through receptor-mediated endocytosis after binding to the cell membrane receptor proteins. This process involves several pathways after reaching the early endosome. 1) Most antibodies can move from the early endosome to the late endosome and then to the lysosome, where they can be completely degraded under acidic conditions, and 2) some antibodies can bind to FcRn in the early endosome under acidic conditions and exit the cell via the recycling endosome pathway. As the endosome matures in the endosomal pathway, the endosome lumen is gradually acidified by a proton pump. It is known that the early endosome has a pH of 5.5 to 6.5, the late endosome has a pH of 4.5 to 5.5, and the lysosome has a pH of 3.5 to 4.5. In the acidic environment inside the lysosome, many proteases are activated, and thus proteins internalized from the external environment are degraded inside the lysosome.

Most antibodies that bind strongly to target membrane proteins are degraded via the endosome-lysosome pathway. Therefore, in order for an antibody to localize in the cytosol, the antibody must undergo an endosomal escape process. This process occurs when the antibody moves along the endosomal pathway after receptor-mediated endocytosis, dissociates from the target membrane protein in the early or late endosome before reaching the lysosome, and then escapes from the endosome to the cytosol through endosomal membrane destabilization and collapse.

Some toxins found in nature and cell-penetrating peptides (CPPs) studied to date are known to penetrate living cells, but the mechanism by which these substances penetrate the cytosol has not yet been clearly elucidated. There are three main hypotheses regarding the mechanism by which they penetrate the cytosol.

The first hypothesis is the pore formation model, in which pores are formed in the cell membrane. In this hypothesis, substances such as cationic amphiphilic peptides bind to the negatively charged cell membrane, causing internal stress or membrane contraction, or destabilizing the cell membrane, resulting in the formation of barrel-stave pores or toroidal channels. Through these formed pores, substances within the endosome localize in the cytosol. The barrel-stave pore is the model primarily used to explain the endosomal escape mechanism of amphiphilic α-helix cell-penetrating peptides. The hydrophobic region of the amphiphilic cell-penetrating peptide interacts directly with the hydrophobic hydrocarbon core of the cell membrane, while the hydrophilic region is exposed to the outside to form a cylindrical pore. The toroidal channels are donut-shaped holes formed when cytosol-penetrating substances interact with the hydrophilic head of the cell membrane, structurally distorting the lipid bilayer. The difference between barrel-stave pores and toroidal channels is whether the hydrophobic hydrocarbon core of the cell membrane phospholipid bilayer is exposed, which can vary depending on the composition, secondary structure, and multimer formation of a cell-penetrating peptide.

The second hypothesis is the vesicle budding and collapse model, in which a cell-penetrating peptide binds to the endosomal membrane and then causes the membrane to curve, inducing the formation of small vesicles in the endosome, and the formed vesicles collapse immediately after budding, releasing the substances inside the vesicles into the cytosol. In order for vesicles to be formed in the endosomal membrane, the endosomal membrane must undergo structural deformation. At this time, a cell-penetrating peptide binds to the endosomal membrane and acts to destabilize the endosomal membrane, thereby lowering the energy barrier for vesicle formation. Finally, the budded vesicle is unstable due to its small size and collapses immediately, releasing the substances inside the vesicle into the cytosol.

The third hypothesis is that endosomes rupture due to the proton sponge effect, releasing the substance inside the endosome into the cytosol. Endosomes formed after endocytosis actively transport protons into the endosomal lumen via the V-ATPase proton pump, thereby making the endosomal lumen acidic. Cytosol-penetrating substances with a large buffering effect intercept protons that enter the endosome, preventing acidification of the endosome, and proton pumps transport much more protons into the endosomal lumen to lower the pH. At this time, chloride ions (Cl⁻) also enter the endosomal lumen to maintain the charge balance in the endosome, increasing the ion concentration in the endosome, and water enters to maintain osmotic pressure. As a result, the endosome swells, and this process is repeated to induce endosomal rupture. In this hypothesis, the endosomal escape efficiency is determined by how well the cytosol-penetrating substance can bind to the protons entering the endosome, that is, how effective the cytosol-penetrating substance is at providing the buffering effect. Thus, this hypothesis is mainly explained in non-biological polymers that can bind to many protons, such as cationic polymers, rather than proteins including peptides.

Among the above-described endosomal escape hypotheses, the two hypotheses excluding the proton sponge effect have in common that a cell-penetrating substance first binds to the cell membrane, and the cytosol-penetrating substance should locally concentrate at the cell membrane surface to reach a critical concentration, and at this stage, membrane destabilization is involved. In addition, the above-described endosomal escape hypotheses are based on the premise that the endosomal escape process involves changes in the internal environment and in the endosomal membrane, and that a cytosol-penetrating substance lowers the high energy barrier required in the process. Therefore, the endosomal escape efficiency of the cytosol-penetrating substance depends on its ability to efficiently lower this energy barrier.

Various methods have been attempted to deliver antibodies into the cytosol or nucleus of living cells. One method involves creating IgG-CPP or antibody fragment-CPP fusion proteins by linking a cell-penetrating peptide (CPP) to a full-length IgG antibody or a fragment thereof such as scFv (single chain variable fragment) by genetic fusion or chemical conjugation (CPP) (Bioconjugate Chem. 2019, 30:4:1028-1041).

Representative CPPs such as TAT, Penetratin, and a polyarginine peptide have been reported to be able to pass directly through the plasma membrane or localize in the cytosol via the endosomal escape pathway, and deliver biomolecules such as proteins and DNA into the cell. For antibody-CPP fusion proteins in which the CPP is fused to an antibody by genetic or chemical methods, there is a technique of directly fusing TAT or polyarginine to an antibody, or linking the CPP to protein A or protein G, which binds to the Fc region of an antibody with high affinity, and fusing the same to the antibody. Another example is a technique of conferring pH-dependent cell-penetrating ability to a membrane-lytic spider venom peptide (M-lycotoxin) and then treating a cell with the peptide together with an antibody to deliver the antibody into the cell (US Patent No. US 11179471B2).

However, the technique of directly fusing the CPP to an antibody has a problem in that the linker between the protein and the CPP can affect the cytosol-penetrating ability of the CPP. In addition, problems with the CPP itself are a limitation of the above-described antibody-CPP fusion protein technologies. Because the CPP has low stability and a short *in vivo* half-life and lacks cell selectivity, it has potential toxicity issues due to non-specific cell penetration. In the case of virus-derived or synthetic CPPs, there is also a potential immunogenicity issue. Additionally, there is a limitation in that a significant number of CPPs are unable to escape from the endosome after endocytosis and remain trapped inside the endosome (endosomal entrapment), and thus only about 0.1 to 2% of the endocytosed peptides localize in the cytosol (Nat Commun. 2021, 12(1):3721).

Another method involves antibody-nanoparticle complexes in which an antibody is entangled in polymer nanoparticles (NPs) or an antibody is incorporated into polymer nanoparticles. For example, there is a technique of incorporating an antibody into mesoporous silica nanoparticles (MSNs) and delivering the same into cells (Nanotechnology, 2012, 23(8):085101). Although the antibody-nanoparticle complexes are easy to synthesize and chemically optimize, they have low cytosolic delivery efficiency due to the phenomenon in which they are mostly trapped in the endosome (endosomal entrapment) after internalization, similar to cell-penetrating peptides. In addition, there is a limitation in that when an antibody is directly bound to the nanoparticles, the antibody function may be hindered because the antibody remains bound to nanoparticle residue even after being released into the cytosol.

In addition, there are antibody-liposome complexes that deliver antibodies into living cells using liposomes (BioTechniques, 2008, 44(7S):vii-xi). Liposomes have the advantages of excellent biocompatibility and biodegradability, but they have the disadvantages of having low efficiency in accommodating water-soluble molecules in the liposome lumen. In addition, because they are often positively charged, they have the disadvantage of non-specifically binding to the cell surface, like CPPs, resulting in low specificity for target cells. There is also a problem in that the half-life is short because they can be removed by macrophages, etc. through opsonization, which is the adsorption of other serum proteins in the blood.

Another method reported is an antibody-nucleotide fusion protein in which phosphorothioated DNA oligonucleotides are covalently fused to an antibody (JCI Insight, 2019, 25;4(14):e127474). Unlike DNA nucleic acids linked by a typical phosphodiester covalent bond, phosphorothioated DNA oligonucleotides are linked by a phosphorothioate covalent bond in which sulfur is located at the oxygen site. Although the exact mechanism by which the antibody-phosphorothioated DNA oligonucleotide fusion protein penetrates the cytosol is uncertain, it is presumed that the phosphorothioated DNA oligonucleotide portion binds to the lipid bilayer of the endosome to induce endosomal escape.

It has been reported that an anti-DNA antibody derived from a patient with an autoimmune disease exhibits the ability to penetrate the cytosol in full-length IgG form, without an external portion that confers cell-penetrating ability. In particular, a mouse IgG anti-DNA 3D8 scFv antibody has been reported to be able to penetrate the cytosol by interacting with the endosomal lipid bilayer, as a specific sequence of the light chain variable region (VL) is internalized into a cell by binding to heparan sulfate proteoglycans on the cell surface, and amino acid residues in the light chain variable region (VL) CDR1 and CDR3 act as endosomal escape motifs in the endosomal environment (European Patent No. EP 2279471B2, Cellular and Molecular Life Sciences, 2009, 66(11-12):1985-97). In addition, it has been reported that a humanized antibody generated by transferring the endosomal escape motif of the light chain variable region (VL) of the mouse antibody to the heavy chain variable region (VH) and light chain variable region (VL) of a human antibody exhibits cytosolic-penetrating ability in full-length IgG form (Korean Patent No. KR 10-1602870; mAbs, 2014, 6(6):1402-1414; Biochem Biophys Res Commun, 2015, 467(4):771-777). Such an antibody having, in the light chain variable region (VL) and/or heavy chain variable region (VH), an endosomal escape motif that induces cytosol-penetrating ability in a full-length IgG antibody, has been reported as a cytosol-penetrating antibody (cytotransmab).

It has been reported that a cytosol-penetrating antibody produced by fusing a cyclic peptide that binds to integrin αvβ3/αvβ5 or EpCAM (epithelial cell adhesion molecule) receptor, which is overexpressed on the surface of tumor cells, or a monobody that binds to EGFR (epidermal growth factor receptor), to the N-terminus of the light chain of the cytotransmab antibody that exhibits cytosol-penetrating ability in the form of IgG, exhibits target cell-specific cytosol-penetrating ability (Biochem Biophys Res Commun, 2018, 503:2510-2516; Biochem Biophys Res Commun, 2021, 573:35-41; Korean Patent Application No. 10-2023-0181933). The above antibody has the ability to penetrate the cytosol in a tumor cell-specific manner through a mechanism by which it binds to integrin αvβ3/αvβ5 or a receptor (such as EpCAM or EGFR) overexpressed on tumor cells, enters the cell through receptor-mediated endocytosis, and then dissociates from the tumor cell-overexpressed receptor in an endosome-specific environment (acidic or reducing conditions), and escapes from the endosome (endosomal escape). The integrin αvβ3/αvβ5-specific cytosol-penetrating antibody was named inCT (Science Advances, 2020, 6(3):eaay2174), the EpCAM-specific cytosol-penetrating antibody was named epCT (Biochem Biophys Res Commun, 2018, 503:2510-2516), and the EGFR-specific cytosol-penetrating antibody was named ERCT (Korean Patent Application No. 10-2023-08181933) (Biochem Biophys Res Commun, 2018, 503:2510-2516). The inCT cytosol-penetrating antibody has, as endosomal escape motifs, ⁹²WYW⁹⁴ (where the amino acid residue numbers follow the Kabat numbering scheme) in the CDR3 region of the VL and ⁹⁶WYW⁹⁸ (the Kabat numbering scheme) in the CDR3 region of the VH. The inCT (AAA) antibody, in which all of the above endosomal escape motifs WYW were substituted with alanine (Ala), did not show cytosol-penetrating ability. The inCT(AAA) antibody obtained by completely replacing the WYW endosomal escape motifs with alanine (Ala) did not show cytosol-penetrating ability (Science Advances, 2020, 6(3):eaay2174; Biochem Biophys Res Commun, 2018, 503:2510-2516). The cytosol-penetrating antibody (cytotransmab) was found to have a pore formation mechanism in which aromatic residues of the antibody's CDR3 region are exposed to the surface through pH-dependent structural changes in the early endosome, and thus interact with the endosomal membrane, and toroidal pores are formed due to the destabilization of the endosomal membrane (Journal of Controlled Release, 2016, 235:165-175).

Cell/tissue-specific cytosol-penetrating interfering antibody (iMab) technology has been reported as an antibody technology for directly targeting a target protein in the cytosol from outside the cell based on the above-described cytosol-penetrating antibody (Korean Patent Nos. KR10-1602870, KR10-2000000, KR10-2091195, and KR10-2163305; Nature Communications, 2017. 8:15090; Science Advances, 2020, 6(3):eaay2174). Cell-penetrating interfering antibodies are in a form in which the VH in full-length IgG form targets a target protein in the cytosol, the VL has endosomal escape ability, and a cyclic peptide specific to integrin αvβ3/αvβ5 is fused to the N-terminus of the VL.

Cell-penetrating interfering antibodies (iMabs) are in full-length immunoglobulin form. They can localize in the cytosol through endosomal escape ability after being endocytosed by specifically binding to cell surface membrane protein receptors that are overexpressed in tumor cells or tissues, and have the ability to bind to target proteins in the cytosol. For example, a cell-penetrating interfering antibody has been reported that specifically binds to activated RAS (RAS·GTP) bound to GTP in the cytosol and inhibits the activity of tumor-derived variant RAS (Korean Patent No. KR10-2163305; Science Advances, 2020, 6(3):eaay2174; Nature Communications, 2017, 8:15090).

As described above, various derivative technologies based on cytosol-penetrating antibody technology have been studied. However, existing cytosol-penetrating antibodies (cytotransmab) have endosomal escape motifs in only the CDR3 of the light chain variable region (VL) and heavy chain variable region (VH), and thus there is a limitation in that the amount of antibody reaching the cytosol is insufficient due to the low endosomal escape efficiency (about 13%). In addition, when these cytosol-penetrating antibodies were applied as cell-penetrating interfering antibodies, they did not show a potent effect because the amount reaching the cytosol was small. Furthermore, since existing cytosol-penetrating antibodies have endosomal escape motifs in the CDR3 of the light chain variable region (VL) and heavy chain variable region (VH), they have the limitation of being less versatile because they should utilize only one of the two regions, VH or VL, to have the ability to target proteins in the cytosol.

Variable domains (VH and VL) and constant domains (CL, CH1, CH2, and CH3) that make up an antibody belong to the immunoglobulin domain. The immunoglobulin domain is a type of protein domain in the form of a two-layer sandwich consisting of antiparallel β-sheets made up of 7 to 9 β-strands. An important feature of the immunoglobulin domain is that when appropriate mutations are introduced into the loop region connecting β-strands or the amino acid length changes, the overall structure and function are maintained without being significantly damaged. In addition, among proteins that have an immunoglobulin domain, there are many that are involved in protein-protein interactions or protein-ligand interactions.

The most representative examples are the CDRs of the variable region (VH and VL) of an antibody. The variable region of an antibody consists of a total of 9 β-strands, which are called A, B, C, C', C", D, E, F, and G strands, respectively, and there are AB, BC, CC', C'C", C"D, DE, EF, and FG loops connecting the β-strands. Among these loops, the BC, C'C", and FG loops contain CDR1, 2, and 3, respectively. CDRs have very high sequence and length variability, and thus are able to bind to numerous target proteins.

The constant region of an antibody consists of a total of 7 β-strands, which are called A, B, C, D, E, F, and G strands, respectively, and there are AB, BC, CD, DE, EF, and FG loops connecting the β-strands. Because the constant region of an antibody shows a high structural similarity to the variable region, the loops of the constant region of the antibody can also be conferred the ability to bind to target proteins, like the CDRs.

For example, there is an Fcab (Fc fragment with antigen-binding) improved to have the ability to bind to an antigen such as HER2 (human epidermal growth factor receptor 2) or VEGF (vascular endothelial growth factor receptor), in the AB and EF loops of the heavy chain constant region CH3 domain of (Protein Eng Des Sel, 2017, 30(9):567-581). Through Fcab technology, which confers target protein-binding ability to the AB and EF loops of the antibody's constant region, it can be confirmed that the AB and EF loops of the antibody's constant region are regions capable of withstanding introduction of various mutations and length extension, i.e., are engineerable regions.

The domains that make up antibodies are all immunoglobulin domains and are structurally very similar to each other. In particular, the three-dimensional structure of the CL domain is very similar to the three-dimensional structure of the CH3 domain, with a root mean square deviation (RMSD) value of 1.12 Å. Therefore, by introducing mutations introduced into the CH3 domain into structurally equivalent positions in the CL domain, the function conferred to the CH3 domain can be conferred to the CL domain as well, thereby enhancing the function.

In addition, the CH1-CL pair is very similar to the CH3-CH3 pair in overall shape as well as at the single domain level, and the RMSD value of the three-dimensional structure of the CH1-CL pair for the three-dimensional structure of the CH3-CH3 pair is about 1.612 Å. At this time, the CH3-CH3 pair is structurally more stable because the interacting interface area of the CH1-CL pair is 947.2 Å, whereas that of the CH3-CH3 pair is 1,127.4 Å, which is wider. Based on this, there is a FabCH3 technology in which the CH1-CL pair is replaced with the CH3-CH3 pair, and the FabCH3 antibody has improved thermal stability while maintaining the ability to target proteins through VH and VL.

There is also a FabCab technology that is a fusion of Fcab technology and FabCH3 technology. Specifically, there is a bispecific antibody that simultaneously targets HER2 and VEGF, obtained by replacing the CH1-CL pair in an IgG antibody that targets HER2 through VH and VL domains with a CH3-CH3 pair that targets VEGF (Biochem Biophys Rep, 2021, 2021;26:100959). Through the FabCab technology, desired functions can be added by replacing the CH1-CL pair of an IgG antibody with a CH3-CH3 pair that has a separate function. In the case of the FabCab technology, the CH3 domain that replaced the CH1 and CL domains may combine with the CH3 domain of the heavy chain constant region (Fc) of the original antibody to form an undesirable oligomer. To prevent this, heterodimeric Fc technology can be applied to the CH3-CH3 pair that replaces the CH1-CL pair.

The heterodimeric Fc technology was introduced for the development of bispecific antibodies. This technology involves inducing mutations in the CH3 domains of two different Ig heavy chains through genetic engineering, causing the two Ig heavy chains to form a heterodimer. Antibodies found in nature exist as homodimers, which are assembled from two heavy chains with the same amino acid sequence and two light chains with the same sequence. Formation of this homodimer is induced through the interaction between the last domains of the antibody's constant regions (Fc) (CH3 domains in the case of IgG), and then the formation of a disulfide bond between the hinge regions is induced, forming a homodimer between the heavy chains. At this time, through the heterodimeric Fc technology, mutations can be introduced into amino acids that contribute to the formation of a homodimer between the CH3 domains, thereby promoting heterodimer formation rather than homodimer formation. For example, there is EW-RVT technology that promotes heterodimeric Fc pair protein formation by introducing K360E and K409W mutations into the CH3-A domain in the CH3 domain of a human IgG1 antibody and E347R, D399V, and F405T mutations into the CH3-B domain (Korean Patent No. KR10-1522954; amino acid positions follow the EU numbering system). When the CH1-CL pair is replaced with the CH3 (heterodimeric CH3) pair into which the EW-RVT mutations have been introduced, the formation of a heterodimer between the light chain and the heavy chain may be enhanced, allowing for the expression of a stable FabCab antibody.

Toxin derived from diphtheria or *Pseudomonas* (DT toxin or PE toxin) is known to inhibit protein synthesis by inactivating eEF2 in the cytoplasm and to exhibit potent cytotoxicity capable of killing cells even with a single molecule. Due to these characteristics, various studies have been conducted to develop bacterial toxins into therapeutic proteins. However, when the toxin is expressed in mammalian cells, a problem arises in that the host cells die during the expression process. Due to this problem, expressing a recombinant protein fused to the toxin in mammalian cells remains a major challenge.

Split intein technology can be utilized as a strategy to address this problem (PNAS, 2019, 166(44):22164-22172). A split intein is an intein that is transcribed and translated by two independent genes to perform trans-splicing, wherein the trans-splicing occurs when N-intein (intein(N), fused to the C-terminus of N-extein) and C-intein (intein(C), fused to the N-terminus of C-extein) are co-expressed. The two split intein fragments combine to restore activation and promote the linkage between N-extein and C-extein. This process does not require enzymes or cofactors, and is sufficient with a structure in which the expressed protein folds properly.

Through this technology, an antibody fused with N-intein can be expressed in mammalian cells, and a toxin fused with C-intein can be expressed in *E. coli*, and then the two fragments can be mixed together *in vitro* to purify the antibody fused with the toxin.

Under this technical background, the inventors of the present application have sought to develop a high-efficiency cytosol-penetrating antibody by introducing an endosomal escape motif, different from existing cytosol-penetrating antibodies, into the constant region of an IgG antibody. Specifically, previously reported cytosol-penetrating antibodies had endosomal escape motifs in the VL and VH regions, and showed low endosomal escape ability. By introducing an endosomal escape motif, different from these cytosol-penetrating antibodies, into the heavy chain constant region and/or light chain constant region of an IgG antibody, the inventors have designed, produced, and evaluated an antibody with high endosomal escape ability, thereby developing a completely new high-efficiency cytosol-penetrating antibody with high endosomal escape ability.

Specifically, the inventors of the present application have developed a novel high-efficiency IgG cytosol-penetrating antibody (cytotransmab) by producing an IgG antibody wherein an endosomal escape motif is introduced into the heavy chain constant region CH3 domain of the IgG antibody. That is, it has been quantitatively confirmed that the developed cytosol-penetrating antibody has improved cytosol-penetrating ability compared to existing cytosol-penetrating antibodies such as inCT and epCT65, which have the endosomal escape motifs in the existing VH and VL regions.

In addition, the inventors of the present application have developed a novel IgG cytosol-penetrating antibody by producing an IgG antibody wherein an endosomal escape motif is introduced into the light chain constant region CL domain of the IgG antibody.

In addition, the inventors of the present application have developed a novel high-efficiency IgG cytosol-penetrating antibody by producing an IgG antibody wherein an endosomal escape motif is introduced into the CH3 domain and the CL domain, which are constant regions.

Furthermore, the inventors of the present application have developed a novel high-efficiency IgG cytosol-penetrating antibody by producing an IgG antibody in which the CL-CH1 pair is replaced with a CH3-CH3 pair having an endosomal escape motif.

By identifying and evaluating the cytosol-penetrating ability and biophysical properties of the above-described high-efficiency IgG cytosol-penetrating antibodies, the inventors have developed a novel cytosol-penetrating antibody (cytotransmab) with high endosomal escape ability, which has the potential to be developed into a therapeutic antibody.

In addition, the inventors of the present application have found that the high-efficiency cytosol-penetrating antibody, in which an endosomal escape motif is introduced into the constant region of the IgG antibody, has no significant difference in the antibody's intrinsic FcRn-binding characteristics compared to a wild-type IgG antibody.

In addition, the inventors of the present application have found that, when an IgG antibody is produced by inserting a VH, which targets α-tubulin or GTP-bound activated RAS, into the heavy chain variable region (VH) of the novel high-efficiency cytosol-penetrating antibody, an anti-α-tubulin cytosol-penetrating antibody or an anti-RAS cytosol-penetrating antibody is produced, which can actively penetrate the cytosol without cell permeabilization with detergents or the like and more effectively target α-tubulin or activated RAS in the cytosol than existing cytosol-penetrating antibodies.

In addition, the inventors of the present invention have found that, by fusing a DT toxin to the novel high-efficiency cytosol-penetrating antibody using split intein technology, an immunotoxin based on the cytosol-penetrating antibody is produced, which can actively penetrate the cytosol without treatment with detergents or the like and effectively kill target cancer cells both *in vitro* and *in vivo*. Thereby, the inventors have demonstrated the versatility and scalability of the technology, thereby completing the present invention.

The above information disclosed in this Background section is only for enhancement of understanding of the background of the present invention. Therefore, it may not contain information that forms a conventional art that is already known in the art to which the present invention pertains.

### Summary of Invention

To overcome the problem that existing IgG cytosol-penetrating antibodies have low endosomal escape efficiency because they have an endosomal escape motif in only the CDR3 of the light chain variable region (VL) and heavy chain variable region (VH), the inventors of the present application have sought to develop an antibody constant domain produced by introducing a new endosomal escape motif into the constant region of an antibody, and a high-efficiency cytosol-penetrating antibody comprising the same.

An object of the present invention is to provide a cytosol-penetrating antibody or a fragment thereof comprising an endosomal escape motif in a constant region.

Another object of the present invention is to provide a polynucleotide encoding the cytosol-penetrating antibody or fragment thereof.

Still another object of the present invention is to provide a recombinant expression vector comprising the polynucleotide.

Yet another object of the present invention is to provide an isolated host cell comprising the recombinant expression vector.

Still yet object of the present invention is to provide a composition for delivering an active substance into cytosol, comprising the cytosol-penetrating antibody or fragment thereof.

### Technical Solution

The present invention provides an antibody constant domain having cytosol-penetrating ability produced by introducing an endosomal escape motif that induces endosomal escape into the heavy chain constant region CH3 domain of an IgG antibody and/or the light chain constant region CL domain, a high-efficiency cytosol-penetrating antibody comprising the same, and a method for producing the same.

The present invention relates to a cytosol-penetrating antibody or a fragment thereof comprising an endosomal escape motif in the heavy chain constant region and/or the light chain constant region.

The present invention relates to a polynucleotide encoding the cytosol-penetrating antibody or fragment thereof.

The present invention relates to a recombinant expression vector comprising the polynucleotide.

The present invention relates to an isolated host cell comprising the recombinant expression vector.

The present invention relates to a composition for delivering an active substance into cytosol comprising the cytosol-penetrating antibody or a fragment thereof.

The present invention relates to a high-efficiency cytosol-penetrating antibody or an Fc fragment thereof wherein an endosomal escape motif is introduced into a specific region of the heavy chain constant region CH3 domain of an IgG antibody through amino acid mutation so as to cause the IgG antibody internalized into a cell to induce endosomal membrane destabilization and endosomal membrane collapse by interaction with lipid bilayer endosomal membranes in response to changes in the weakly acidic environment of the endosome, causing the antibody to escape from the endosome into the cytosol, and a method for producing the same.

The present invention also relates to a high-efficiency cytosol-penetrating antibody or an Fab fragment thereof wherein an endosomal escape motif is introduced into a specific region of the light chain constant region CL domain of an IgG antibody through amino acid mutation so as to induce endosomal membrane destabilization and endosomal membrane collapse by interaction with lipid bilayer endosomal membranes in response to changes in the weakly acidic environment of the endosome, thereby causing the antibody to escape from the endosome into the cytosol, and a method for producing the same.

The present invention also relates to a high-efficiency cytosol-penetrating antibody having the above-described endosomal escape motif in both the heavy chain constant region CH3 domain and the light chain constant region CL domain, and a method for producing the same.

The present invention also relates to an antibody wherein the CL-CH1 pair position of an IgG antibody is replaced with a heterodimeric CH3 pair having the above-described endosomal escape motif, and a high-efficiency cytosol-penetrating antibody comprising the same.

The present invention also relates to an endosomal escape motif obtained through amino acid substitutions at specific positions or combinations thereof, which are used to improve an IgG antibody comprising a heavy chain constant region CH3 domain and/or light chain constant region CL domain having an endosomal escape motif, maintain the inherent physicochemical properties of the antibody, and eliminate the non-specific binding of the antibody.

The present invention relates to a specific amino acid composition and position for providing an endosomal escape motif to the CH3 domain and/or CL domain, which is the constant region of an antibody.

The present invention relates to a cytosol-penetrating antibody or a Fab fragment thereof with high-efficiency endosomal escape ability wherein the CL-CH1 pair of a conventional IgG antibody is replaced with a heterodimeric CH3-CH3 pair containing the endosomal escape motif, and a method for producing the same.

The present invention relates to a specific amino acid composition and position for replacing a CL-CH1 pair of an antibody with a heterodimeric CH3-CH3 pair having the endosomal escape motif.

In addition, the present invention relates to an antibody-drug conjugate, or a composition for delivering an active substance into cytosol, which comprises the above-described high-efficiency cytosol-penetrating antibody or Fc fragment or Fab fragment thereof. Specifically, the present invention relates to cytosol-penetrating antibody fusion proteins wherein various payloads (e.g., proteins, fusion proteins, enzymes, toxins, siRNA, small molecule compounds, peptides, DNA, mRNA, etc.) are fused to the high-efficiency cytosol-penetrating antibody and which may deliver the loaded payloads into the cytosol very efficiently and exhibit the actions and effects of the payloads.

In addition, the present invention relates to a cytosol-penetrating interfering antibody with endosomal escape ability, which has the ability to target a target protein in the cytosol in the CDRs of the heavy chain variable region (VH) and light chain variable region (VL) of the high-efficiency cytosol-penetrating antibody and thus has the property of modulating the activity of the target protein in the cytosol, and a method for producing the same.

In addition, the present invention provides polynucleotide and amino acid sequences encoding all of the above-described cytosol-penetrating antibodies or Fc fragments thereof.

### Brief Description of Drawings

FIG. 1 is a schematic diagram showing cytosol-penetrating antibody in full-length IgG form having an endosomal escape motif in the CH3 domain.
FIG. 2 shows the results of modeling the three-dimensional structures of the Fc regions of CH3 domain variants in full-length IgG form having an endosomal escape motif using AlphaFold2 and the results of analyzing the intramolecular interaction patterns of the antibodies using the PyMOL molecular visualizer.
FIG. 3 is a schematic diagram showing the expected endosomal escape mechanism of a cytosol-penetrating antibody in full-length IgG form having an endosomal escape motif in the constant region CH3 domain of the antibody.
FIG. 4 is a schematic diagram illustrating the process in which GFP fluorescence generated by complementation of enhanced split green fluorescent protein is observed when a cytosol-penetrating antibody localizes in the cytosol.
FIG. 5 shows the results of analyzing the CH3 domain variants of FIG. 2 by 12% SDS-PAGE under reducing or non-reducing conditions after purification and the results of analyzing the domain variants by size exclusion chromatography.
FIG. 6 shows the results of evaluating the non-specific binding of the CH3 domain variants of FIG. 2 at concentrations of 100 nM and 500 nM by ELISA.
FIG. 7 shows the results of evaluating the non-specific binding of the CH3 domain variants of FIG. 2 at concentrations of 500 nM and 1 µM to cell membranes by flow cytometry.
FIG. 8 shows the results of evaluating, through Western blot analysis, the expression levels of SA-GFP1-10 reporter protein in cell lines constructed by transfecting SA-GFP1-10 reporter protein into MDA-MB-231 and SW480 cells, which belong to cell lines expressing integrin αvβ3 and αvβ5, in order to evaluate the cytosol-penetrating ability of the CH3 domain variants of FIG. 2 through an enhanced split green fluorescent protein complementation system.
FIGS. 9a and 9b show the results of evaluating the cytosol-penetrating ability of the CH3 domain variants of FIG. 2 at a concentration of 500 nM in MDA-MB-231 and SW480 cell lines by live-cell imaging through an enhanced split green fluorescent protein complementation system.
FIG. 10 shows the results of evaluating the concentration-dependent cytosol-penetrating ability of the CH3 domain variants of FIG. 2 in MDA-MB-231 cells by live-cell imaging through an enhanced split green fluorescent protein complementation system. FIG. 11 shows the results of evaluating the pH-dependent interaction of the CH3 domain variants of FIG. 2 with the cell membrane in Ramos cells, an endosomal membrane model cell line, in order to indirectly confirm the endosomal escape mechanism of the CH3 domain variants.
FIG. 12 is a schematic diagram showing a cytosol-penetrating antibody in full-length IgG form having an endosomal escape motif in the CL domain.
FIG. 13 shows the results of modeling the three-dimensional structures of Fab of CL domain variants in full-length IgG form CL domain having an endosomal escape motif using AlphaFold2 and analyzing the intramolecular interaction patterns of the antibodies using the PyMOL molecular visualizer.
FIG. 14 shows the results of analyzing the CL domain variants of FIG. 13 by 12% SDS-PAGE under reducing or non-reducing conditions after purification and the results of analyzing the domain variants by size exclusion chromatography.
FIG. 15 shows the results of evaluating the cytosol-penetrating ability of the CL domain variants of FIG. 13 at a concentration of 100 nM or 500 nM by cell-live imaging through an enhanced split green fluorescent protein complementation system.
FIG. 16 is a schematic diagram showing a cytosol-penetrating antibody in full-length IgG form having an endosomal escape motif in the heavy chain constant region CH3 domain and the light chain constant region CL domain.
FIG. 17 shows the results of analyzing the CH3 and CL domain variants of FIG. 16 by 12% SDS-PAGE under reducing or non-reducing conditions after purification and the results of analyzing the domain variants by size exclusion chromatography.
FIG. 18 shows the results of evaluating the non-specific binding of the CH3 and CL domain variants of FIG. 16 at concentrations of 100 nM and 500 nM by ELISA.
FIG. 19 shows the results of evaluating the non-specific binding of the CH3 and CL domain variants of FIG. 16 at concentrations of 500 nM and 1 µM to cell membranes by flow cytometry.
FIG. 20 shows the results of evaluating the cytosol-penetrating ability of variants with no non-specific binding among the CH3 and CL domain variants of FIG. 16 at a concentration of 100 nM or 500 nM by live-cell imaging through an enhanced split green fluorescent protein complementation system.
FIG. 21 shows the results of confocal microscope observation using an enhanced split green fluorescent protein complementation system to evaluate the concentration-dependent cytosol-penetrating ability of the cytosol-penetrating antibody in2C41.
FIG. 22 is a schematic diagram illustrating the process in which bioluminescence by complementation of split nanoluciferase luminescent protein is observed when a cytosol-penetrating antibody localizes in the cytosol.
FIG. 23 is a schematic diagram showing a calculation formula for quantifying the endosomal escape efficiency in the split nanoluciferase luminescent protein complementation system of FIG. 22, along with the experimental process.
FIG. 24 shows the results of analyzing HiBiT-fused cytosol-penetrating antibodies having an endosomal escape motif in the constant region by 12% SDS-PAGE under reducing or non-reducing conditions after purification and the results of analyzing the antibodies by size exclusion chromatography.
FIG. 25 shows the results of quantifying the endosomal escape efficiency of HiBiT-fused cytosol-penetrating antibodies having an endosomal escape motif in the constant region at a concentration of 500 nM through a split nanoluciferase luminescent protein complementation system.
FIG. 26 shows the results of a pulse-chase experiment and confocal microscopy performed to observe the transport process of the cytosol-penetrating antibody in2C41 introduced into cells.
FIG. 27 shows the results of observing the cytosolic localization of the cytosol-penetrating antibody in2C41 by confocal microscopy using calcein in the presence or absence of pharmacological inhibitors.
FIG. 28 shows the results of analyzing the binding of the cytosol-penetrating antibody in2C41 to the cell membrane depending on pH by flow cytometry.
FIG. 29 shows the results of performing evaluation in the Ramos cell line by live-cell imaging to examine whether the cells can take up membrane-impermeable trypan blue through cell membrane permeabilization by the cytosol-penetrating antibody in2C41 depending on pH and whether the cell membrane permeabilization is a transient and reversible phenomenon.
FIG. 30 shows the results of confocal microscopy observation performed to examine whether the cytosol-penetrating antibody in2C41 passes through the cell membrane and enters the cell, depending on pH.
FIG. 31 shows the results of evaluating the thermal stability of cytosol-penetrating antibodies (in2C11, in2C21, and in2C41) having endosomal escape motifs in the antibody constant region by differential scanning calorimetry.
FIG. 32 shows the results of analyzing, by BLI, the pH-dependent affinity of in2C41 having endosomal escape motifs in the heavy chain constant region CH3 domain and the light chain constant region CL domain for the heavy chain constant region receptor FcRn.
FIG. 33 shows the results of examining the pharmacokinetics of the cytosol-penetrating antibody in2C41 in BALB/c nude mice.
FIG. 34 is a schematic diagram showing an in2C7N cytosol-penetrating antibody having an endosomal escape ability enhanced through a FabCab domain in which the CH1-CL pair of a full-length IgG antibody is replaced with a CH3-CH3 pair having an endosomal escape motif.
FIG. 35 shows the results of modeling, using AlphaFold2, the Fab structures of FabCab domain variants in which the CL-CH1 pair of a full-length IgG antibody is replaced with a CH3-CH3 pair having an endosomal escape motif, and analyzing the intramolecular interaction patterns of the antibodies using the PyMOL molecular visualizer.
FIG. 36 shows the results of analyzing the FabCab domain variants of FIG. 35 by 12% SDS-PAGE under reducing or non-reducing conditions after purification and the results of analyzing the domain variants by size exclusion chromatography.
FIG. 37 shows the results of evaluating the non-specific binding of the cytosol-penetrating antibody in2C71, in which the CL-CH1 pair of a full-length IgG antibody is replaced with a CH3-CH3 pair having an endosomal escape motif, at concentrations of 100 nM and 500 nM by ELISA.
FIG. 38 shows the results of evaluating the non-specific binding of the cytosol-penetrating antibody in2C71, in which the CL-CH1 pair of a full-length IgG antibody is replaced with a CH3-CH3 pair having an endosomal escape motif, to the cell membrane at concentrations of 500 nM and 1 µM by flow cytometry.
FIG. 39 shows the results of evaluating the endosomal escape ability of the cytosol-penetrating antibody in2C71, in which the CL-CH1 pair of a full-length IgG antibody is replaced with a CH3-CH3 pair having an endosomal escape motif, at a concentration of 500 nM by live-cell imaging through an enhanced split green fluorescent protein complementation system.
FIG. 40 shows the results of evaluating the pH-dependent interactions of cytosol-penetrating antibodies (in2C41 and in2C71) having endosomal escape motifs in the constant region with the cell membrane in Ramos cells, an endosomal membrane model cell line, in order to indirectly confirm the endosomal escape mechanism of the cytosol-penetrating antibodies.
FIG. 41 is a schematic diagram illustrating the construction of an anti-α-tubulin cytosol-penetrating antibody (inTu41) in full-length IgG form to evaluatre the activity of a cytosol-penetrating antibody (in2C41) having endosomal escape motifs in the constant region.
FIG. 42 shows the results of analyzing an anti-α-tubulin cytosol-penetrating antibody (inTu41) in full-length IgG form by 12% SDS-PAGE under reducing or non-reducing conditions after purification and the results of analyzing the antibody by size exclusion chromatography.
FIG. 43 shows the results of confocal microscopy performed to examine whether the anti-α-tubulin cytosol-penetrating antibody (inTu41) in full-length IgG form overlaps with the cytoskeletal protein α-tubulin localized in the cytosol.
FIG. 44 is a schematic diagram illustrating the production of an anti-RAS cytosol-penetrating antibody (inRas41) in full-length IgG form to confirm the activity of a cytosol-penetrating antibody (in2C41) having an endosomal escape motif in the constant region.
FIG. 45 shows the results of analyzing an anti-RAS cytosol-penetrating antibody (inRas41) in full-length IgG form by 12% SDS-PAGE under reducing or non-reducing conditions after purification and the results of analyzing the antibody by size exclusion chromatography.
FIG. 46 shows the results of confocal microscopy performed to examine whether the anti-RAS cytosol-penetrating antibody (inRas41) in full-length IgG form overlaps with intracellular RAS in SW480 (KRAS^{G12V}) cells.
FIG. 47 is a schematic diagram illustrating the production of an immunotoxin (_{ER}2C41-DTA) by fusing a bacterial-derived toxin (DT toxin) to a cytosol-penetrating antibody (_{ER}2C41) that targets the cancer cell-overexpressed receptor EGFR and has an endosomal escape motif in the constant region, as well as the splicing process.
FIG. 48 shows the results of analysis by 12% SDS-PAGE under reducing or non-reducing conditions and analysis by size exclusion chromatography to examine whether the toxin would be well fused to the antibody after fusing the bacterial-derived toxin to the antibody through splicing.
FIG. 49 is a schematic diagram illustrating the process in which an immunotoxin having cytosol-penetrating ability conferred by an endosomal escape motif in the constant region exhibits toxicity to target cells.
FIG. 50 shows the results of evaluating the cytotoxicity of a cytosol-penetrating antibody-based immunotoxin (_{ER}2C41-DTA), which targets the cancer cell-overexpressed receptor EGFR, in cell lines with different EGFR expression levels to evaluate the target cell-specific cytotoxicity of the immunotoxin.
FIG. 51 shows the results of evaluating the efficacy of a cytosol-penetrating antibody-based immunotoxin (_{ER}2C41-DTA) targeting the cancer cell-overexpressed EGFR receptor in a mouse xenograft model using the EGFR-overexpressing cell line A431 and measuring the tumor weight after the completion of treatment.
FIG. 52 shows the results of evaluating the survival rate and weight gain/loss of mice during treatment and the serum AST and ALT levels after the completion of treatment in the mouse xenograft model of FIG. 51.

### Mode for Invention

Unless otherwise defined, all technical and scientific terms used in the present specification have the same meanings as commonly understood by those skilled in the art to which the present invention pertains. In general, the nomenclature used in the present specification is well known and commonly used in the art.

The present invention is intended to provide a high-efficiency cytosol-penetrating antibody (cytotransmab) by introducing an endosomal escape motif for conferring endosomal escape ability into the heavy chain constant region and/or light chain constant region of an immunoglobulin G (IgG) antibody, thereby enabling the IgG antibody comprising the endosomal escape motif to actively move from outside a cell into the cytosol inside the cell.

The "cytosol-penetrating antibody having highly efficient cytosol-penetrating ability in the antibody constant region" according to the present invention refers to an antibody in immunoglobulin form that is internalized by binding to a membrane protein receptor or the like, which is overexpressed in target cells in tumor tissue, etc., and then localizes in the cytosol through endosomal escape.

Specifically, the present invention has sought to develop a cytosol-penetrating antibody by introducing amino acid mutations that induce interaction with the cell membrane into the constant region of the antibody as set forth in SEQ ID NOs: 2 to 12, so as to confer an endosomal escape motif to each of the antibody constant region CH3 and CL domains. The cytosol-penetrating antibody induces membrane destabilization through its increased interaction with the endosome membrane through changes in the charge state of amino acids in the constant region in the endosomal weakly acidic environment after endocytosis into living cells, and ultimately, localizes in the cytosol through collapse of the endosomal membrane.

More specifically, the present inventors have modified the antibody constant region to confer cytosol-penetrating ability to the antibody constant region, identified the biophysical properties of the resulting variants, and confirmed and evaluated their improved cytosol-penetrating ability compared to existing cytosol-penetrating antibodies in a living cell-based assay system, thereby completing the present invention.

In the present invention, the term "endosomal escape" may mean that an antibody localized in an endosome through endocytosis escapes from the endosome in an endosome-specific environment and localizes in the cytosol.

In the present invention, the term "endosomal escape motif" refers to a one-dimensional structure comprising a specific amino acid sequence having a structural property of inducing endosomal escape and a three-dimensional structure formed thereby. The term "endosomal escape motif" may be used interchangeably with the term "motif having endosomal escape ability". An antibody comprising a constant region an "endosomal escape motif" and a substance that targets a target cell or tissue-specific antigen is capable of "penetrating the cytosol in a target cell/tissue-specific manner". The term "cytosol-penetrating antibody" means that an antibody that penetrated cells by endocytosis escapes from the endosome into the cytosol, and may be used interchangeably with the term "antibody having cytosol-penetrating ability".

The present invention relates to an antibody in full-length immunoglobulin form having an endosomal escape motif in the antibody constant region.

### Logic for selecting amino acids that constitute endosomal escape motif in antibody constant region

The endosomal escape motif uses three amino acid residues (arginine (R), tryptophan (W), and glutamic acid (E)) so that the antibody may induce endosomal membrane destabilization through interaction with the endosomal membrane, which is specific to the endosomal weakly acidic environment, after endocytosis.

Arginine (R) and tryptophan (W) are amino acids known to contribute most significantly to interactions with cell membranes and destabilization of cell membranes in many studies on cell-penetrating peptides. Arginine may contribute to initial binding to the phospholipid head group of the cell membrane and membrane destabilization, and tryptophan may be located at the interface between the hydrophilic and hydrophobic regions of the cell membrane and destabilize the cell membrane. In particular, when a cation-π interaction is formed between arginine and tryptophan, tryptophan may enhance the interaction of arginine with the membrane. In one embodiment, the endosomal escape motif in the antibody constant region may comprise two or more arginine and tryptophan residues, which may be located at an adjacent distance so that a cation-π interaction is formed therebetween.

Specifically, in the process of cytosolic penetration, the positively charged amino acid arginine is involved in initial binding to the cell membrane through electrostatic interaction with the negatively charged surface of the cell membrane. The amine group of lysine (K), another positively charged amino acid, may form a hydrogen bond with one phosphate group per molecule, whereas the guanidinium group of arginine may form bidentate hydrogen bonds with two or more phosphate groups per molecule. Thereby, arginine may destabilize lipid packing by inducing the formation of negative Gaussian curvature on the cell membrane. Also, the guanidine group of arginine has a larger dipole moment than the amine group of lysine. Therefore, unlike lysine, arginine may maintain a positive charge even in a hydrophobic environment, so it may attract water molecules and the hydrophilic heads of phospholipids into the hydrocarbon core of the cell membrane through hydrogen bonding. When arginine attracts water molecules or the hydrophilic heads of phospholipids into the hydrophobic phospholipid bilayer, it may significantly destabilize or structurally distort the cell membrane, causing temporary and/or local collapse of the cell membrane.

Tryptophan (W) is the most hydrophobic amino acid. In addition, it may form hydrogen bonds through the nitrogen contained in its side chain, indole, and may interact through the π-electron system. Due to its complex characteristics of being highly hydrophobic and capable of electrostatic interactions, tryptophan may be located at the boundary between hydrophilic and hydrophobic environments, thereby destabilizing the cell membrane by forming a positive Gaussian curvature on the cell membrane surface, increasing lateral pressure, or lowering the density of cell membrane lipid packing.

However, arginine or tryptophan alone faces a significant energy barrier when passing through the hydrophobic core of the lipid bilayer. To address this, a strategy of facilitating a cation-π interaction induced in an arginine-tryptophan pair (R-W pair) by placing arginine and tryptophan within a distance of 3.5 to 6 Å was used. Tryptophan may exhibit a synergistic effect in terms of cell membrane destabilization when acting together with arginine. When a cation-π interaction is formed between tryptophan and arginine, tryptophan may help arginine form an ion pair with the hydrophilic head of the cell membrane phospholipid. Additionally, the cation-π interaction between tryptophan and arginine may partially cover the positive charge of arginine, thereby lowering the energy barrier required for hydrophilic arginine to penetrate into the cell membrane. Because the cation-π interaction between tryptophan and arginine may be maintained even in the hydrocarbon core, it may help arginine penetrate deeper into the hydrocarbon core of the cell membrane when hydrophilic arginine interacts with the cell membrane. Thereby, tryptophan may help arginine participate in initial binding to the cell membrane and cell membrane destabilization, thereby promoting cell membrane permeabilization more efficiently and ultimately enhancing the endosomal escape ability of the antibody.

Since the cell membrane of all cells has a negative charge on the surface and a hydrophobic interior, an endosomal escape motif may be composed of arginine and tryptophan. In this case, interaction with the cell membrane may be induced, but the non-specific binding of the antibody to the cell membrane may be increased. Non-specific binding may lead to problems such as toxic effects and reduced half-life due to indiscriminate interaction with cells in vivo. Additionally, since tryptophan may lower protein solubility and induce aggregate formation, an endosomal escape motif should be designed to interact with the cell membrane in an endosomal environment-specific manner and should be designed so as not to have disadvantages in terms of developability.

To prevent non-specific interactions between an arginine-tryptophan pair (R-W pair) and the cell membrane and to ensure selective interactions with the endosomal membrane, the present inventors utilized a weakly acidic endosomal lumen (pH 5 to 6) by placing a cluster of Glu residues (so-called E patch) near the R-W pair. The carboxyl side chain of Glu has a low pKₐ (4.3), but clustering of Glu residues may increase this pKa.

It is known that after endocytosis, the interior of the endosome is acidified by vacuolar ATPase, so the internal environment of the early endosome is pH 5.5 to 6.5, and the late endosome is weakly acidic with a pH of about 4.5 to 5.5. Therefore, by introducing an amino acid that may change its charge state depending on pH into an endosomal escape motif, the endosomal escape motif may have different physicochemical properties than at neutral pH when exposed to the weakly acidic pH of the endosome. Through this pH-dependent endosomal escape motif, interactions with the cell membrane may be induced specifically in the endosomal environment, and non-specific binding may be inhibited in the circulatory process in the body, which is a neutral pH environment.

Aspartic acid (D) and glutamic acid (E) are negatively charged amino acids that carry a negative charge at neutral pH, but as pH decreases, the carboxylic acid (COO-) in the amino acid side chain may be protonated. Thus, they are pH-dependent amino acids as they have increased hydrophobicity and are capable of hydrophobic interactions with surrounding hydrophobic substances. The pKₐ values of aspartic acid and glutamic acid is around 3.8 and 4.2, respectively, and glutamic acid has a pKₐ closer to the endosomal weakly acidic environment (pH 4.5-6.5) than aspartic acid.

At this time, the pKₐ of glutamic acid (about 4.3) is still lower than that of the endosomal weakly acidic environment (pH 4.5 to 6.5), so most of the carboxylic acids in the side chain remain negatively charged and the rate of protonation is low. As a strategy to overcome this, the pKₐ of glutamic acid may be increased by placing multiple glutamic acids in adjacent positions in the three-dimensional structure. When functional groups with the same charge are present in adjacent positions, the equilibrium shifts so that they lose their charge to reduce charge repulsion, making it easier for glutamic acids to lose their charge. That is, when glutamic acids are clustered in adjacent positions in the three-dimensional structure, the protonation of the carboxyl group in the side chain of glutamic acid to lose its charge may reduce charge repulsion, resulting in a more stable state. This allows the pKₐ of the side chain carboxylic acid of glutamic acid to increase, and ultimately allows glutamic acid to be protonated more easily even under weakly acidic conditions.

Therefore, the present inventors have devised a pH-dependent endosomal escape motif by incorporating a glutamic acid patch, in which glutamic acids are clustered in adjacent positions in a specific three-dimensional structure, into an endosomal escape motif. The glutamic acid patch of the endosomal escape motif is electrostatically repelled by the negatively charged cell surface under physiological conditions of neutral pH, but in the endosomal environment with a weakly acidic pH, the glutamic acid is protonated and loses its charge, so it is not electrostatically repelled by the cell surface and may induce cell membrane destabilization along with arginine and tryptophan through hydrophobic interactions with the hydrocarbon chains of phospholipids. In addition, glutamic acid is an amino acid known to contribute most to improving the solubility of proteins, and the introduction of glutamic acid may be expected to offset the deterioration of physical properties caused by the introduction of tryptophan and arginine.

Accordingly, the above-described endosomal escape motif has an amino acid composition of arginine, tryptophan, and glutamic acid so that the antibody may induce endosomal membrane destabilization through interaction with the endosomal membrane in a manner which is specific to the endosomal weakly acidic environment, after endocytosis.

### Logic for selecting amino acid positions of endosomal escape motif in antibody constant region

The position of the endosomal escape motif having an amino acid composition of arginine, tryptophan, and glutamic acid was devised considering the one-dimensional, two-dimensional, and three-dimensional structures of the antibody constant region so that the antibody could induce pH-dependent interaction with the endosomal membrane and endosomal membrane destabilization through the endosomal escape motif of the constant region.

Specifically, endosomal escape motifs were introduced into the AB loop, EF loop and C-terminal loop of the antibody constant region CH3 and CL domains. The AB loop, EF loops and C-terminal loops of the constant region are all located at the bottom of each domain, and the bottom of each domain forms a dimer (CL-CH1 or CH3-CH3 pair) through interaction with the bottom of the opposite domain. Therefore, when endosomal escape motifs are introduced into the AB loop, EF loop and C-terminal loop of the constant region, an avidity effect may be expected as two endosomal escape motifs are located close to each other due to the dimer characteristics.

Each amino acid position of the endosomal escape motif having an amino acid composition of arginine, tryptophan, and glutamic acid was selected based on the following three criteria, taking into account the interactions between amino acids and the change in the charge state of each amino acid in the 3D structures of the AB, loop, EF loop and C-terminal loop of the CH3 or CL domain.

### 1) Identification of surface-exposed amino acid positions

The present inventors sought to expose all amino acids constituting the endosomal escape motifs of the AB loop, EF loop and C-terminal loop at the bottom of the antibody constant region on the surface of the 3D structure to facilitate interaction with the cell membrane and pH-dependent changes in charge state. To this end, the surface-exposed amino acid positions in the CH3 and CL domains were identified from the three-dimensional model structures of the Fc region and Fab region using the GETAREA web server (https://curie.utmb.edu/getarea.html).

At this time, GETAREA identifies surface-exposed amino acid positions, non-surface-exposed amino acid positions, and buried amino acid positions by calculating the ratio (%) of solvent accessible surface area (SASA) based on the three-dimensional structure. Here, "surface-exposed amino acid positions" refers to a state in which the ratio (%) is 50 or higher. Also, "non-surface-exposed amino acid positions" refers to a state in which the ratio (%) is 50 or lower, and "buried amino acid positions" refers to a state in which the ratio (%) is 20 or lower.

The three-dimensional structure (PDB file) of the wild-type Fc region or Fab region was submitted to the GETAREA web server to obtain the SASA ratio (%) for the AB, EF, and C-terminal loops of each domain. Based on the surface-exposed amino acid position information, the surface-exposed amino acid positions in the AB, EF, and C-terminal loops were selected.

### 2) Selection of positions capable of induction cation-π interaction between arginine (R) and tryptophan (W) in endosomal escape motif

When arginine (R) and tryptophan (W) in an endosomal escape motif form a cation-π interaction, their binding force to the cell membrane is strengthened, and they may efficiently destabilize the cell membrane after binding. Therefore, among the surface-exposed amino acid positions in the AB, EF, and C-terminal loops, positions were selected where the distance between the Cα of the two amino acids or the distance between their side chains could be within 6 Å so that arginine and tryptophan could form a cation-π interaction pair. In addition, when an arginine-tryptophan pair is placed at positions where the amino acid side chains are oriented in the same direction in the AB loop and EF loop where the α-helix is present, the cation-π interaction between arginine and tryptophan may stabilize the α-helix. Therefore, by introducing an arginine-tryptophan pair at surface-exposed amino acid positions in the α-helix regions of the AB loop and EF loop, interaction with the cell membrane was enhanced and, at the same time, structural stability was provided.

At this time, since steric hindrance formed by introducing a number of large-sized amino acids, such as tryptophan, into a narrow region may affect protein folding, the amino acids around the positions of arginine and tryptophan were replaced with small amino acids such as alanine (A) to prevent potential steric hindrance.

### 3) Selection of position of glutamic acid (E) patch in endosomal escape motif

To induce the interaction of an endosomal escape motif with the endosomal membrane, which is specific to the endosomal weakly acidic pH, a glutamic acid (E) patch was introduced at the surface-exposed amino acid positions in the AB, EF, and C-terminal loops.

In this case, when the glutamic acids of the glutamic acid patch are clustered in adjacent positions, the distance between the side chains may increase due to electrostatic repulsion between the side chains. Therefore, the glutamic acid patch was introduced at positions where the distance between the amino acid side chains as well as the distance between the Cα of amino acids were as short as possible, so that even if the distance between the side chains increased somewhat, the glutamic acids could electrostatically influence each other, increasing the pKₐ.

In addition, the glutamic acid patch was positioned around an arginine-tryptophan pair. This was done because when the glutamic acid patch is positioned around the arginine-tryptophan pair, at neutral pH, the glutamic acid patch may inhibit the non-specific binding that may occur due to arginine-tryptophan, and at endosomal weakly acidic pH, the glutamic acid patch is protonated, facilitating the interaction of arginine-tryptophan with the cell membrane.

At this time, if a positively charged amino acid such as lysine or arginine is at a distance and angle where it may form a salt bridge with the glutamic acid, the equilibrium shifts toward maintaining the negative charge because the glutamic acid is negatively charged and forms a more stable salt bridge with the positively charged amino acid. That is, when the glutamic acid forms a salt bridge with a surrounding positively charged amino acid, the pKₐ of the side chain carboxylic acid of the glutamic acid may decrease, and as a result, protonation of the glutamic acid may become difficult in a weakly acidic pH environment. Considering the pKa change of glutamic acid depending on the surrounding amino acid environment, glutamic acid was introduced at a position where it could not form a salt bridge with the surrounding arginine or lysine, among the surface-exposed amino acid positions in the AB, EF and C-terminal loops of the constant region.

In addition, for the reasons described above, if there is lysine or arginine that may form a salt bridge with glutamic acid at a position adjacent position to glutamic acid, such lysine or arginine may be replaced with asparagine (N) or glutamine (Q), which a non-charged amino acid having a similar size.

For the glutamic acid patch introduced based on the above-described logic, the PROPKA 3.1 web server (https://biolib.com/bio-utils/propka/) was used to predict the pKₐ of the glutamic acid side chain and check whether the pKₐ increased. PROPKA 3.1 predicts the pKₐ of the charged amino acid side chain based on the 3D structure. Therefore, the 3D structure (PDB file) of the wild-type Fc region or Fab region and the 3D model structure (PDB file) of the variant's Fc region or Fab region were submitted to the PROPKA 3.1 web server to check whether the pKₐ of the glutamic acid side chain was increased through the glutamic acid patch.

Therefore, the glutamic acid patch was introduced at amino acid positions where the distance between the Cα of amino acids is short, an arginine-tryptophan pair is nearby, and the glutamic acid does not form a salt bridge with the surrounding positive amino acid, among the surface-exposed amino acid positions in the AB, EF and C-terminal loop of the constant region.

The present invention provides the amino acid composition and position of the endosomal escape motif of the antibody constant region, designed based on the above-described logic.

The endosomal escape motif may be contained in one or more selected from the group consisting of a heavy chain constant region CH3 domain of a heavy chain constant region and a light chain constant region CL domain.

In one aspect of the present invention, the endosomal escape motif may be located in the AB loop, EF loop and C-terminus of the heavy chain constant region CH3 domain of SEQ ID NO: 1.

[SEQ ID NO: 1]

LPPSRDELTKNQVSLTCLVKGFYPSDIAVEWESNGQPENNYKTTPPVLDSDGSF

FLYSKLTVDKSRWQQGNVFSCSVMHEALHNHYTQKSLSLSPGK

The N-terminal position of the sequence of SEQ ID NO: 1 is 351 and the C-terminal position is 447, indicating that the sequence comprises 97aa.

The CH3 domain contains endosomal escape motifs consisting of the following amino acid sequences in the AB loop, EF loop and C-terminal positions. The following amino acid residue numbers follow the EU numbering system.

### AB loop: ³⁵³PS-X-X-E-X-X-K-X-X³⁶²

In the above AB loop, X may be selected from the group consisting of arginine (R), tryptophan (W), glutamic acid (E), alanine (A), asparagine (N), and glutamine (Q).

### EF loop: ⁴¹¹TV-X-X-X-RW-XX-GNVF⁴²³

In the above EF loop, X may be selected from the group consisting of arginine (R), tryptophan (W), glutamic acid (E), alanine (A), asparagine (N), and glutamine (Q).

### C-terminus: ⁴³⁹X-SLS-X-SPGK⁴⁴⁷

In the above C-terminus, X may be selected from the group consisting of arginine (R), tryptophan (W), glutamic acid (E), alanine (A), asparagine (N), and glutamine (Q).

In one aspect of the present invention, the endosomal escape motif may be located in the AB loop, EF loop and C-terminus of the light chain constant region CL domain of SEQ ID NO: 17.

[SEQ ID NO: 17]

FPPSDEQLKSGTASVVCLLNNFYPREAKVQWKVDNALQSGNSQESVTEQDSKDS

TYSLSSTLTLSKADYEKHKVYACEVTHQGLSSPVTKSFNRGEC

The N-terminal position of the sequence of SEQ ID NO: 17 is 118 and the C-terminal position is 217, indicating that the sequence comprises 97aa.

The light constant region CL domain contains endosomal escape motifs consisting of the following amino acid sequences at the following AB loop, CD loop, EF loop and C-terminal positions. The following amino acid residue numbers follow the EU numbering system.

### AB loop: ¹²⁰PS-X-X-Q-X-X-S-X-X¹²⁹

In the above AB loop, X may be selected from the group consisting of arginine (R), tryptophan (W), glutamic acid (E), alanine (A), asparagine (N), and glutamine (Q).

### EF loop: ¹⁸⁰TL-X-X-X-DY-XX-HKVY¹⁹²

In the above EF loop, X may be selected from the group consisting of arginine (R), tryptophan (W), glutamic acid (E), alanine (A), asparagine (N), and glutamine (Q).

### C-terminus: ²⁰⁷X-SFN-X-GEC²¹⁴

In the above C-terminus, X may be selected from the group consisting of arginine (R), tryptophan (W), glutamic acid (E), alanine (A), asparagine (N), and glutamine (Q).

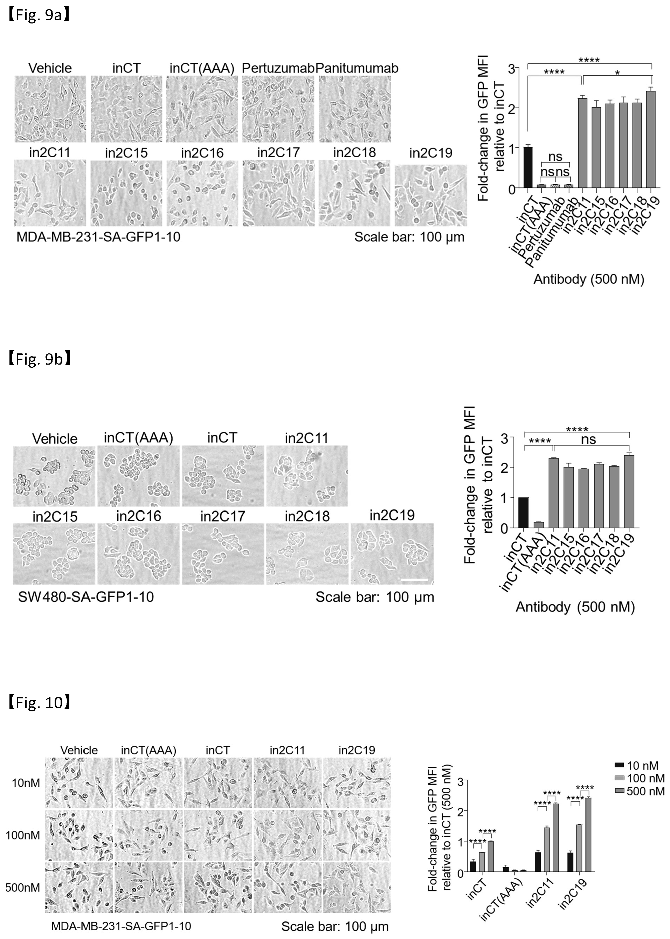

The present invention relates to a cytosol-penetrating antibody or a Fc fragment thereof or a Fab fragment thereof comprising a heavy chain constant region having the ability to escape from the endosome into the cytosol through the endosomal escape motif. In one example, as shown in FIG. 9, it was confirmed that the endosomal escape ability of in2C11, in which an endosomal escape motif has been introduced into the heavy chain constant region CH3 domain, increased by about 2.2 times compared to the existing cytosol-penetrating antibody inCT.

The endosomal escape motif may be introduced at corresponding positions in the sequences and/or structures of other isotypes (IgA, IgE, IgD, and IgM) and subclasses (IgG1, IgG2, IgG3, IgG4, IgA1, and IgA2) of immunoglobulin where there is high sequence homology and/or structural homology.

The heavy chain constant region CH3 domain having the endosomal escape motif may comprise one or more sequences selected from the group consisting of SEQ ID NOs: 2 to 12.

The heavy chain constant region CH3 domain having the endosomal escape motif may comprising a sequence having at least 80% homology, for example, 85%, 90%, 95%, 96%, 97%, 98%, 99% or 100% homology to the sequence of a heavy chain constant region selected from the group consisting of SEQ ID NOs: 2 to 12.

The light chain invariant region CL domain having the endosomal escape motif may comprise one or more sequences selected from the group consisting of SEQ ID NOs: 18 and 19.

The light chain invariant region CL domain having the endosomal escape motif may comprise a sequence having at least 80% homology, for example, 85%, 90%, 95%, 96%, 97%, 98%, 99% or 100% homology to the sequence of a light chain constant region selected from the group consisting of SEQ ID NOs: 18 and 19.

In the present invention, the cytosol-penetrating antibody or fragment thereof may comprise the following structure:
(1) a CH1-CL pair comprising a heavy chain constant region CH1 domain and a light chain constant region CL domain containing an endosomal escape motif;
(2) a CH3-CH3 pair containing an endosomal escape motif in a heavy chain constant region CH3 domain; and/or
(3) a constant region CH3-CH3 pair in which a constant region CH1-CL pair is replaced with the constant region CH3-CH3 pair of (2) and which contains an endosomal escape motif in a heavy chain invariant region CH3 domain. The pair of the heavy chain constant region CH1 and the light chain constant region CL may be replaced with a CH3-CH3 pair containing an endosomal escape motif, so that endosomal escape motifs may be contained in six CH3 domains, and the CH3-CH3 pair replacing the CH1-CL pair may be a heterodimer.

In the present invention, a FabCab format antibody in which the constant region CH1-CL pair is replaced with a CH3-CH3 pair containing an endosomal escape motif may be produced, so that the cytosol-penetrating ability may be enhanced by introducing endosomal escape motifs into a total of six CH3 domains. In this case, to prevent the CH3 domains replacing the CH1-CL pair from improperly assembling with the CH3 domain of the Fc region to form oligomers, heterodimeric Fc technology may be introduced. Specifically, the CH3 domain replacing the CH1 domain may contain Q347R, D399V, and F405T (EU numbering) mutations, and the CH3 domain replacing the CL domain may contain K360E and K409W (EU numbering) mutations.

The constant region heterodimeric CH3 pair having the endosomal escape motif may comprise one or more sequences selected from the group consisting of SEQ ID NOs: 23 to 26. The antibody comprising the CH3-CH3 pair replacing the CH1-CL pair may comprise one or more sequences selected from the group consisting of SEQ ID NOs: 23 to 26.

The heterodimeric CH3 pair having the endosomal escape motif may comprise a sequence having at least 80% homology, for example, 85%, 90%, 95%, 96%, 97%, 98%, 99% or 100% homology to the sequence of a constant region selected from the group consisting of SEQ ID NOs: 23 to 26.

According to one embodiment of the present invention, the high-efficiency cytosol-penetrating antibody having an endosomal escape motif in the antibody constant region CH3 domain and/or CL domain comprises a substance that specifically binds to a cell surface membrane protein receptor that is overexpressed in a target cell/tissue.

The high-efficiency cytosol-penetrating antibody having an endosomal escape motif in the constant region CH3 domain and/or CL domain is a cytosol-penetrating antibody with the constant region, which localizes in the cytosol by its endosomal escape ability after being endocytosed by specifically binding to a cell surface membrane protein receptor that is overexpressed in a target cell/tissue.

The high-efficiency cytosol-penetrating antibody having an endosomal escape motif in the CH3 domain and/or CL domain may specifically recognize one or more selected of target cell/tissue-specific antigens, e.g., EpCAM (epithelial cellular adhesion molecule), EGFR (epidermal growth factor receptor), and integrin αvβ3/αvβ5, which are overexpressed in tumor cells.

The tumor cell line may overexpress one or more selected from EpCAM, EGFR, and integrin αvβ3/αvβ5.

The substance targeting the target cell- or tissue-specific antigen may be, for example, a ligand, an oligopeptide, an antibody or a fragment thereof, or an aptamer, which may specifically bind to the antigen, without being limited thereto.

According to one embodiment of the present invention, the cytosol-penetrating antibody according to the present invention may be fused with a cyclic peptide or monobody targeting a receptor overexpressed in tumor tissue for tumor tissue-specific cytosolic penetration, wherein the cyclic peptide may be a cyclic peptide in4 (DGVRQCRGDCFDGPL) that targets integrin αvβ3/αvβ5 (Science Advances, 2020, 6(3):eaay2174). The monobody may be an EGFR-targeting monobody ER (VSDVPRDLEVVAATPTSLLISWDSGRGS YQYYRITYGETGGNSPVQEFHVPGPHHTATISGLKPGVDYTITVYAVTDHKPHADGPHT YHESPISINYRTEIDKPSQ) (Korean Patent Application No. 10-2023-0181933).

The cyclic peptide may be linked directly or via a linker to the N- or C-terminus of the antibody. The linker may be selected from among peptides having various sequences and lengths, and may be a linker having the sequence GGGGS, (GGGGS)₂, (GGGGS)₄, ASTKGP, or ASTKGPSVFPLAP, which provides structural flexibility without being cleaved by protease.

An object of the present invention is to develop a high-efficiency IgG cytosol-penetrating antibody by introducing an endosomal escape motif into the heavy chain constant region and/or light chain constant region of an IgG antibody.

To achieve the above object, the heavy chain constant region CH3 domain of the high-efficiency cytosol-penetrating antibody according to the present invention consists of an AB loop comprising an amino acid sequence of Formula 1 below, an EF loop comprising an amino acid sequence of Formula 2 below, and a C-terminal loop comprising an amino acid sequence of Formula 3 below.

In addition, the light chain constant region CL domain of the high-efficiency cytosol-penetrating antibody according to the present invention consists of an AB loop comprising an amino acid sequence of Formula 4 below, an EF loop comprising an amino acid sequence of Formula 5 below, and a C-terminal loop comprising an amino acid sequence of Formula 6 below. The amino acid residue numbers below follow the EU numbering system.

In addition, the heterodimeric CH3 pair of the high-efficiency cytosol-penetrating antibody according to the present invention consists of an AB loop comprising an amino acid sequence of Formula 1 below, an EF loop comprising an amino acid sequence of Formula 2 below, and a C-terminal loop comprising an amino acid sequence of Formula 3 below. The amino acid residue numbers below follow the EU numbering system.

[Formula 1] ³⁵³PS-X-X-E-X-X-K-X-X³⁶²

In Formula 1 above, X may be R, W, E, A, N or Q.

[Formula 2] ⁴¹¹TV-X-X-X-RW-XX-GNVF⁴²³

In Formula 2 above, X may be R, W, E, A, N or Q.

[Formula 3] ⁴³⁹X-SLS-X-SPGK⁴⁴⁷

In Formula 3 above, X may be R, W, E, A, N or Q.

[Formula 4] ¹²⁰PS-X-X-Q-X-X-S-X-X¹²⁹

In Formula 4 above, X may be R, W, E, A, N or Q.

[Formula 5] ¹⁸⁰TL-X-X-X-DY-XX-HKVY¹⁹²

In Formula 5 above, X may be R, W, E, A, N or Q.

[Formula 6] ²⁰⁷X-SFN-X-GEC²¹⁴

In Formula 6 above, X may be R, W, E, A, N or Q.

The fragments of the present invention include Fc, Fab, F(ab'), F(ab')2, and Fv. "Fc" is a fragment obtained when an immunoglobulin (Ig) molecule is degraded by papain, and is a region from which the variable region (VL) and constant region (CL) of the light chain and the variable region (VH) and constant region 1 (CH1) of the heavy chain have been removed.

Among antibody fragments, Fab is a structure having the variable regions of the light chain and heavy chain, the constant region of the light chain, and the first constant region (CH1) of the heavy chain, and has one antigen-binding site. Fab' differs from Fab in that it has a hinge region containing one or more cysteine residues at the C-terminus of the heavy chain CH1 domain. An F(ab')2 antibody is produced when the cysteine residues in the hinge region of Fab' form disulfide bonds.

Specifically, the present invention provides a full-length immunoglobulin antibody or an Fc fragment thereof comprising a CH3 domain selected from the amino acid sequences of SEQ ID NOs: 2 to 12.

The present invention also provides a full-length immunoglobulin antibody or an Fab fragment thereof comprising a CL domain selected from the amino acid sequence of SEQ ID NOs: 18 and 19.

The present invention also provides a full-length immunoglobulin antibody comprising a CH3 domain selected from the amino acid sequences of SEQ ID NOs: 2 to 12 and a CL domain selected from the amino acid sequences of SEQ ID NOs: 18 and 19.

The present invention also provides a full-length immunoglobulin antibody or an Fab fragment thereof comprising a CH3 domain selected from the amino acid sequences of SEQ ID NOs: 2 to 12 and a heterodimeric CH3 pair having the amino acid sequences of SEQ ID NOs 23 to 26.

The present invention also provides a target cell/tissue-specific high-efficiency cytosol-penetrating antibody that localizes in the cytosol through the endosomal escape ability of the endosomal escape motif of the antibody constant region after being endocytosed by specifically binding to a cell surface membrane protein receptor that is overexpressed in a living target cell or tissue.

The present invention provides a nucleic acid encoding the antibody or Fc fragment or Fab fragment thereof.

The present invention also provides an expression vector comprising the nucleic acid.

The present invention also provides a recombinant cell transformed with the expression vector.

The present invention also provides an Fc wherein an endosomal escape motif is introduced into a specific region of the heavy chain constant region CH3 domain of a conventional IgG antibody through amino acid mutation, a cytosol-penetrating antibody comprising the same, and a method for producing the same comprising the following steps: step (1) of selecting an amino acid position, into which an endosomal escape motif is to be introduced, from bottom loops (AB, EF, and C-terminal loops) of the heavy chain constant region CH3 domain of the antibody; step (2) of constructing a heavy chain expression vector for the cytosol-penetrating antibody by cloning a heavy chain nucleic acid comprising a CH3 domain having an endosomal escape motif at the selected amino acid position; step (3) of constructing a light chain expression vector by cloning a wild-type light chain nucleic acid; step (4) of co-transforming the constructed heavy chain and light chain expression vectors into animal cells for protein expression and expressing a cytosol-penetrating antibody in full-length immunoglobulin form in the cells; and step (5) of purifying and recovering the expressed cytosol-penetrating antibody.

The present invention also provides an Fab wherein an endosomal escape motif is introduced into a specific region of the light chain constant region CL domain of a conventional IgG antibody through amino acid mutation, a cytosol-penetrating antibody comprising the same, and a method for producing the same comprising the following steps: step (1) of selecting an amino acid position, into which an endosomal escape motif is to be introduced, from bottom loops (AB, EF, and C-terminal loops) of the light chain constant region CL domain of the antibody; step (2) of constructing a light chain expression vector for the cytosol-penetrating antibody by cloning a light chain nucleic acid comprising a CL domain having an endosomal escape motif at the selected amino acid position; step (3) of constructing a heavy chain expression vector by cloning a wild-type IgG heavy chain nucleic acid; step (4) of co-transforming the constructed heavy chain and light chain expression vectors into animal cells for protein expression and expressing a cytosol-penetrating antibody in full-length immunoglobulin form in the cells; and step (5) of purifying and recovering the expressed cytosol-penetrating antibody.

The present invention also provides a high-efficiency cytosol-penetrating antibody comprising a heavy chain constant region CH3 domain and light chain constant region CL domain having the endosomal escape motif devised above, and a method for producing the same comprising the following steps: step (1) of constructing a heavy chain expression vector by cloning a heavy chain nucleic acid comprising a CH3 domain having an endosomal escape motif; step (2) of constructing a light chain expression vector for the cytosol-penetrating antibody by cloning a light chain nucleic acid comprising a CL domain having an endosomal escape motif; step (3) of co-transforming the constructed heavy chain and light chain expression vectors into animal cells for protein expression and expressing a cytosol-penetrating antibody in full-length immunoglobulin form in the cells; and step (4) of purifying and recovering the expressed cytosol-penetrating antibody.

The present invention also provides a method of producing a cytosol-penetrating antibody having highly efficient endosomal escape ability by replacing the CL-CH1 pair of a conventional IgG antibody with a heterodimeric CH3 pair having the endosomal escape motif, the method comprising the following steps.

Specifically, to facilitate the formation of the heterodimeric CH3 pair replacing the CL-CH1 pair, EW-RVT mutations may be used in the heterodimeric Fc technology. The CH3 (EW) replacing CL has K360E and K409W mutations for the formation of the heterodimeric CH3 pair in CH3, the domain starting from position 109 (EU numbering) of the light chain is replaced with the CH3 (EW) domain, and 445Pro, 446Gly, and 447Lys are substituted with Gly, Glu, and Cys, respectively, for the formation of disulfide bonds with the heavy chain. The CH3 (RVT) replacing CH1 has Q347R, D399V, F405T mutations for the formation of the heterodimeric CH3 pair in CH3, the domain starting from position 119 (EU numbering) of the heavy chain is replaced with CH3 (RVT), and 445Pro, 446Gly, and 447Lys are substituted with Gly, Glu, and Cys, respectively, for the formation of disulfide bonds with the light chain. The above-described amino acid positions all follow the EU numbering system.

Step (1) of constructing a heavy chain expression vector for the cytosol-penetrating antibody by cloning a heavy chain nucleic acid comprising a heavy chain variable region (VH) and a heavy chain constant region (CH3(RVT)-hinge-CH2-CH3) in which CH1 is replaced with a CH3-RVT having an endosomal escape motif and a heterodimeric CH3-forming mutation; step (2) of constructing a light chain expression vector for the cytosol-penetrating antibody by cloning a light chain nucleic acid containing a light chain variable region (VL) and a light chain constant region (CH3(EW)) in which CL is replaced with a CH3-EW having an endosomal escape motif and a heterodimer-forming mutation; step (3) of co-transforming the constructed heavy chain and light chain expression vectors into animal cells for protein expression and expressing a cytosol-penetrating antibody in full-length immunoglobulin form in the cells; and step (4) of purifying and recovering the expressed cytosol-penetrating antibody.

The present invention also provides a method for producing a target cell/tissue-specific cytosol-penetrating antibody which has an endosomal escape motif in the CH3 domain and/or CL domain and in which GFP11-SBP2 or HiBiT is fused to the C-terminus of the heavy chain, the method comprising the following steps: step (1) of constructing a heavy chain expression vector for a cytosol-penetrating antibody fused with GFP11-SBP2 or HiBiT by cloning a nucleic acid comprising a heavy chain comprising a CH3 domain having an endosomal escape motif, wherein GFP11-SBP2 or HiBiT is linked to the C-terminus of the heavy chain; step (2) of constructing a light chain expression vector by cloning a nucleic acid comprising a light chain comprising a CL domain having an endosomal escape motif or a wild-type CL domain and a target cell/tissue-specific overexpressed receptor-targeting cyclic peptide fused to the N-terminus of the light chain; step (3) of co-transforming the constructed heavy chain and light chain expression vectors into animal cells for protein expression and expressing a cytosol-penetrating antibody in full-length immunoglobulin in the cells; and step (4) of purifying and recovering the expressed cytosol-penetrating antibody.

The present invention also provides a method for producing a target cell/tissue-specific cytosol-penetrating antibody having an endosomal escape motif in the CH3 domain and/or CL domain and having, in the heavy chain variable region (VH), the ability to target the cytoskeletal protein alpha-tubulin present in the cytosol, the method comprising the following steps: step (1) of constructing an anti-α-tubulin heavy chain expression vector comprising a CH3 domain having an endosomal escape motif and a heavy chain variable region (VH) having the ability to target α-tubulin; step (2) of constructing a light chain expression vector by cloning a nucleic acid comprising a light chain comprising a CL domain having an endosomal escape motif and a target cell/tissue-specific overexpressed receptor-targeting cyclic peptide fused to the N-terminus of the light chain; step (3) of co-transforming the constructed heavy chain and light chain expression vectors into animal cells for protein expression and expressing an anti-α-tubulin cytosol-penetrating antibody in full-length immunoglobulin form in the cells; and step (4) of purifying and recovering the expressed anti-α-tubulin cytosol-penetrating antibody.

The present invention also provides a method for producing a target cell/tissue-specific cell-penetrating interfering antibody having an endosomal escape motif in the CH3 domain and/or CL domain and having, in the heavy chain variable region (VH), the ability to target activated RAS, which is one of proteins present in the cytosol, the method comprising the following steps: step (1) of constructing an anti-RAS heavy chain expression vector comprising a CH3 domain having an endosomal escape motif and a heavy chain variable region (VH) having the ability to target activated RAS; step (2) of constructing a light chain expression vector by cloning a nucleic acid comprising a light chain comprising a CL domain having an endosomal escape motif, wherein a cyclic peptide targeting a target cell/tissue-specific overexpressed receptor is fused to the N-terminus of the light chain; step (3) of co-transforming the constructed heavy chain and light chain expression vectors into animal cells for protein expression and expressing an anti-RAS cell-penetrating interfering antibody in full-length immunoglobulin form in the cells; and step (4) of purifying and recovering the expressed anti-RAS cell-penetrating interfering antibody.

The present invention also provides a method for producing an antibody having an endosomal escape motif in the CH3 domain and/or CL domain and having N-intein fused to the light chain constant region (CL) and a C-intein-fused specific functional protein fused thereto by trans-splicing, the method comprising the following steps: step (1) of constructing a heavy chain expression vector comprising a CH3 domain having an endosomal escape motif; step (2) of constructing a light chain expression vector comprising a CL domain having an endosomal escape motif, wherein a cyclic peptide targeting a target cell/tissue-specific overexpressed receptor is fused to the N-terminus of the light chain and N-intein is fused to the C-terminus of the light chain; step (3) of constructing a functional protein expression vector in which C-intein is fused to the N-terminus; step (4) of co-transforming the constructed heavy chain and light chain expression vectors into animal cells for protein expression and expressing a cytosol-penetrating antibody in full-length immunoglobulin form in the cells; step (5) of transforming the constructed C-intein-fused functional protein expression vector into *E. coli* for protein expression and expressing a functional protein; step (6) of purifying and recovering the expressed cytosol-penetrating antibody and functional protein; and step (7) of fusing the purified cytosol-penetrating antibody and functional protein by trans-splicing and purifying and recovering the fusion protein.

The present invention also provides a conjugate comprising the antibody or Fc fragment or Fab fragment thereof and a bioactive molecule linked to the cytosol-penetrating antibody.

The present invention also provides a bi- or multispecific antibody comprising the antibody or Fc fragment or Fab fragment thereof and the cytosol-penetrating antibody.

The cytosol-penetrating antibody according to the present invention may be of IgG, IgM, IgA, IgD, or IgE type, for example, IgG1, IgG2, IgG3, IgG4, IgM, IgE, IgA1, IgA2, or IgD type. Most preferably, it may be a full-length IgG-type monoclonal antibody. The heavy chain constant region is of gamma (γ), mu (µ), alpha (α), delta (δ), or epsilon (ε) type, and subclasses thereof include gamma1 (γ1), gamma2 (γ2), gamma3 (γ3), gamma4 (γ4), alpha1 (α1), and alpha2 (α2). The light chain constant region is of kappa (κ) or lambda (λ) type.

"Heavy chain" means both a full-length heavy chain and a fragment thereof comprising a variable region domain VH comprising an amino acid sequence having a variable region sequence sufficient to confer specificity to an antigen, as well as three constant region domains CH1, CH2 and CH3. Also, the term "light chain" as used in the present specification means both a full-length light chain and a fragment thereof comprising a variable region domain VL comprising an amino acid sequence having a variable region sequence sufficient to confer specificity to an antigen, as well as a constant region domain CL.

Antibodies include, but are not limited to, monoclonal antibodies, multispecific antibodies, human antibodies, humanized antibodies, chimeric antibodies, single-chain Fvs (scFV), single-chain antibodies, Fab fragments, F(ab') fragments, disulfide-binding Fvs (sdFV) and anti-idiotype (anti-id) antibodies, or epitope-binding fragments of such antibodies.

"Interaction" is meant to include interactions between molecules, such as, for example, those that may be detected using yeast-2 hybrid analysis. The term "interaction" is meant to include "binding" interaction between two molecules.

The term "monoclonal antibody" refers to an antibody obtained from a substantially homogeneous population of antibodies, that is, the individual antibodies comprising the population are identical except for possible naturally-occurring mutations that may be present in minor amounts. Monoclonal antibodies are highly specific, being directed against a single antigenic site. Furthermore, in contrast to conventional (polyclonal) antibody preparations, which typically include different antibodies directed against different determinants (epitopes), each monoclonal antibody is directed against a single determinant on the antigen.

For example, the monoclonal antibodies useful in the present invention may be produced by hybridoma methods, or may be produced in bacterial, eukaryotic or plant cells using recombinant DNA methods. In addition, monoclonal antibodies may be isolated from phage antibody libraries.

The term "affinity" refers to the ability to specifically recognize and bind to a specific site of an antigen. Along with the specificity of an antibody to an antigen, high affinity is an important factor in the immune response. The affinity constant (*K*_{D}) may be determined using bio-layer interferometry (BLI), for example, Octet Qke. Based on bio-layer interferometry data, the affinity constant (*K*_{D}) is obtained from a 1:1 Langmuir binding model (simultaneously *k*ₒₙ and *k*_{off}) and the ratio of the rate constant *k_{off}*/*kₒₙ*.

When taking into consideration variations having biologically equivalent activity, the antibody of the present invention or a nucleotide molecule encoding the same is interpreted to include a sequence having substantial identity with the sequence set forth in the SEQ ID NO. The term "substantial identity" means that a sequence has a homology of at least 90%, more preferably a homology of at least 95%, at least 96%, at least 97%, at least 98% or at least 99%, when aligning the sequence of the present invention with another sequence so as to correspond thereto as closely as possible and analyzing the aligned sequence using algorithms commonly used in the art.

Alignment methods for sequence comparison are known in the art. The NCBI Basic Local Alignment Search Tool (BLAST) is accessible through NCBI or the like, and may be used on the Internet in connection with sequence analysis programs, such as blastp, blastm, blastx, tblastn, and tblastx. BLAST is available at www.ncbi.nlm.nih.gov/BLAST/. A method of comparing sequence homology using this program may be found at www.ncbi.nlm.nih.gov/BLAST/blast help.html.

Based thereon, the antibody or antigen-binding fragment thereof according to the present invention may have 90%, 91%, 92%, 93%, 94%, 95%, 96%, 97%, 98%, 99%, or higher homology with a specified sequence or all of the sequences described in the present specification. Such homology may be determined by sequence comparison and/or alignment using methods known in the art. For example, the percent sequence homology of the nucleic acid or protein of the present invention may be determined using a sequence comparison algorithm (i.e., BLAST or BLAST 2.0), manual alignment, or visual inspection.

The term "nucleic acid" is meant to encompass DNA (gDNA and cDNA) and RNA molecules, and nucleotides, which are the basic structural units of nucleic acids, include not only natural nucleotides, but also analogues having modified sugar or base moieties. The sequences of nucleic acids encoding the heavy chain and light chain variable regions of the present invention may be modified. The modifications include additions, deletions, or non-conservative or conservative substitutions of nucleotides.

DNA encoding the antibody may be easily isolated or synthesized using conventional molecular biology techniques (e.g., by using oligonucleotide probes that are capable of binding specifically to DNA encoding the heavy and light chains of the antibody). The nucleic acid is isolated and inserted into a replicable vector for further cloning (DNA amplification) or further expression. Therefore, in another aspect, the present invention relates to a recombinant expression vector comprising the nucleic acid.

As used herein, the term "vector" refers to a means for expressing a target gene in a host cell, and includes plasmid vectors, cosmid vectors, and viral vectors such as bacteriophage vectors, adenovirus vectors, retroviral vectors and adeno-associated viral vectors. Vector components generally include, but are not limited to, one or more of the following components: signal sequences, replication origins, one or more antibiotic resistance marker genes, enhancer elements, promoters, and transcription termination sequences. The nucleic acid encoding the antibody is operably linked to a promoter, a transcription termination sequence and the like. The term "operably linked" refers to functional linkage between a nucleic acid expression control sequence (such as a promoter, signal sequence, or array of transcription factor binding sites) and another nucleic acid sequence, wherein the expression control sequence controls transcription and/or translation of the other nucleic acid sequence. The vector contains antibiotic-resistance genes commonly used in the art as selectable markers, and examples of the selectable markers include genes conferring resistance to ampicillin, gentamycin, carbenicillin, chloramphenicol, streptomycin, kanamycin, geneticin, neomycin and tetracycline.

In another aspect, the present invention relates to an isolated host cell transfected with the recombinant expression vector. The host cell used to produce the antibody of the present invention may be, but is not limited to, a prokaryotic, yeast or higher eukaryotic cell.

Prokaryotic host cells such as *Escherichia coli,* strains of the genus *Bacillus,* such as *Bacillus subtilis* and *Bacillus thuringiensis, Streptomyces, Pseudomonas* (for example, *Pseudomonas putida), Proteus mirabilis* and *Staphylococcus* (for example, *Staphylococcus carnosus*) may be used.

Meanwhile, an example of a host cell line useful as animal cells may be, but is not limited to, MDA-MB-231, A549, A431, HT29, HCC827, SK-MEL-2, SW620, COS-7, BHK, CHO, CHOK1, DXB-11, DG-44, CHO/-DHFR, CV1, COS-7, HEK293, BHK, TM4, VERO, HELA, MDCK, BRL 3A, W138, Hep G2, SK-Hep, MMT, TRI, MRC 5, FS4, 3T3, RIN, PC12, K562, PER.C6, SP2/0, NS-0, U20S, or HT1080.

The host cell may be cultured in various media. Commercially available media may be used as a culture medium without limitation. All other essential supplements known to those skilled in the art may be contained at appropriate concentrations. Culturing conditions, e.g., temperature and pH have already been used with the selected host cells for expression, which will be apparent to those skilled in the art.

For recovery, impurities may be removed, e.g., by centrifugation or ultrafiltration, and the resultant may be purified, for example, by affinity chromatography. Additional purification techniques may be used, such as anion or cation exchange chromatography, hydrophobic interaction chromatography, and hydroxyl apatite chromatography.

The present invention also provides a composition for delivering an active substance into cytosol, comprising the cytosol-penetrating antibody or Fc fragment or Fab fragment thereof.

The active substance may be fused or linked to the antibody. For example, the active substance may be one or more selected from the group consisting of peptides, proteins, toxins, antibodies, antibody fragments, RNA, siRNA, DNA, small molecule drugs, nanoparticles, and liposomes, without being limited thereto.

The proteins may be antibodies, antibody fragments, immunoglobulins, peptides, enzymes, growth factors, cytokines, transcription factors, toxins, antigenic peptides, hormones, transport proteins, motor function proteins, receptors, signaling proteins, storage proteins, membrane proteins, transmembrane proteins, internal proteins, external proteins, secretory proteins, viral proteins, glycoproteins, cleaved proteins, protein complexes, or chemically modified proteins.

The RNA or ribonucleic acid is based on ribose, a kind of pentose, is a kind of nucleic acid consisting of a chain of nucleotides, has a single-stranded structure, and is formed by transcription of a portion of DNA. In one embodiment, the RNA may be selected from the group consisting of rRNA, mRNA, tRNA, miRNA, snRNA, snoRNA, and aRNA, without being limited thereto.

The siRNA (small interfering RNA) is a small RNA interference molecule composed of dsRNA, and functions to bind to and degrade an mRNA having a target sequence. siRNA can be used as a therapeutic agent or to otherwise inhibit expression of a protein translated from a target mRNA by degrading the target mRNA. Due to this activity, it is widely used herein.

The DNA or deoxyribonucleic acid is a kind of nucleic acid, is composed of a backbone chain comprising monosaccharide deoxyriboses linked by phosphate, together with two types of nucleobases (purines and pyrimidines), and stores the genetic information of cells.

The small-molecule drugs refer to organic compounds, inorganic compounds or organometallic compounds that have a molecular weight of less than about 1,000 Da and are active as therapeutic agents against diseases. The small-molecule drugs are widely used herein. The small-molecule drugs that are used in the present invention encompass oligopeptides and other biomolecules having a molecular weight of less than about 1,000 Da.

The nanoparticle refers to particles comprising substances ranging between 1 and 1,000 nm in diameter. The nanoparticle may be a metal nanoparticle, a metal/metal core shell complex consisting of a metal nanoparticle core and a metal shell surrounding the core, a metal/non-metal core shell consisting of a metal nanoparticle core and a non-metal shell surrounding the core, or a non-metal/metal core shell complex consisting of a non-metal nanoparticle core and a metal shell surrounding the core. According to one embodiment, the metal may be selected from gold, silver, copper, aluminum, nickel, palladium, platinum, magnetic iron, and oxides thereof, without being limited thereto, and the non-metal may be selected from silica, polystyrene, latex and acrylate-based materials, without being limited thereto.

The liposomes comprise at least one lipid bilayer enclosing the inner aqueous compartment, which is capable of self-association. Liposomes may be characterized by membrane type and size thereof. Small unilamellar vesicles (SUVs) may have a single membrane and may be 20 to 50 nm in diameter. Large unilamellar vesicles (LUVs) may be at least 50 nm in diameter. Oligolamellar large vesicles and multilamellar large vesicles may have multiple, usually concentric, membrane layers and may be at least 100 nm in diameter. Liposomes with several nonconcentric membranes, i.e., several small vesicles contained within a larger vesicle, are referred to as multivesicular vesicles.

The term "fusion" or "linking" refers to unifying two molecules having the same or different functions or structures, and the methods of fusing may include any physical, chemical or biological method capable of linking the tumor-penetrating peptide to the protein, small-molecule drug, nanoparticle or liposome. Preferably, the fusion may be performed by a linker peptide, wherein the linker peptide may mediate the fusion with the active substance at various positions of the antibody light-chain variable region, antibody, or fragment thereof according to the present invention.

The present invention provides a pharmaceutical composition for preventing or treating cancer comprising the cytosol-penetrating antibody or fragment thereof and an active substance to be delivered into cytosol thereby.

The cancer may be selected from the group consisting of squamous cell carcinoma, small cell lung cancer, non-small cell lung cancer, adenocarcinoma of lung, squamous cell carcinoma of lung, peritoneal cancer, skin cancer, skin or ocular melanoma, rectal cancer, anal cancer, esophageal cancer, small intestine cancer, endocrine cancer, parathyroid cancer, adrenal cancer, soft tissue sarcoma, urethral cancer, chronic or acute leukemia, lymphoma, hepatoma, gastrointestinal cancer, pancreatic cancer, glioblastoma, cervical cancer, ovarian cancer, liver cancer, bladder cancer, liver tumor, breast cancer, colon cancer, colorectal cancer, endometrial cancer or uterine cancer, salivary gland cancer, kidney cancer, liver cancer, prostate cancer, vulva cancer, thyroid cancer, liver cancer, and head and neck cancer.

When the composition is prepared as a pharmaceutical composition for preventing or treating cancer, the composition may contain a pharmaceutically acceptable carrier. The pharmaceutically acceptable carrier contained in the composition is one commonly used in formulation, and examples of the pharmaceutically acceptable carrier include, but are not limited to, lactose, dextrose, sucrose, sorbitol, mannitol, starch, gum acacia, calcium phosphate, alginate, gelatin, calcium silicate, minute crystalline cellulose, polyvinyl pyrrolidone, cellulose, water, syrup, methyl cellulose, methyl hydroxy benzoate, propyl hydroxy benzoate, talc, magnesium stearate and mineral oil. In addition to the above ingredients, the pharmaceutical composition may further contain a lubricant, a wetting agent, a sweetener, a flavoring agent, an emulsifier, a suspending agent, a preservative, etc.

The proper dose of the pharmaceutical composition for preventing or treating cancer may vary depending on various factors such as method for formulation, administration mode, patient's age, weight, gender, and pathological state, diet, administration time, administration route, excretion rate, and reaction sensitivity. The preferred dose of the composition is within the range of 0.001 and 100 mg/kg for an adult. The term "pharmaceutically effective dose" refers to an amount sufficient to prevent or treat cancer or angiogenesis-related diseases.

The composition may be formulated with pharmaceutically acceptable carriers and/or excipients according to a method that can be easily carried out by those skilled in the art, and may be provided in a unit-dose form or enclosed in a multiple-dose vial. Here, the formulation of the pharmaceutical composition may be in the form of a solution, suspension, syrup or emulsion in oily or aqueous medium, or may be in the form of extracts, powders, granules, tablets or capsules, and may further contain a dispersant or a stabilizer. Also, the composition may be administered as an individual therapeutic agent or in combination with other therapeutic agents, and may be administered sequentially or simultaneously with conventional therapeutic agents. Meanwhile, as the composition comprises the antibody or the antigen-binding fragment, it may be formulated into immunoliposome. Liposome containing an antibody may be produced according to a method widely known in the art. The immunoliposome is a lipid composition comprising phosphatidylcholine, cholesterol and polyethyleneglycol-derived phosphatidylethanolamine, and may be produced by a reverse-phase evaporation method. For example, a Fab' fragment of an antibody may be conjugated to liposome through disulfide exchange reaction. Liposome may further comprise a chemotherapeutic agent such as doxorubicin.

The present invention provides a composition for diagnosis of cancer comprising the cytosol-penetrating antibody or fragment thereof and an active substance to be delivered into cytosol thereby.

The term "diagnosis" refers to confirming the presence or characteristic of a pathophysiological condition. Diagnosis in the present invention refers to confirming the onset and progress of cancer.

The antibody in full-length immunoglobulin form and fragment thereof may be linked to a fluorophore for molecular imaging to diagnose cancer through imaging.

The fluorophore for molecular imaging refers to any substance that fluoresces. Preferably, the fluorophore emits red or near-infrared fluorescence, and fluorescence with high quantum yield, without being limited thereto.

Preferably, the fluorophore for molecular imaging is a fluorophore, a fluorescent protein or other substances for imaging, which may bind to a tumor-penetrating peptide that specifically binds to the antibody in full-length immunoglobulin form and fragment thereof, without being limited thereto.

Preferably, the fluorophore is fluorescein, BODYPY, tetramethylrhodamine, Alexa, cyanine, allophycocyanin, or a derivative thereof, without being limited thereto.

Preferably, the fluorescent protein is Dronpa protein, enhanced green fluorescence protein (EGFP), red fluorescent protein (DsRFP), Cy5.5, which is a cyanine fluorophore that emits near-infrared fluorescence, or other fluorescent proteins, without being limited thereto.

Other substances for imaging are preferably ferric oxide, radioactive isotopes, etc., without being limited thereto, and they may be applied to imaging systems such as MR or PET.

### EXAMPLES

Hereinafter, the present invention will be described in more detail through examples. However, these examples are intended to illustrate the present invention and the scope of the present invention is not limited to these examples.

### Example 1. Design of Antibody (in2C1N) Having Endosomal Escape Motif in Heavy Chain Constant Region CH3 Domain

To develop cytosol-penetrating antibodies having an endosomal escape motif in the CH3 domain, eleven CH3 domain variants as shown in Table 1 below were constructed by designing endosomal escape motifs for inducing interaction with the endosomal membrane in the AB loop, EF loop, and C-terminal loop of the CH3 domain of an IgG1 antibody.

The existing cytosol-penetrating antibody inCT has, as endosomal escape motifs, ⁹²WYW⁹⁴ (where the amino acid residue numbers follow the Kabat numbering scheme) in the CDR3 region of VL and ⁹⁶WYW⁹⁸ (Kabat numbering) in the CDR3 region of VH. In addition, the inCT (AAA) antibody, in which all of the endosomal escape motifs WYW are all replaced with alanine (Ala), did not show cytosol-penetrating ability.

Therefore, the present inventors constructed CH3 domain variant antibodies based on the inCT(AAA) antibody having no cytosol-penetrating ability and evaluated whether these antibodies would exhibit cytosol-penetrating ability solely through the endosomal escape motif of the CH3 domain. That is, the present inventors constructed inCT(AAA)-based CH3 domain variant antibodies using a heavy chain comprising the heavy chain variable region (VH) of SEQ ID NO: 14 and the heavy chain constant region (CH1-hinge-CH2-CH3) having each of the CH3 domain variants of SEQ ID NOs: 2 to 12, and a light chain comprising the light chain variable region (VL) of SEQ ID NO: 16 and the light chain constant region (CL) of SEQ ID NO: 17, and evaluated the endosomal escape ability dependent on the endosomal escape motif of the CH3 domain variants.

At this time, the cytosol-penetrating antibody in which a new endosomal escape motif was introduced into the constant region was named in2C, and the CH3 domain variants in which an endosomal escape motif was introduced into CH3 were named in2C1N. (For example, the CH3 domain variant in which endosomal escape motif 1 was introduced into the CH3 domain is in2C11, and the CH3 domain variant in which endosomal escape motif 2 was introduced into the CH3 domain is in2C12). Additionally, antibodies having only arginine and tryptophan mutations without a glutamic acid patch in the endosomal escape motifs of in2C11 and in2C12 were named in2C11^{W/O E} and in2C12^{W/O E}, respectively (W/O E means that the glutamic acid patch was removed from the endosomal escape motif).

In addition, an inCT cytosol-penetrating antibody comprising a heavy chain comprising the heavy chain variable region (VH) of SEQ ID NO: 13 containing an endosomal escape motif (⁹⁶WYW⁹⁸), and the heavy chain constant region (CH1-hinge-CH2-CH3) of SEQ ID NO: 1 having a wild-type IgG1 CH3 domain, and a light chain comprising the light chain variable region (VL) of SEQ ID NO: 15 containing an endosomal escape motif (⁹²WYW⁹⁴) and the wild-type light chain constant region (CL) of SEQ ID NO: 17 was also expressed, purified, and used as a control for evaluating physical properties and cytosol-penetrating ability.

In addition, an inCT(AAA) cytosol-penetrating antibody having no endosomal escape ability and comprising a heavy chain comprising the heavy chain variable region (VH) of SEQ ID NO: 14 having a substitution of alanine (Ala) for the endosomal escape motif (⁹⁶WYW⁹⁸) and the heavy chain constant region (CH1-hinge-CH2-CH3) of SEQ ID NO: 1 having a wild-type IgG1 CH3 domain, and a light chain comprising the light chain variable region (VL) of SEQ ID NO: 16 having a substitution of alanine (Ala) for the endosomal escape motif (⁹²WYW⁹⁴) and the wild-type light chain constant region (CL) of SEQ ID NO: 17 was also expressed, purified, and used as a control for evaluating physical properties and cytosol-penetrating ability.

The endosomal escape motif of the CH3 domain variant was introduced at the surface-exposed amino acid positions of the AB, EF and C-terminal loops of CH3 domain to facilitate interaction with the endosomal membrane and protonation in response to changes in pH environment. To this end, the 3D structure (PDB file) of the wild-type IgG1 antibody Fc region was submitted to the GETAREA web server (https://curie.utmb.edu/getarea.html), and the surface-exposed amino acid positions in the AB, EF, and C-terminal loops of the CH3 domain were identified on the web server using the solvent-accessible surface area (SASA) ratio (%) as shown in Table 3 below.

CH3 domain variants were designed by introducing arginine, tryptophan, and glutamic acid mutations that form endosomal escape motifs at the surface-exposed amino acid positions of the AB, EF and C-terminal loops of the CH3 domain.

Table 1 below shows the amino acid sequence of the wild-type IgG1 CH3 domain and the amino acid sequences of 11 CH3 domain variants having an endosomal escape motif are listed in Table 1 below.

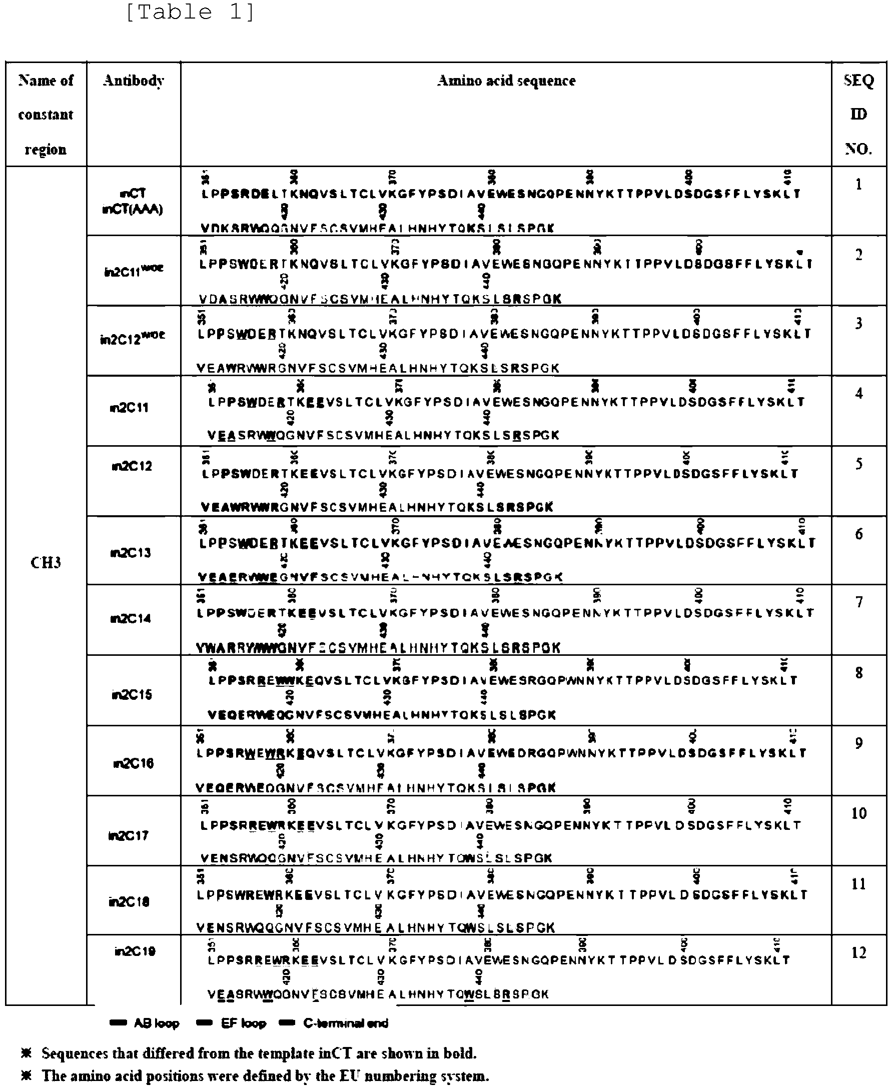

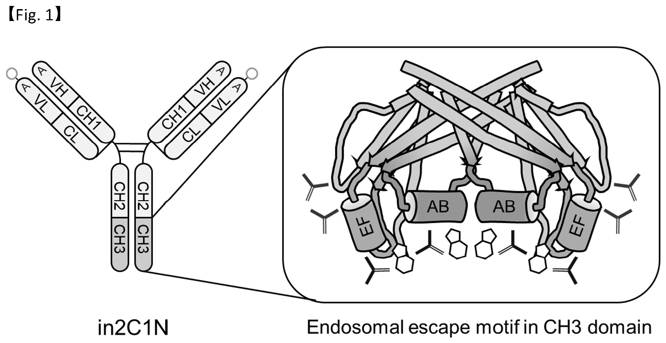

FIG. 1 is a schematic diagram showing a cytosol-penetrating antibody in which endosomal escape motifs are introduced into the AB loop, EF loop and C-terminal loop of the CH3 domain.

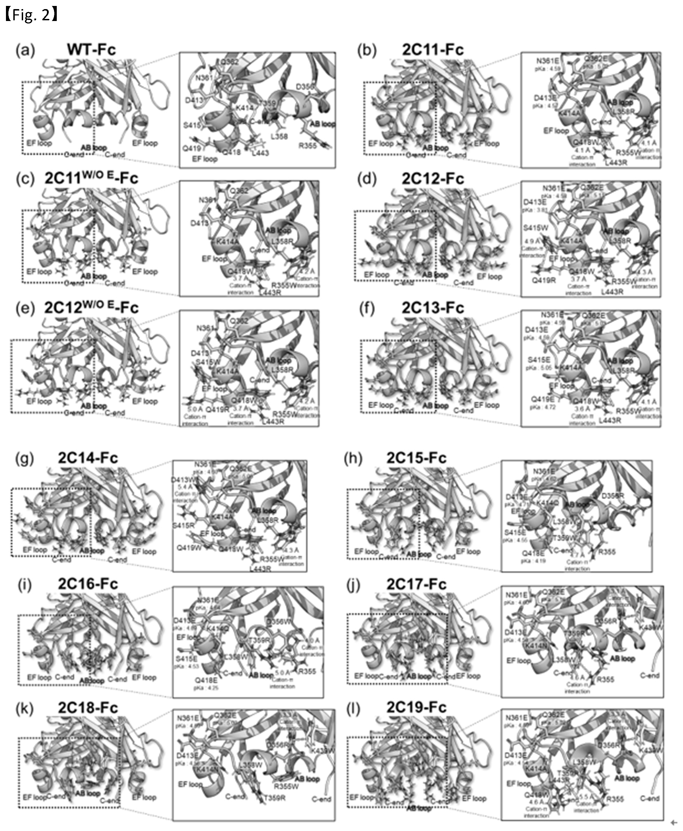

FIG. 2 shows the results of modeling the three-dimensional structures of Fc regions comprising eleven CH3 domain variants, respectively, in which the endosomal escape motifs of the amino acid sequences are introduced into the AB, EF and C-terminal loops of the CH3 domain, using AlphaFold2, and the results of analyzing the intramolecular interaction patterns of the antibodies using the PyMOL molecular visualizer.

To analyze the difference between the 3D structure of the wild-type CH3 domain and the 3D structure of the CH3 domain variant, the root mean square deviation (RMSD) of each of the CH3 domain variants of FIG. 2 with respect to the wild-type CH3 domain variant was calculated. The results are shown in Table 2 below.

**[Table 2]**

| CH3 variants | RMSD (Å) |
|---|---|
| in2C11^{W/O E} | 0.052 |
| in2C12^{W/O E} | 0.050 |
| in2C11 | 0.060 |
| in2C12 | 0.061 |
| in2C13 | 0.090 |
| in2C14 | 0.085 |
| in2C15 | 0.137 |
| in2C16 | 0.133 |
| in2C17 | 2.240 |
| in2C18 | 0.119 |
| in2C19 | 0.175 |

Referring to FIG. 2, it was confirmed that the overall shape and protein folding state of the 3D structures of the CH3 domain variants were similar to those of the 3D structure of the wild-type CH3 domain, and as shown in Table 2 above, it was confirmed that the RMSD values of the CH3 domain variants were 0.2 Å or less, indicating that the structures remained very similar to that of the wild-type CH3 domain. However, in2C17 had a slightly increased RMSD value of 2.24 Å, but the overall folding state of the antibody remained similar.

In addition, the 3D structures of the CH3 domain variants, modeled with AlphaFold2 as shown in FIG. 2, were submitted to GETAREA to calculate the solvent-accessible surface area ratio (%), and the results are shown in Table 3 below. Thereby, it was confirmed that the degree of exposure of each amino acid to the solvent did not change significantly even when the endosomal escape motifs were introduced into the AB, EF, and C-terminal loops of the CH3 domain. However, there were cases where the ratio of the solvent-accessible surface area was decreased from about 50% to about 30% or less, such as with Q418W and K439W.

The following paragraph describes in detail the endosomal escape motifs of CH3 domain variants.

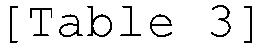

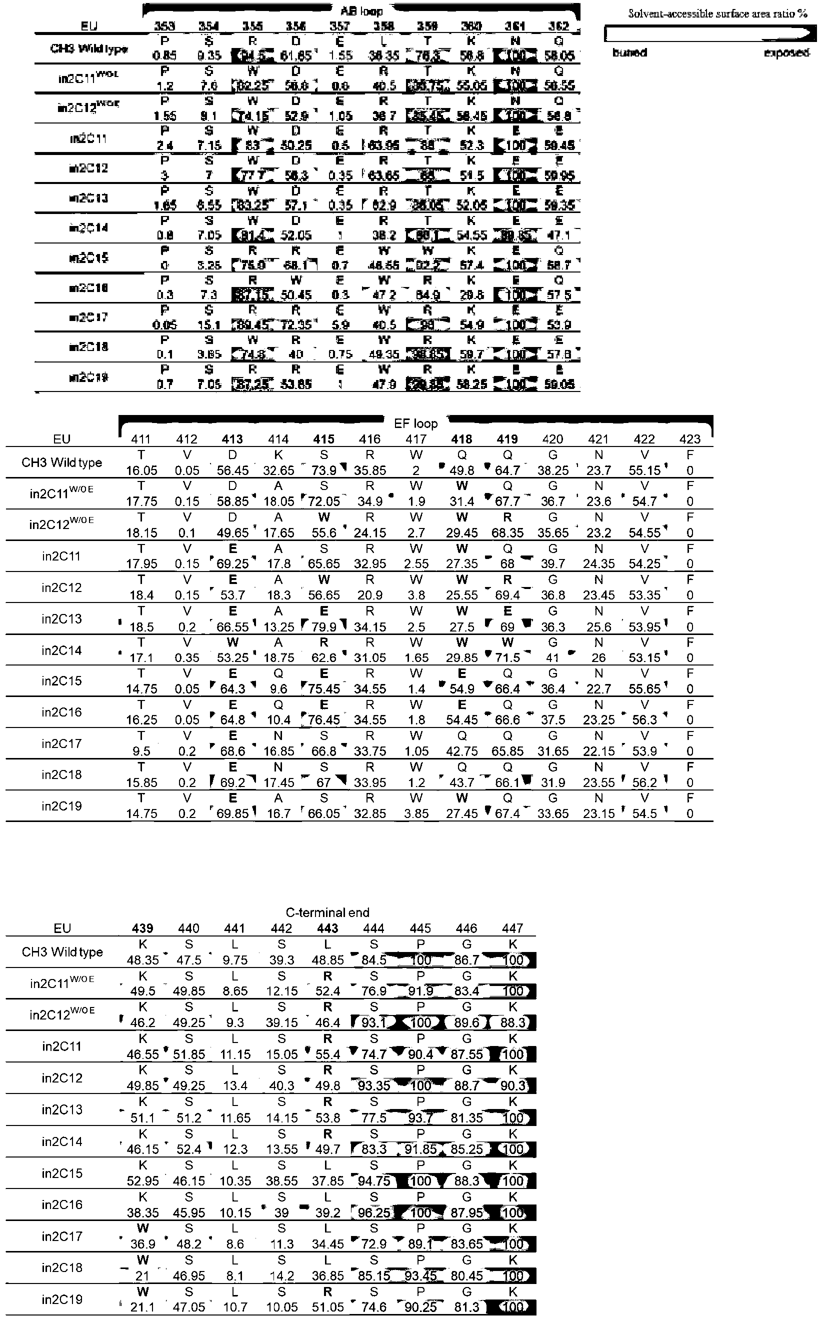

The endosomal escape motif of in2C11 of SEQ ID NO: 4 consists of two tryptophan mutations (R355W and Q418W), two arginine mutations (L358R and L443R), one alanine mutation (K414A), and three glutamic acid mutations (N361E, Q362E, and D413E), which are present in the AB loop, EF loop, and C-terminal loop.

In the case of arginine and tryptophan mutations, as shown in FIG. 2b, L358R and R355W form a cation-π interaction at a distance of 4.1 Å, and L443R and Q418W form a form a cation-π interaction at a distance of 4.1 Å. In this case, K414 was substituted with a smaller alanine (K414A) to prevent steric hindrance between Q418W and W417.

In the case of the glutamic acid mutations, three glutamic acids were clustered in adjacent positions in a patch form to increase the pKₐ of the glutamic acid side-chain carboxyl group. The pKₐ of the glutamic acid side-chain carboxyl group is around 4.2, and in the endosomal environment with a weakly acidic pH of 4.5 to 6.5, the rate of deprotonation is much higher than the rate of protonation, so a large number of glutamic acids remain negatively charged in the endosome. However, when negative charges are clustered, the loss of negative charges by protonation is energetically preferred because it can reduce charge repulsion, so the pKₐ may increase and protonation may be promoted under weakly acidic conditions.

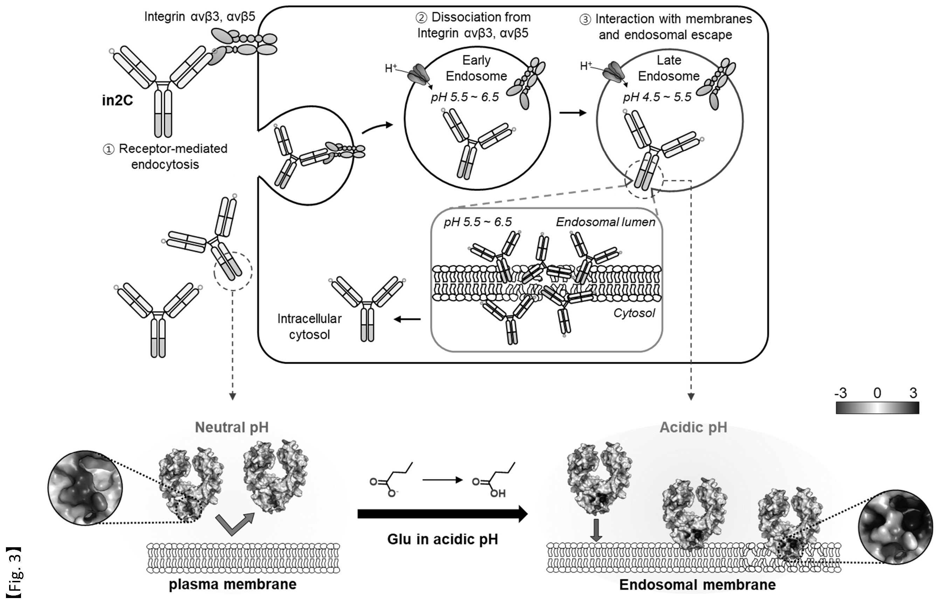

Specifically, the distance between the Cα of D413 and the Cα of Q362 is 4.5 Å, and the distance between the Cα of Q362 and the Cα of N361 is 3.8 Å. When the above three amino acids are substituted with glutamic acid, Q362E, which is located in the middle, is surrounded by amino acids with the same negative charge that are located within a Cα distance 5 Å. When the 3D model structure of in2C11 into which a glutamic acid patch of the three amino acids (N361E, Q362E, D413E) had been introduced was submitted to the PROPKA 3.1 web server (https://biolib.com/bio-utils/propka/) to predict the pKₐ of the glutamic acids, it was confirmed as shown in FIG. 3d that the pKₐ of Q362E was 5.02, which was about 0.8 higher than the original pKₐ of 4.2. According to the Henderson-Hasselbalch equation, the ratio of protonated glutamic acid to deprotonated glutamic acid is about 1:20 when the original pKa is 4.2 in an early endosomal weakly acidic environment (pH 5.5), whereas at a pKₐ of 5.0, which is 0.5 higher, this ratio is about 1:3, meaning that the ratio of protonated glutamic acid has increased 6 times or more. In a late endosomal environment with a weakly acidic pH of 4.5, this ratio is about 2:1, indicating that the ratio of protonated glutamic acid becomes higher. Therefore, it was confirmed that the increase in pKₐ through the glutamic acid patch of glutamic acid mutations clustered in adjacent positions leads to a higher rate of protonation in the endosome, enabling cytosolic penetration specifically into target cells where receptor-mediated endocytosis occurs.

Therefore, in the case of in2C11, four pairs of arginine and tryptophan that form cation-π interactions in the CH3 dimer were clustered at the bottom of the CH3 domain of the antibody Fc region, and a surrounding glutamic acid patch was introduced on both sides of the CH3 domain of the antibody Fc region to confer cytosol-penetrating ability and pH dependence.

As shown in FIG. 2c, 2C11^{W/O E} of SEQ ID NO: 2 is a variant obtained by removing the glutamic acid patch from in2C11 and introducing only arginine and tryptophan mutations. It is a variant constructed to verify the function of the glutamic acid patch with respect to whether the glutamic acid patch introduced into in2C11 can actually prevent non-specific binding and degradation of physical properties caused by arginine and tryptophan and whether it can confer pH dependence.

The endosomal escape motif in in2C13 of SEQ ID NO: 6 consists of two tryptophan mutations (R355W and Q418W), two arginine mutations (L358R and L443R), one alanine mutation (K414A), and five glutamic acid mutations (N361E, Q362E, D413E, S415E, and Q419E), which are present in the AB loop, EF loop, and C-terminal loop. It is a variant constructed to enhance the effect of the glutamic acid patch by introducing two additional glutamic acid (S415E and Q419E) to the side of the α-helix present in the EF loop in in2C11. When the 3D model structure of in2C13, in which a glutamic acid patch consisting of five glutamic acids (N361E, Q362E, D413E, S415E, and Q419E) was introduced, was submitted to the PROPKA 3.1 web server to predict the pKₐ of the glutamic acids, it was confirmed as shown in FIG. 2f that the pKₐ of Q362E was 5.03 and the pKₐ of S415E was 5.05, indicating that the pKₐ increased by about 0.8 at the position surrounded by negative charges.

The endosomal escape motif in in2C12 of SEQ ID NO: 5 consists of three tryptophan mutations (R355W, S415W, and Q418W), three arginine mutations (L358R, Q419R, and L443R), one alanine mutation (K414A) and three glutamic acid mutations (N361E, Q362E, and D413E), which are present in the AB loop, EF loop, and C-terminal loop. in2C12 is a variant constructed to enhance the interaction with the cell membrane by introducing an additional pair of arginine-tryptophan (S415W and Q419R) to the side of the α-helix present in the EF loop of in2C11, thereby introducing arginine and tryptophan to a wider area than in2C11. As shown in FIG. 2d, it was confirmed that the additionally introduced S415W and Q419R formed a cation-π interaction at a distance of 4.9 Å while the cation-π interaction introduced in in2C11 and the increase in pKₐ of Q362E caused by the glutamic acid patch were maintained.

Therefore, in the case of in2C12, six pairs of arginine and tryptophan that form cation-π interactions in the CH3 dimer were clustered at the bottom of the CH3 domain of the antibody Fc region, and a surrounding glutamic acid patch was introduced on both sides of the CH3 domain of the antibody Fc region to confer cytosol-penetrating ability and pH dependence.

As shown in FIG. 2e, in2C12^{W/O E} of SEQ ID NO: 3 is a variant obtained by removing the glutamic acid patch from in2C12 and introducing only arginine and tryptophan mutations. It is a variant constructed to verify the function of the glutamic acid patch with respect to whether the glutamic acid patch introduced into in2C12 can actually prevent non-specific binding and degradation of physical properties caused by arginine and tryptophan and whether it can confer pH dependence.

The endosomal escape motif in in2C14 of SEQ ID NO: 7 consists of four tryptophan mutations (R355W, D413W, Q418W, and Q419W), three arginine mutations (L358R, S415R, and L443R), one alanine mutation (K414A) and two glutamic acid mutations (N361E, and Q362E), which are present in the AB loop, EF loop, and C-terminal loop. in2C14 is a variant in which the arrangement of arginine and tryptophan in in2C12 was altered, and D413E in in2C11 was replaced with tryptophan (D413W) and S415W was replaced with arginine (S415R) to form an arginine-tryptophan pair of S415R and D413W, thereby inducing interaction with the cell membrane. As shown in FIG. 2g, it was confirmed that D413W and S415R formed a cation-π interaction at a distance of 5.4 Å, and the pKₐ of Q362E was 5.05 despite the presence of three glutamic acid mutations, indicating that the increase in pKₐ was maintained.

Therefore, in the case of in2C14, six pairs of arginine and tryptophan that form cation-π interactions in the CH3 dimer were clustered at the bottom of the CH3 domain of the antibody Fc region, and a surrounding glutamic acid patch was introduced on both sides of the CH3 domain of the antibody Fc region to confer cytosol-penetrating ability and pH dependence.

In2C15 of SEQ ID NO: 8 and in2C16 of SEQ ID NO: 9 are variants that have an endosomal escape motif constructed according to the same logic as in2C11, but have different positions of arginine, tryptophan, and glutamic acid.

The endosomal escape motif in in2C15 consists of two tryptophan mutations (L358W, and T359W), one arginine mutation (D356R), one glutamine mutation (K414Q), and four glutamic acid mutations (N361E, D413E, S415E, and Q418E), which are present in the AB loop, EF loop, and C-terminal loop. As shown in FIG. 2h, the cation-π interaction formed between R355W and L358R in in2C11 was changed to the cation-π interaction between R355 and L358W. In addition, when the 3D model structure of in2C15 into which a glutamic acid patch consisting of four glutamic acids (N361E, D413E, S415E, and Q418E) has been introduced was submitted to the PROPKA 3.1 web server to predict the pKₐ of the glutamic acids, it was confirmed as shown in FIG. 3h that the pKₐ of D413E was 4.7, which is about 0.5 higher than the original pKₐ of 4.2. In addition, to prevent glutamic acid from forming a salt bridge with lysine (K414), which lowers the pKₐ of the glutamic acid, glutamine (K414Q), which has a side chain length similar to glutamic acid but no charge, was introduced to remove the positive charge.

The endosomal escape motif in in2C16 consists of two tryptophan mutations (D356W, and L358W), one arginine mutation (T359R), one glutamine mutation (K414Q), and four glutamic acid mutations (N361E, D413E, S415E, and Q418E), which are present in the AB loop, EF loop, and C-terminal loop. As shown in FIG. 2i, a cation-π interaction between R355 and D356W and a cation-π interaction between T359R and D356W are formed. The glutamic acid patch is identical to that in in2C15, and the positive charge at position 414 was removed by introducing K414Q to prevent glutamic acid from forming a salt bridge with lysine (K414).

Therefore, in the case of in2C15 and in2C16, four pairs of arginine and tryptophan that form cation-π interactions in the CH3 dimer were clustered at the bottom of the CH3 domain of the antibody Fc region, and a glutamic acid patch was constructed in a different form from those in in2C11, in2C12, in2C13, and in2C14 and introduced on both sides of the CH3 domain of the antibody Fc region to confer cytosol-penetrating ability and pH dependence.

In2C17 of SEQ ID NO: 10 and in2C18 of SEQ ID NO: 11 are variants in which arginine, tryptophan, and glutamic acid are arranged with reference to in2C11, in2C15, and in2C16, which show no non-specific binding ability and have excellent yield and/or endosomal escape ability.

The endosomal escape motif in in2C17 consists of two tryptophan mutations (L358W, and K439W), two arginine mutations (D356R, and T359R), one asparagine mutation (K414N), and three glutamic acid mutations (N361E, Q362E, and D413E), which are present in the AB loop, EF loop, and C-terminal loop.

In the case of in2C17, the arginine-tryptophan pair retained the R355-L358W of in2C15 and in2C16, and D356R-K439W was newly introduced, replacing the existing salt bridge at the interface between the CH3 domains with a cation-π interaction. When wild-type CH3 domains form a homodimer, various interactions occur at the interface between the CH3 domains. As shown in FIG. 2j, D356 of the wild-type CH3 domain and K439 of the opposite CH3 domain form a salt bridge at a distance of 4.7 Å. When D356R and K439W were introduced to change the salt bridge into a cation-π interaction and the 3D model structure was examined, it was confirmed as shown in FIG. 3k that a cation-π interaction was formed between D356R and K439W at a distance of 3.1 Å.

When the same glutamic acid patch (N361E, Q362E, and D413E) as in in2C11 was introduced and the 3D model structure of in2C17 was submitted to the PROPKA 3.1 web server to predict the pKₐ of the glutamic acids, it was confirmed as shown in FIG. 3k that the pKₐ of Q362E was 5.16, which is about 0.9 higher than the original pKₐ of glutamic acid (4.2). In addition, glutamine (K414Q), which was introduced to prevent glutamic acid from forming a salt bridge with lysine (K414), was changed to asparagine (N), which is smaller in size.

The endosomal escape motif in in2C18 of SEQ ID NO: 11 consists of three tryptophan mutations (R355W, L358W, and K439W), two arginine mutations (D356R, and T359R), one asparagine mutation (K414N), and three glutamic acid mutations (N361E, Q362E, and D413E), which are present in the AB loop, EF loop, and C-terminal loop. D356R and K439W form a cation-π interaction at a distance of 3.3 Å. When the pKₐ values of the glutamic acids were predicted using the PROPKA 3.1 web server in the same way as described above, it was confirmed as shown in FIG. 2k that the pKₐ of Q362E was 5.03, which is about 0.8 higher than the original pKₐ of glutamic acid (4.2).

The endosomal escape motif in in2C19 of SEQ ID NO: 12 consists of three tryptophan mutations (L358W, Q418W, and K439W), three arginine mutations (D356R, T359R, and L443R), one alanine mutation (K414A), and three glutamic acid mutations (N361E, Q362E, and D413E), which are present in the AB loop, EF loop, and C-terminal loop. in2C19 is a variant formed by combining in2C11 and in2C17. As shown in FIG. 2l, a cation-π interaction was formed between R355 and T359R at 5.5 Å, and L443R and Q418W of in2C11 were introduced to induce a cation-π interaction between the two amino acids at a distance of 4.6 Å. At this time, lysine was substituted with alanine (K414A) to prevent steric hindrance caused by Q418W. In addition, D356R and K439W of in2C17 were introduced to induce a cation-π interaction between the two amino acids at a distance of 3.3 Å. The glutamic patch was identical to that in in2C11, and when the pKₐ of the glutamic acids was predicted using the PROPKA 3.1 web server in the same way as described above, it was confirmed that the pKₐ of Q362E increased to 5.02 as shown in FIG. 2l.

Therefore, in the case of in2C19, six pairs of arginine and tryptophan that form cation-π interactions in the CH3 dimer were clustered at the bottom of the CH3 domain of the antibody Fc region, and a glutamic acid patch identical to that in in2C11 was introduced on both sides of the CH3 domain of the antibody Fc region to confer cytosol-penetrating ability and pH dependence.

FIG. 3 is a schematic diagram showing the expected mechanism by which a cytosol-penetrating antibody having an endosomal escape motif in the CH3 domain is endocytosed into a cell and then localizes in the cytosol, and the design strategy of the endosomal escape motif to confer pH dependence to the cytosol-penetrating antibody.

The cytosol-penetrating antibody is endocytosed specifically into a target cell that overexpress integrin αvβ3/αvβ5 receptors through an integrin αvβ3/αvβ5 receptor-specific in4 cyclic peptide fused to the N-terminus of the light chain. After endocytosis, the cytosol-penetrating antibody dissociates from the receptors under the weakly acidic and reducing conditions of the endosome, and interacts with the endosomal membrane through arginine and tryptophan of the endosomal escape motif introduced into the CH3 domain and/or CL domain, thereby destabilizing the membrane and, as a result, collapsing the endosomal membrane and escaping from the endosome into the cytosol. Therefore, the cytosol-penetrating antibody does not go to the Golgi apparatus or lysosome.

At this time, the glutamic acid patch present around arginine and tryptophan in the endosomal escape motif is electrostatically repelled by the negatively charged cell membrane in a physiological environment with pH 7.4, so the interaction with the cell membrane is inhibited. However, in the late endosomal environment where the pH is lowered to 4.5 to 5.5, the carboxyl group (COO-) of the glutamic acid side chain is protonated and loses its negative charge, so the electrostatic repulsion with the cell membrane is reduced, and the hydrophobic interaction between the protonated glutamic acid and the hydrophobic tail region of the cell membrane phospholipid is enhanced, thereby promoting interaction with the cell membrane.

Example 2. Confirmation of Cytosolic Localization and Evaluation of Cytosol-Penetration Ability of Cytosol-Penetrating Antibody through Enhanced Split Green Fluorescent Protein Complementation.

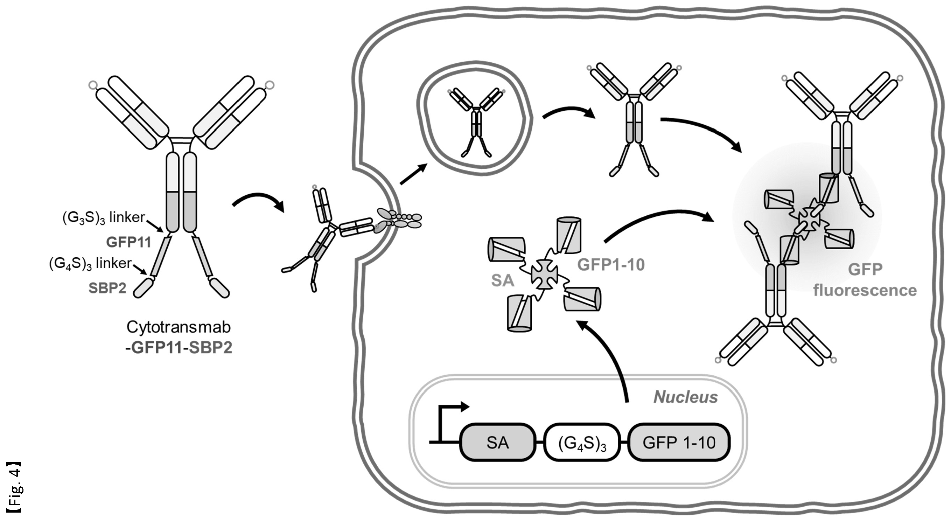

To confirm that the constructed CH3 domain variants localize in the cytosol and to quantitatively compare and evaluate the cytosol-penetrating ability between the variants, an enhanced split green fluorescent protein complementation system (Korean Patent No. KR10-1522954, Biochem Biophys Res Commun, 2015, 467(4):771-777) was used. FIG. 4 is a schematic diagram illustrating the process in which GFP fluorescence generated by complementation of enhanced split green fluorescent protein is observed when a cytosol-penetrating antibody localizes in the cytosol. Observed GFP fluorescence is evidence that the cytosol-penetrating antibody localizes in the cytosol, and the cytosol-penetrating ability of each variant can be evaluated through quantitative analysis of GFP fluorescence. Therefore, all the cytosol-penetrating antibodies in full-length IgG form having cytosol-penetrating ability in the CH3 domain were constructed in a form in which a GFP11-SBP2 fragment was fused to the C-terminus of the heavy chain, and the constructed antibodies were evaluated.

### Example 3. Expression and Purification of Cytosol-Penetrating Antibody (in2C1N-GFP11-SBP2) Which Has Endosomal Escape Motif in CH3 Domain and to Which Cyclic Peptide and GFP11-SBP2 That Target Membrane Protein Receptor Overexpressed on Tumor Cell Surface Have Been Fused.

To identify the biophysical properties of CH3 domain variants and to evaluate the cytosol-penetrating ability through an enhanced split green fluorescent protein complementation system, the present inventors constructed the CH3 domain variant in2C1N-GFP11-SBP2 in which GFP11-SBP2 is fused to the C-terminus of the antibody heavy chain.

At this time, a cyclic peptide (in4, DGVRQCRGDCFDGPL) (Science Advances, 2020, 6(3):eaay2174) that targets integrin αvβ3/αvβ5, membrane protein receptors overexpressed on the surface of tumor cells/tissues, was fused to the N-terminus of the light chain of the cytosol-penetrating antibody to induce endocytosis mediated by the tumor cell/tissue-specific receptors.

Specifically, to construct a heavy chain expression vector for producing a full-length IgG monoclonal antibody, a DNA, which has a secretory signal peptide-encoding DNA fused to the 5' end and encodes a heavy chain comprising a heavy chain comprising an antibody heavy chain variable region (VH, SEQ ID NO: 14) and a heavy chain constant region of CH1-hinge-CH2-CH3 having each of CH3 domain variants (SEQ ID NOs: 2 to 12), was cloned into the pcDNA3.4 vector by NotI/HindIII. At this time, to construct a cytosol-penetrating antibody fused with GFP11-SBP2, GFP11- GFP11-(G₄S)₃-SBP2 was fused to the C-terminus of the heavy chain using three GGGS linkers. In addition, to construct a vector expressing the light chain, a DNA, which has a secretory signal peptide fused to the 5' end and encodes a light chain comprising a antibody light chain variable region (VL, SEQ NO: 16) and a light chain constant region (CL, SEQ No. 17), was cloned into the pcDNA3.4 vector by NotI/HindIII. The variable region sequences of inCT and inCT(AAA) of SEQ ID NOs: 13 to 16 are shown in Table 4 below.

A heavy chain expression vector comprising the VH of SEQ ID NO: 14 and each of the CH3 domain variants of SEQ ID NO: 2 to 12 and a common light chain expression vector comprising the VL of SEQ ID NO: 16 and the light chain constant region sequence of SEQ ID NO: 17 were transiently co-transfected, and the cytosol-penetrating antibody was expressed and purified.

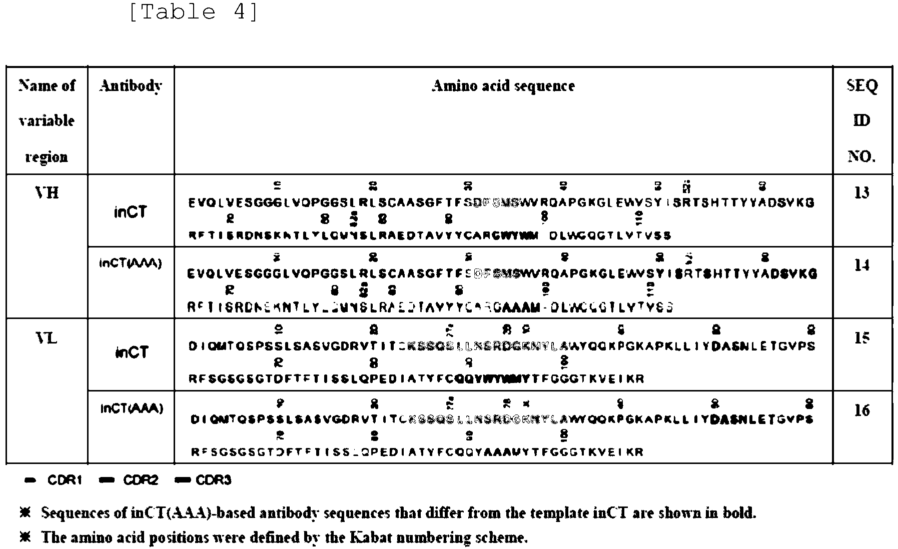

HEK293-F cells (Invitrogen) growing in suspension in serum-free FreeStyle 293 expression medium (Invitrogen) in a shaking flask were transfected with a mixture of the plasmid and polyethyleneimine (PEI) (Polyscience, 24765-1). For 200 mL transfection in a shaking flask (Corning), HEK293-F cells were seeded in 100 mL of medium at a density of 2.0 × 10⁶ cells/mL and cultured at 130 rpm, 8% CO₂, and 37°C. To produce antibodies, suitable heavy and light chain plasmids (125 µg of the heavy chain and 125 µg of the light chain, for a total of 250 µg (2.5 µg/mL)) were diluted in 10 mL of FreeStyle 293 expression medium, filtered, and mixed with 10 mL of medium in which 750 µg (7.5 µg/mL) of PEI was diluted, and the medium mixture was incubated at room temperature for 10 minutes. Thereafter, the incubated medium mixture was added to the cells previously seeded in 100 mL, followed by culture for 4 hours at 130 rpm, 8% CO₂, and 37 °C, and then the remaining 100 mL of FreeStyle 293 expression medium was added thereto, followed by culture for 6 days.

Protein was purified from the collected cell culture supernatant with reference to the standard protocol. The antibody was applied to a Protein A Sepharose column (GE healthcare) and washed with PBS (pH 7.4). The antibody was eluted at pH 3.0 using 0.1 M glycine buffer, and the sample was immediately neutralized using 1 M Tris buffer (pH 9.0). The eluted antibody fraction was concentrated by exchanging the buffer with histidine buffer (25 mM histidine, 150 mM NaCl, pH 6.5) via dialysis. After purification, the protein concentration was quantified using a BCA protein assay kit (Pierce, 23225).

**[Table 5]**

| GFP11-SBP2 fused antibodies | Size (kDa) | Purification yield (mg/L of transfected cells) | Relative yield (%) |
|---|---|---|---|
| inCT | 162.9 | 40.6±2.6 | 84.5 |
| inCT(AAA) | 162.1 | 50.4±3.8 | 100 |
| in2C11^{W/OE} | 162.3 | 40.8±1.2 | 88 |
| in2C12^{W/OE} | 162.6 | 40.7±4.4 | 86 |
| in2C11 | 162.7 | 40.5±6.3 | 82.5 |
| in2C12 | 162.6 | 40.3±6.3 | 79 |
| in2C13 | 162.4 | 40.0±8.3 | 73 |
| in2C14 | 162.7 | 30.9±11.5 | 71 |
| in2C15 | 162.6 | 30.3 | 60 |
| in2C16 | 162.6 | 40.8 | 89 |
| in2C17 | 162.6 | 52.9±3.5 | 97 |
| in2C18 | 162.6 | 29.9±3.7 | 55 |
| in2C19 | 162.7 | 32.4±7.5 | 60 |

The production yields of the CH3 domain variants are shown in Table 5 above. When compared to the wild type inCT(AAA)-GFP11-SBP2, there are cases where the yield decreased by about 55%, such as in2C18-GFP11-SBP2, but it was confirmed that all the CH3 domain variants had a good production yield of 30 mg or more per liter.

The cytosol-penetrating antibodies (in2C1N-GFP11-SBP2), which have an endosomal escape motif in the CH3 domain and to which a cyclic peptide and GFP11-SBP2 that target a membrane protein receptor overexpressed on the tumor cell surface have been fused, are hereinafter referred to as in2C1N. For example, in2C11-GFP11-SBP2 is in2C11.

### Example 4. Evaluation of Physical Properties of Cytosol-Penetrating Antibody (in2C1N) Having Endosomal Escape Motif in Heavy Chain Constant Region CH3 Domain.

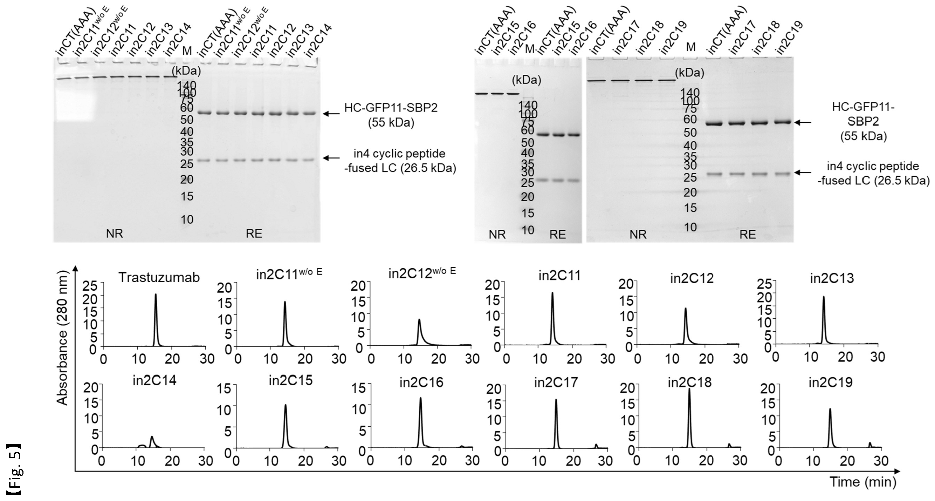

FIG. 5 shows the results of analyzing eleven antibodies having an endosomal escape motif in the CH3 domain by 12% SDS-PAGE under reducing or non-reducing conditions after purification together with the control antibody inCT or inCT(AAA) and the results of analyzing the antibodies by size exclusion chromatography. Specifically, it was confirmed that all of the eleven CH3 domain variants had a molecular weight of about 162 to 163 kDa under non-reducing conditions, similar to the control antibody, and that under reducing conditions, the molecular weight of the heavy chain was about 55 kDa (antibody heavy chain: about 50 kDa, and GFP11-SBP2 fragment: about 5 kDa) and the molecular weight of the light chain was about 26.5 kDa (antibody light chain: about 25 kDa, and in4 cyclic peptide: 1.5 kDa). This shows that the expressed and purified cytosol-penetrating antibody to which the in4 cyclic peptide and GFP11-SBP2 have been fused exists as a monomer in solution and does not form a dimer or oligomer through an unnatural disulfide bond.

The graph at the bottom of FIG. 5 shows the mAU values at 280 nm, measured using size exclusion chromatography (Superdex 200 Increase 10/300GL, Cytiva) for the eleven antibodies having an endosomal escape motif in the CH3 domain. Specifically, 20 µL of each of the eleven CH3 domain variants and control inCT (AAA) and/or Trastuzumab was prepared at a concentration of 1 mg/mL and then placed in an insert, thereby preparing samples. PBS (10 mM Na₂HPO₄, 137 mM NaCl, 2.7 mM KCl, 1.8 mM KH₂PO₄, pH 7.4) buffer was flowed at a flow rate of 0.75 mL/min and the pressure and mAU value at 280 nm of the sample were allowed to stabilize. Next, 10 µL of each of the prepared samples was loaded at the same flow rate and the mAU value at 280 nm was measured for 40 minutes. Single peaks without oligomers or tailing were observed for 8 variants except in2C12^{W/O E}, in2C12 and in2C14, as in the control antibody inCT(AAA) or Trastuzumab. Thereby, it was confirmed that the remaining 8 variants, excluding in2C12^{W/O E}, in2C12 and in2C14, have physical properties similar to the control wild-type antibody.

### Example 5. Evaluation of Non-Specific Binding of Cytosol-Penetrating Antibody (in2C1N) Having Endosomal Escape Motif in Heavy Chain Constant Region CH3 Domain.

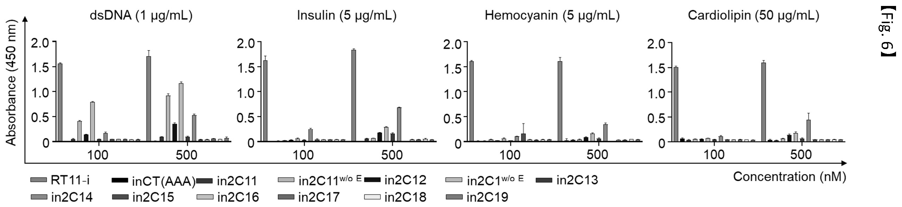

FIG. 6 shows the results of performing ELISA on four antigens, dsDNA, insulin, keyhole limpet hemocyanin (KLH) and cardiolipin, which allow the evaluation of the non-specific binding of antibodies, to evaluate the non-specific binding of the cytosol-penetrating antibodies having an endosomal escape motif in the CH3 domain.

Specifically, 50 µL of each of four antigens (1 µg/mL dsDNA (Sigma, D4522), 5 µg/mL insulin (Sigma, I9278), 5 µg/mL keyhole limpet hemocyanin (Sigma, H8283), and 10 µg/mL cardiolipin (Sigma, C0563)) diluted in PBS (pH 7.4) was dispensed into each well of a 96-well EIA/RIA plate and adsorbed onto the plate surface at room temperature for 1 hour. Then, the plate was washed by repeating a process of dispensing 100 µL of PBS-T solution (0.1% Tween 20 in PBS) into each well of the plate and shaking off, three times. Then, to block the surface to which no antigen was adsorbed, 50 µL of blocking solution (2% skim milk in PBS-T) was dispensed into each well and adsorbed at room temperature for 1 hour. Next, the plate was washed by repeating three times a process of dispensing 100 µL of PBS-T solution into each well, and then 50 µL of each antibody diluted in blocking solution to a concentration of 100 nM or 500 nM was dispensed into each well and incubated at room temperature for 1 hour. RT11-i antibody is an antibody having a large number of positively charged amino acids in CDR-L1 and has the ability to bind non-specifically to various antigens including HSPG, so it was used as a positive control in this experiment. As a negative control, inCT(AAA) with a wild-type IgG1 CH3 domain was used. Thereafter, the plate was washed by repeating three times a process of dispensing 100 µL of PBS-T solution into each well and shaking off, and then 50 µL of HRP-conjugated anti-human monoclonal antibody (Invitrogen, 31413) diluted 1:8,000 in blocking solution was dispensed into each well and incubated at room temperature for 1 hour. Finally, the plate was washed by repeating three times a process of dispensing 100 µL of PBS-T solution into each well and shaking off, and then 25 µL of TMB ELISA solution (Thermo Scientific^{™}, 34028), which provides a substrate for HRP, was dispensed into each well and incubated for 2 minutes. After 2 minutes, 25 µL of 2 N sulfuric acid solution (SAMCHUN, S2129) was dispensed into each well to stop the reaction, and the absorbance was measured at 450 nm.

In the above experiment, when the antibody at a concentration of 100 nM shows an absorbance of 0.05 or higher at 450 nm for at least one of the four antigens, it is determined to have non-specific binding. As a result of the experiment, in2C11^{+W/O E}, in2C12^{W/O E}, in2C12, and in2C14 showed non-specific binding to the dsDNA antigen at 100 nM.

In this case, in2C11^{W/O E} and in2C12^{W/O E} are variants obtained by removing the glutamic acid patches from in2C11 and in2C12, respectively, and introducing only arginine and tryptophan mutations, in order to confirm the function of the glutamic acid patches. As shown in FIG. 6, when non-specific binding to dsDNA was evaluated in2C11^{W/O E} and in2C12^{W/O E} without a glutamic acid patch showed high non-specific binding to dsDNA, but in2C11 and in2C12 with a glutamic acid patch showed significantly reduced non-specific binding to dsDNA. Thereby, it was confirmed that the glutamic acid patch actually reduces non-specific binding that can be induced by arginine-tryptophan. As a result, it was confirmed that four variants, in2C11^{W/O E}, in2C12^{W/O E}, in2C12, and in2C14, had non-specific binding to the four antigens. The remaining seven variants, excluding the four variants, were selected as candidates.

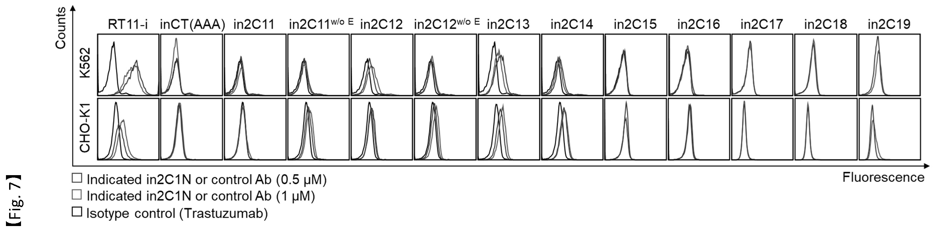

FIG. 7 shows the results of performing flow cytometry analysis to evaluate the non-specific binding of the cytosol-penetrating antibodies having an endosomal escape motif in the CH3 domain to the cell membrane. Specifically, it was checked whether the antibodies would show non-specific binding to the cell membrane in two cell lines (K-562, and CHO-K1) that do not express integrin αvβ3/αvβ5. The cell lines were each cultured in RPMI medium containing 10% FBS and 1% ABAM at 37°C under 5% CO₂, and 1 × 10⁵ cells per sample were prepared in PBS-F solution (pH 7.4 PBS, 1% BSA). Each cell line was treated with each of the CH3 domain variants at a concentration of 500 nM and 1 µM for 1 hour at 4°C, along with the wild-type negative control inCT or inCT(AAA) and the positive control RT11-i. After washing with PBS-F solution, the cells were treated with Alexa Fluor 647-conjugated anti-human IgG (H+L) antibody (Invitrogen, A21445) at 4°C for 30 minutes. After washing with PBS-F solution, the cells were analyzed using a flow cytometer (FACSCalibur, BD). The experimental results showed that in2C11^{W/O E}, in2C12^{W/O E}, in2C12, in2C13, and in2C14 showed significantly higher non-specific binding to the cell surfaces of K-562 and CHO-K1 than the wild-type negative control. Thus, six variants excluding these variants were selected as candidates.

### Example 6. Construction of Reporter Cell Lines for Evaluating Cytosol-Penetrating Ability of Cytosol-Penetrating Antibodies through Complementation of Enhanced Split Green Fluorescent Protein.

An assay system was constructed to quantitatively evaluate the cytosol-penetrating ability of the cytosol-penetrating antibodies using complementation of enhanced split green fluorescent protein. Specifically, MDA-MB-231 and SW480-SA-GFP1-10 cell lines that stably express SA-GFP1-10 were constructed using MDA-MB-231 and SW480 expressing integrin αvβ3/αvβ5. First, to produce lentiviral particles, 8 × 10⁵ HEK293T cells per well were cultured in DMEM containing 10% FBS and 1% ABAM in a 6-well plate for 16 hours 37°C under 5% CO₂. After 16 hours, the medium was replaced with 2 mL of Opti-MEM medium. The cells were transfected with lentiviral packaging plasmid (pMDLg/Prre (Addgene, 12251), pRSV-Rev (Addgene, 12253), pMD2.G (Addgene, 12259)) and lentiviral transfer plasmids (pLJM-SA-GFP1-10) using Lipofectamine^{™} 3000 transfection reagent (Invitrogen, L3000001) according to the methods provided by the manufacturers. After 6 hours, the medium was replaced with 2 mL of DMEM and then the cells were cultured for 48 hours to produce viral particles. After obtaining the supernatant from each well, the supernatant was centrifuged at 500 g for 10 minutes at room temperature to remove cell debris. The supernatant was filtered through a 0.45 µm filter, and then the viral particles were concentrated using a Lenti-X concentrator (TAKARA, 631231).

To transduce MDA-MB-231 cells with the lentiviral particles, 8 × 10⁵ MDA-MB-231 or SW480 cells were placed in each well of 6-well plates with 2 mL of RPMI medium containing 10% FBS and 1% ABAM and cultured for 16 hours at 37°C under 5% CO₂. To improve transduction efficiency, polybrene infection/transfection reagent (Sigma, TR-1003) diluted in RPMI medium at 12 µg/mL was prepared, and the concentrated viral particles were diluted in this medium and used to infect MDA-MB-231 or SW480 cells. The 6-well plate was centrifuged at 1,200 g for 60 minutes at 30°C and then incubated 37°C under 5% CO₂. After 6 hours, the medium was replaced with RPMI medium containing 10% FBS and 1% ABAM and the plate was incubated for 48 hours. Thereafter, cells were collected from each well and cultured in RPMI medium containing 1 µg/mL puromycin and 10% FBS, and only the cells containing the target plasmid were selected. Single cell lines that stably express SA-GFP1-10 were selected from the constructed cell lines through single cell selection.

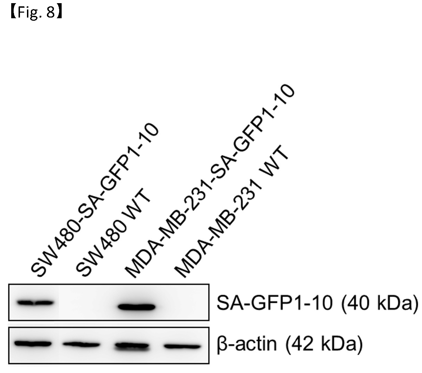

FIG. 8 shows the results of performing Western blot analysis to confirm intracellular SA-GFP1-10 expression in the selected single cell lines MDA-MB-231-SA-GFP1-10 and SW480-SA-GFP1-10 cells. To obtain cell lysates, lysis buffer (10 mM Tris-HCl pH 7.4, 100 mM NaCl, 1% SDS, 1 mM EDTA, Inhibitor cocktail (Sigma, 78440)) was added to the cells which were then lysed by boiling at 95°C for 20 minutes, and the concentration of protein in the lysates was quantified using a BCA protein assay kit (Pierce, 23225). A gel loaded with 15 µg of each cell lysate and subjected to SDS-PAGE was transferred to a PVDF membrane which was then treated with a blocking solution (4% BSA in 0.1% TBS-T) at room temperature for 1 hour. Then, as a primary antibody, an anti-flag M2 mouse monoclonal antibody (Sigma, F1804) that recognizes a flag tag fused to SA-GFP1-10 was diluted 1:1,000 in the blocking solution and the membrane was treated with the dilution at room temperature for 3 hours. As a primary antibody to confirm β-actin, anti-β-actin monoclonal antibody (Santa Cruz, 69879) was diluted 1:1,000 in blocking solution and the membrane was treated with the dilution at room temperature for 2 hours. After washing twice TBS-T solution (0.1% Tween 20 in TBS) for 10 minutes each time, the membrane was treated with anti-mouse IgG-HRP-conjugated polyclonal antibody (Sigma, A9044) diluted 1:4,000 in 0.1% TBS-T solution at room temperature for 1 hour. After washing twice with 0.1% TBS-T solution for 10 minutes each time, Target bands were detected using West-Q Femto Clean ECL solution (GenDEPOT, W3680-010). Analysis was performed using ImageQuant LAS4000 mini (GE Healthcare). Target bands were detected in SW480-SA-GFP1-10 single cells (lane 1) and MDA-MB-231-SA-GFP1-10 single cells (lane 3), and no bands were detected in the negative control wild-type SW480 (lane 2) and MDA-MB-231 (lane 4) cells. Thereby, it was confirmed that SA-GFP1-10 fragment protein was expressed in the constructed and selected single cell lines. The cytosol-penetrating ability of the CH3 domain variants was evaluated using two cell lines, MDA-MB-231-SA-GFP1-10 and SW480-SA-GFP1-10.

### Example 7. Evaluation of Cytosol-Penetrating Ability of Cytosol-Penetrating Antibody (in2C1N) Having Endosomal Escape Motif in Heavy Chain Constant Region CH3 Domain.

FIG. 9 shows the results of observing and quantifying GFP fluorescence generated by complementation of enhanced split green fluorescent protein using a live cell imaging device, Lionheart Fx (Biotek), to evaluate the cytosol-penetrating ability of the cytosol-penetrating antibodies (in2C1N) having an endosomal escape motif in the CH3 domain.

Specifically, the transformed MDA-MB-231-SA-GFP1-10 or SW480-SA-GFP1-10 cell line that stably expresses SA-GFP1-10 was cultured in RPMI medium containing 10% FBS and 1% ABAM. 100 µL of 2 × 10³ (MDA-MB-231) or 4 × 10³ (SW480) cells were seeded into each well of a 96-well black plate (Greiner, 655090) and cultured for 48 hours at 37°C under 5% CO₂ and. After removing the supernatant, each well was treated with each of the positive control inCT, the negative control inCT(AAA), and the CH3 domain variants at a concentration of 500 nM at 37°C for 12 hours. Thereafter, to reduce fluorescence signal interference caused by FBS and phenol red contained in the existing medium, the medium in each well was completely removed, and then the medium was replaced by dispensing 50 µL of FluoroBrite^{™} DMEM (Gibco, A1896701), a medium suitable for fluorescence imaging, into each well. Images of living cells were analyzed in real time using the bright field channel and GFP channel of Lionheart Fx. Intracellular fluorescence intensity by each of the CH3 domain variants was quantified using the Gen5 image analysis program (Biotek) by dividing the sum of the fluorescence intensities in the total cells in the image by the total number of the cells to determine the mean GFP value, and was expressed as a relative value to the quantified value of fluorescence intensity of the group treated with the positive control inCT.

FIG. 9a shows the results of evaluating the cytosol-penetrating ability of the six variants (in2C11, in2C15, in2C16, in2C17, in2C18, and in2C19) compared to inCT at a concentration of 500 nM using MDA-MB-231-SA-GFP1-10 cells. The results of quantification indicated that in2C11 and in2C19 exhibited about 2.2-fold and 2.4-fold increased cell-based fluorescence intensities, respectively, compared to inCT, and exhibited better cytosol-penetrating ability than the other variants.

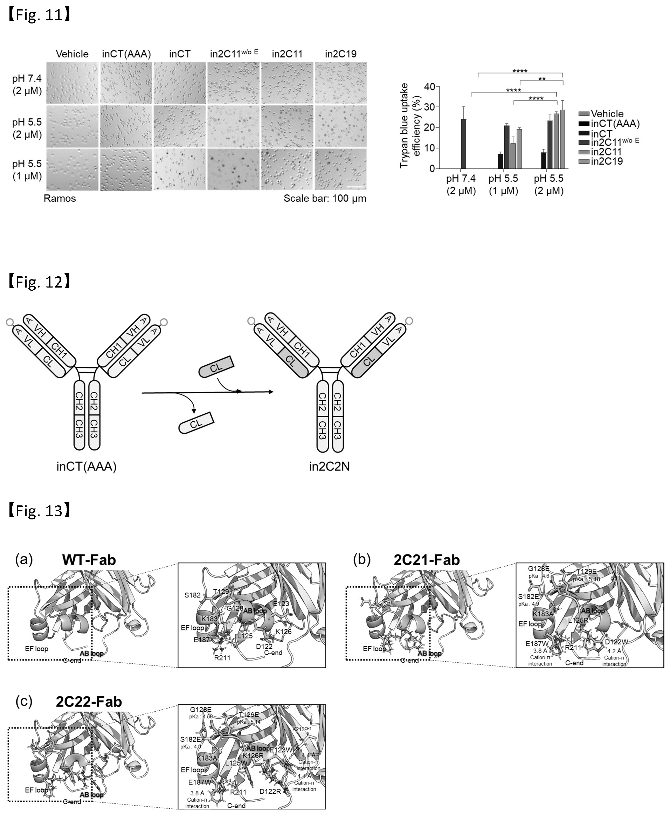

FIG. 9b shows the results of performing evaluation using another cell line, SW480-SA-GFP1-10 cells, under the same conditions as FIG. 11a in order to demonstrate that the results of evaluating the cytosol-penetrating ability in the MDA-MB-231-SA-GFP1-10 cells are not cell line-dependent. The results of quantification indicated that in2C11 and in2C19 exhibited increases in cell-based fluorescence intensity of about 2.2-fold and 2.4-fold, respectively, compared to inCT, and exhibited better cytosol-penetrating ability than the other variants. In2C11 and in2C19 were selected as lead antibodies because they showed the best endosomal escape ability in both cell lines.

### Example 8. Evaluation of Concentration-Dependent Cytosol-Penetrating Ability of Cytosol-Penetrating Antibodies Having Endosomal Escape Motif in Heavy Chain Constant Region CH3 Domain.

FIG. 10 shows the results of observing, through Lionheart Fx (Biotek), GFP fluorescence generated by complementation of enhanced split green fluorescent protein that appears in a concentration-dependent manner for the two lead antibodies in2C11 and in2C19 selected in Example 7, among the cytosol-penetrating antibody CH3 domain variants having an endosomal escape motif in the CH3 domain.

Specifically, 100 µL of 2 × 10³ cells (MDA-MB-231) or 4 × 10³ cells (SW480) were seeded into each well of a 96-well black plate (Greiner, 655090) under the same conditions as in Example 7 and cultured for 48 hours under 5% CO₂ and 37°C. After removing the supernatant, each well was treated with each of the positive control inCT, the negative control inCT(AAA), and the two CH3 domain variants at concentrations of 500 nM, 100 nM, and 10 nM at 37°C for 12 hours. After completely removing the medium from each well, the medium was replaced by dispensing 50 µL of FluoroBrite^{™} DMEM into each well. Then, images of each well were analyzed in real time through the bright field channel and GFP channel of Lionheart Fx in a state in which the cells were alive. Similar to FIG. 9, the cell-based fluorescence intensity by the variant was graphically expressed as a relative value to the quantified fluorescence intensity of the group treated with the positive control inCT at 500 nM.

Thereby, it was confirmed that the two lead antibodies, in2C11 and in2C19, had concentration-dependent cytosol-penetrating ability. At 10 nM, the fluorescence intensities of cells by inCT and most of the variants were very weak, making it difficult to distinguish the difference. However, from a concentration of 100 nM, the fluorescence signals could be compared, and in2C11 and in2C19 showed a higher level of fluorescence intensity at 100 nM than the inCT 500 nM-treated group. Thereby, it was confirmed that the two lead antibodies, in2C11 and in2C19, had concentration-dependent cytosol-penetrating ability.

### Example 9. Evaluation of pH-Dependent Interaction between Cytosol-Penetrating Antibody Having Endosomal Escape Motif in Heavy Chain Constant Region CH3 Domain and Cell Membrane.

In order for the cytosol-penetrating antibody according to the present invention to be endocytosed into a cell and then localize in the cytosol, the process of escaping from the endosome is essential. A previous study revealed that the endosomal escape ability of the existing cytosol-penetrating antibody inCT is attributed to pH-responsive interactions between the cell membrane binding motif located in CDR3 and residues in the framework region (Journal of Controlled Release, 2016, 235:165-175).

Since the CH3 variants have mutations for interactions with the cell membrane, which are specific to the endosomal weakly acidic environment, in the CH3 domain, the pH-dependent interactions with the cell membrane and the endosomal escape mechanism of the cytosol-penetrating antibodies were indirectly investigated through experimental methods similar to those in previous studies. The uptake of trypan blue dye by the non-adherent cell line Ramos human Burkitt's lymphoma cells lacking HSPG expression was evaluated.

FIG. 11 shows the results of examining whether the Ramos cell line can take up membrane-impermeable trypan blue through pore formation caused by the cytosol-penetrating antibody having an endosomal escape motif in the CH3 domain, at varying pHs using the live cell imaging device Lionheart Fx (Biotek).

Specifically, the Ramos cell line was cultured in RPMI medium containing 10% FBS and 1% ABAM. Cell suspensions containing a certain number of cells in each of 1X HBSS buffer (Hank's Balanced Salt Solution (Sigma H4641), 50 mM HEPES (pH 7.4)) with a pH of 7.4 corresponding to the cytosolic pH and HBSS buffer (Hank's Balanced Salt Solution (Sigma H4641), 50 mM MES (pH 5.5) with a pH of 5.5 corresponding to the early endosomal pH were prepared, and 50 µL of 5 × 10⁴ cells were seeded into each well of 96-well plates (SPL, 30096). Each well was treated with 50 µL of each of the CH3 domain variant and control antibody prepared at a concentration of 4 µM, and finally treated with a volume of 100 µL at a concentration of 2 µM at 37°C for 2 hours. The cells were stained by adding 50 µL of trypan blue solution (0.4%), Gibco, 15250061) at a 2:1 ratio, and then the process of attaching the cells to the bottom by centrifugation at 1,300 rpm for 3 minutes and carefully washing with PBS (pH 7.4) was repeated twice. Thereafter, the trypan blue staining pattern of living Ramos cells was observed using the color bright field microscope of the live cell imaging device Lionheart Fx (Biotek) in PBS (pH 7.4).

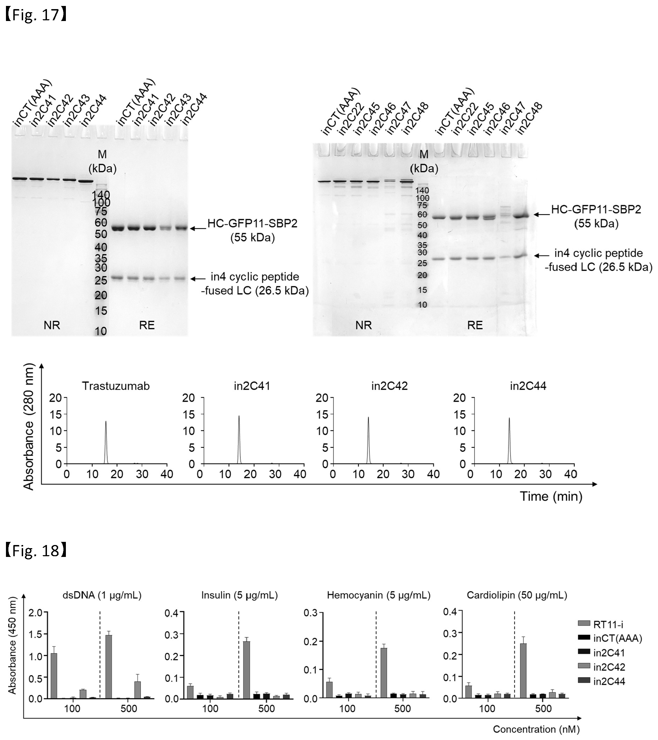

In FIG. 11, the results of quantitatively comparing the number of cells that had taken up trypan blue are shown as a graph. Specifically, the number of cells that had taken up trypan blue out of the total number of cells was counted and expressed as a percentage. A total of 200 cells or more were counted and the mean value is shown in the graph. It was confirmed that, at pH 5.5, the cells treated with the CH3 domain variant took up trypan blue. In particular, in2C11 and in2C19, which have the same glutamic acid patch while having high cytosol-penetrating ability, showed a high trypan blue uptake (%) of about 27% only at pH 5.5 (FIG. 17b). In addition, in2C11^{W/O E} without glutamic acid mutations showed trypan blue uptake (%) of about 24% and 23% at pH 7.4 and pH 5.5, respectively. Thereby, it was confirmed that the endosomal escape motif of the CH3 domain according to the present invention can interact with the cell membrane in a weakly acidic environment-specific manner through the glutamic acid patch.

### Example 10. Design of Cytosol-Penetrating Antibody (in2C2N) Having Endosomal Escape Motif in Light Chain Constant Region CL Domain.

Among the cytosol-penetrating antibodies (in2C1N) having an endosomal escape motif in the CH3 domain in Examples 1 to 9 above, two antibodies, in2C11 and in2C19, which had no problems with yield, physical properties, or non-specificity and had improved cytosol-penetrating ability, were selected. Subsequently, as shown in FIG. 12, the same endosomal escape motifs were introduced into structurally equivalent positions in the CL domain, which have high structural similarity to the CH3 domain, thereby conferring cytosol-penetrating ability to the CL domain.

At this time, CL domain variants in which endosomal escape motifs have been introduced into the CL domain were named in2C2N. Specifically, the CL domain variant in2C21 was constructed by grafting the endosomal escape motif of the in2C11 CH3 domain into the CL domain. Additionally, the CL domain variant in2C22 was constructed by grafting the endosomal escape motif of the in2C19 CH3 domain into the CL domain.

When grafting the endosomal escape motif of the CH3 domain, if an amino acid that can interact with the cell membrane among the wild-type residues of the CH3 domain was not present in the CL domain, the amino acid was introduced into the CL domain. For example, since the wild-type residue R355 of the in2C19 CH3 domain, which forms with a cation-π interaction with tryptophan (R355-L358W), is D122 at the corresponding position of the CL domain, a D122R mutation was added to in2C22.

As shown in FIG. 12, the present inventors constructed CL domain variants based on an inCT(AAA) antibody having no cytosol-penetrating ability to evaluate whether the variant has the ability to penetrate the cytosol through the endosomal escape motif of the CL domain. That is, the present inventors constructed CL domain variants using a heavy chain comprising the heavy chain variable region (VH) of SEQ ID NO: 14 and the heavy chain constant region (CH1-hinge-CH2-CH3) of SEQ ID NO: 1 having the CH3 domain, and a light chain comprising the light chain variable region (VL) of SEQ ID NO: 16 and the light chain constant region (CL) of each of SEQ ID NOs: 18 and 19 having an endosomal escape motif.

In addition, the cytosol-penetrating antibodies inCT and inCT(AAA) were also co-expressed and purified as in Example 1 and used as controls for evaluating physical properties and cytosol-penetrating ability. The specific amino acid sequences of the CL domain variants are shown in Table 6 below.

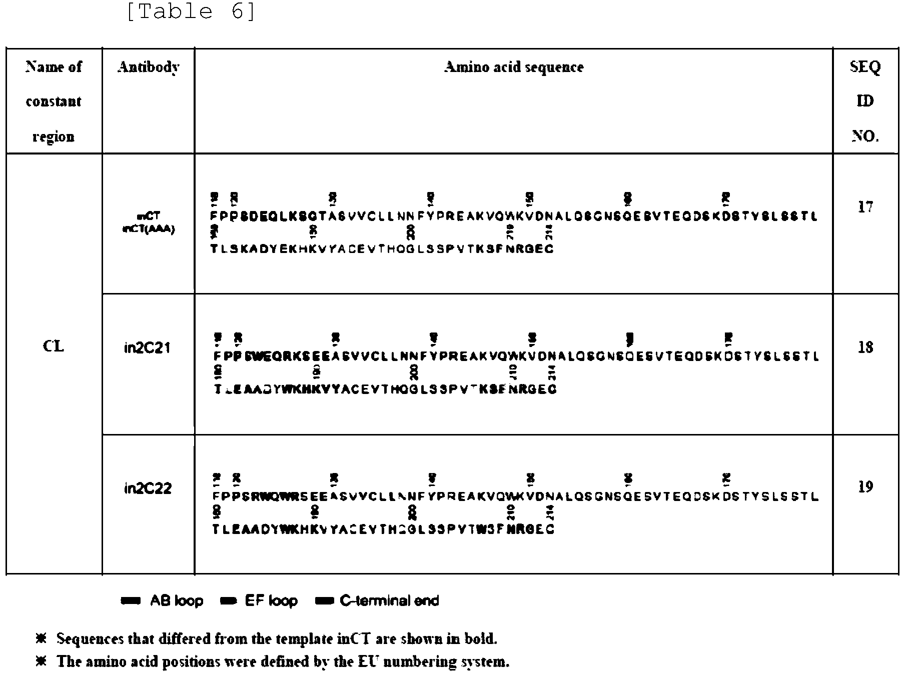

FIG. 13 shows the results of modeling the 3D structures of Fab regions, in which the endosomal escape motif of the CH3 domain variant is grafted into the CL domain, using AlphaFold2, and the results of analyzing the intramolecular interaction patterns of the antibodies using the PyMOL molecular visualizer.

In addition, to analyze the structural differences between each CL domain variant and the CH3 domain variant having the same endosomal escape motif, the root mean square deviation (RMSD) of the CL domain variant with respect to the CH3 domain variant having the same endosomal escape motif was calculated. Specifically, the wild-type CL domain was compared with the wild-type CH3 domain, the CL domain of in2C21 was compared with the CH3 domain of in2C11, and the CL domain of in2C22 was compared with the CH3 domain of in2C19.

**[Table 7]**

| CL variants | RMSD (Å) |
|---|---|
| CL Wild type | 1.425 |
| in2C21 | 1.423 |
| in2C22 | 1.484 |

Referring to Table 7 above, it was confirmed that both in2C21 and in2C22 had values similar to the RMSD value (1.425 Å) between the wild-type CH3 domain and the wild-type CL domain, suggesting that, when the endosomal escape motif of each of the CH3 domain variants was grafted to the corresponding positions of the CL domain, the structure of the CL domain was maintained.

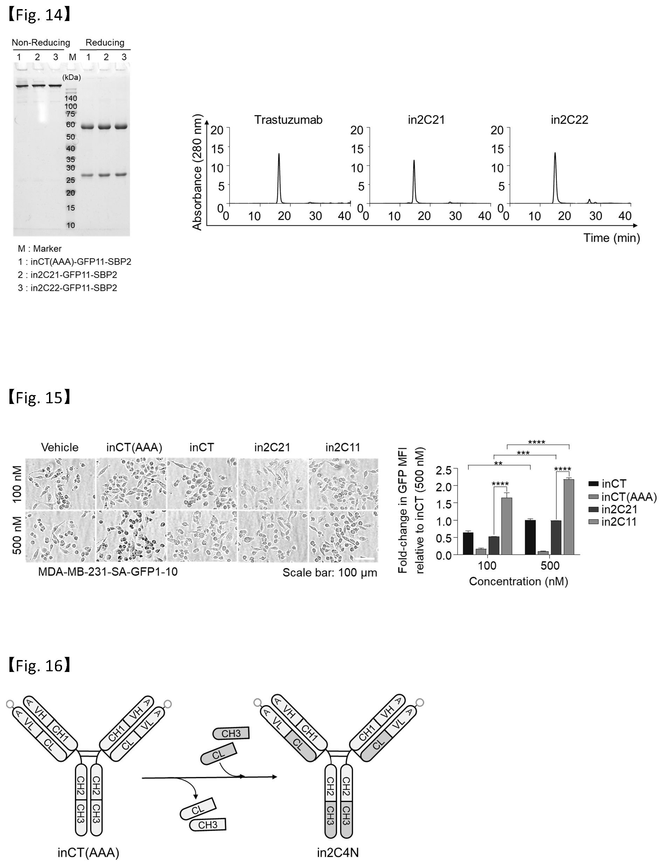

When an endosomal escape motif is formed in the CL domain by introducing the endosomal escape motif of each of the CH3 domain variants into the corresponding positions of the CL domain, the endosomal escape motif should be exposed on the surface of the antibody, just like in the CH3 domain, to ensure smooth interaction with the cell membrane. Therefore, the 3D structures (FIG. 16) of the CL domain variants, modeled with AlphaFold2, were submitted to GETAREA to calculate the solvent-accessible surface area ratio (%), and the results are shown in Table 8 below. Thereby, it was confirmed that, similar to the CH3 domain, the positions where the endosomal escape motifs of the AB, EF, and C-terminal loops of the CL domain are introduced are mostly exposed on the surface.

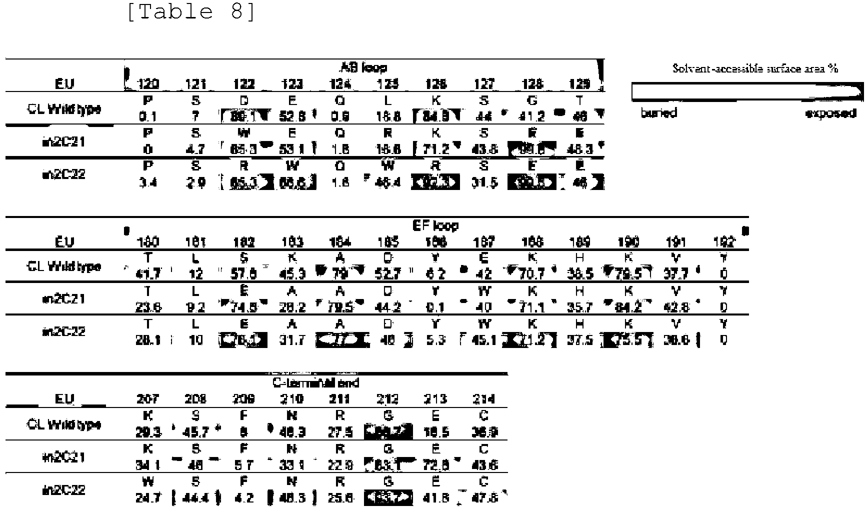

The following paragraph describes in detail the endosomal escape motifs of the CL domain variants.

The endosomal escape motif of in2C21 of SEQ ID NO: 18 consists of two tryptophan mutations (D122W, and E187W), one arginine mutation (L125R), one alanine mutation (K183A), and three glutamic acid mutations (G128E, T129E, and S182E), which are present in the AB loop, EF loop, and C-terminal loop.

Positions 122, 125, 128, 129, 182, 183, and 187 of the CL domain, into which the respective mutations were introduced correspond to positions 355, 358, 361, 362, 413, 414, and 418 of CH3 in the 3D structure (FIGS. 2b and 13b).

For arginine and tryptophan mutations, the mutations (R355W, L358R, and Q418W) introduced into in2C11 were introduced into CL in the same manner so that the interactions between amino acids induced in in2C11 would appear in the CL domain. In the case of L443R introduced into in2C11, the wild-type sequence was preserved because the corresponding position (211) in the wild-type CL is already arginine. Thereby, as shown in FIG. 13b, the cation-π interactions formed by R355W, L358R, Q418W, and L443R in in2C11 were also formed by D122W, L125R, E187W, and R211 even in in2C21. To prevent steric hindrance caused by Q418W in in2C11, the lysine located nearby was substituted with alanine (K414A), and similarly in in2C21, to prevent steric hindrance caused by E187W, the lysine at the structurally equivalent position was substituted with alanine (K183A). In addition, the glutamic acid patch (N361E, Q362E, and D413E) introduced to confer pH dependence to in2C11 was introduced into the same structurally equivalent positions in the CL domain (G128E, T129E, and S182E). As with in2C11, it was confirmed that the pKₐ of glutamic acid increased in the glutamic acid patch where glutamic acids were clustered in adjacent positions, with the pKₐ of T129E being 5.18 and the pKₐ of S182E being 4.9.

Therefore, in the case of in2C21 mimicking the endosomal escape motif of in2C11, two pairs of arginine and tryptophan forming cation-π interactions were clustered at the bottom of the antibody CL domain, and a surrounding glutamic acid patch was introduced on the side of the antibody CL domain to confer cytosol-penetrating ability and pH dependence.

The endosomal escape motif of in2C22 of SEQ ID NO: 19 consists of four tryptophan mutations (E123W, L125W, E187W, and K207W), two arginine mutations (D122R, and K126R), one alanine mutation (K183A), and three glutamate mutations (G128E, T129E, and S182E), which are present in the AB loop, EF loop, and C-terminal loop.

Positions 122, 123, 125, 126, 128, 129, 182, 183, 187, and 207 of CL, into which the respective mutations were introduced, correspond to positions 355, 356, 358, 359, 361, 362, 413, 414, 418, and 439 of CH3 in the 3D structure (FIGS. 2l and 13c).

For arginine and tryptophan mutations, the mutations (D356R, L358W, T359R, Q418W, and K439W) introduced into in2C19 and the wild-type CH3 sequence (R355) were introduced into CL so that the interactions between amino acids induced in in2C19 would appear in the CL domain. In this case, for L443R of in2C19, the wild-type sequence was preserved because the corresponding position (211) in the wild-type CL is already arginine.

Thereby, the cation-π interactions formed by R355, L358W, T359R, Q418W, and L443R in in2C19 were also formed by D122R, L125W, K126R, E187W, K207W, and R211 even in in2C22. In this case, in in2C19, D356R forms a cation-π interaction with K439W on the opposite side of CH3 at the CH3-CH3 interface, and even in in2C22, E123W forms a cation-π interaction with K213 on the CL-CH1 interface in a similar way. In addition, to prevent steric hindrance caused by Q418W in in2C19, the lysine located nearby was substituted with alanine (K414A), and even in in2C22, to prevent steric hindrance caused by E187W, the lysine at the structurally equivalent position was substituted with alanine (K183A).

In addition, the glutamic acid patch (N361E, Q362E, and D413E) introduced to confer pH dependence to in2C19 was introduced at the same structurally equivalent positions in the CL domain (G128E, T129E, and S182E). This is identical to the glutamic acid patch of in2C22, and it was confirmed that the pKₐ of T129E increased to 5.14 and the pKₐ of S182E increased to 4.9.

### Example 11. Expression and Purification of Cytosol-Penetrating Antibody (in2C2N-GFP11-SBP2) Having Endosomal Escape Motif in Light Chain Constant Region CL Domain.

In order to identify the biophysical properties of the CL domain variants and to evaluate the endosomal escape ability thereof through an enhanced split green fluorescent protein complementation system, the present inventors constructed in2C2N-GFP11-SBP2, a CL domain variant in which GFP11-SBP2 is fused to the C-terminus of the antibody heavy chain. Similar to the previous CH3 domain variants, a cyclic peptide (in4, DGVRQCRGDCFDGPL) targeting integrin αvβ3/αvβ5, membrane protein receptors overexpressed on the surface of tumor cells/tissue, was fused to the N-terminus of the light chain to induce endocytosis mediated by the tumor cell/tissue-specific receptors.

Specifically, to construct a heavy chain expression vector to produce a full-length IgG monoclonal antibody, a DNA, which has a secretory signal peptide-encoding DNA fused to the 5' end and encodes a heavy chain comprising an antibody heavy chain variable region (VH, SEQ ID NO: 14) and a CH1-hinge-CH2-CH3 having the wild-type CH3 domain (SEQ ID NO: 1), was cloned into the pcDNA3.4 vector by NotI/HindIII. At this time, to construct a cytosol-penetrating antibody fused with GFP11-SBP2, GFP11-(G₄S)₃-SBP2 was fused to the C-terminus of the heavy chain using three GGGS linkers. In addition, to construct a vector expressing a light chain, a DNA, which has a secretory signal peptide-encoding DNA fused to the 5' end and encodes a light chain comprising the antibody light chain variable region (VL, SEQ ID NO: 16) and a light chain constant region (CL, SEQ ID NO: 18 or 19), was cloned into the pcDNA3.4 vector by NotI/HindIII.

A common heavy chain expression vector comprising the VH of SEQ ID NO: 14 and the wild-type CH3 domain of SEQ ID NO: 1, and a light chain expression vector comprising the VL of SEQ ID NO: 16 and the light chain constant region sequence of SEQ ID NO: 18 or 19 were transiently co-transfected, and the cytosol-penetrating antibody (in2C2N-GFP11-SBP2) was expressed and purified.

HEK293-F cells (Invitrogen) growing in suspension in serum-free FreeStyle 293 expression medium (Invitrogen) in a shaking flask were transfected with a mixture of the plasmid and polyethyleneimine (PEI) (Polyscience, 24765-1) in the same manner as in Example 3. In addition, the purification of the CL variants and protein quantification were also performed in the same manner as in Example 3.

**[Table 9]**

| GFP11-SBP2 fused antibodies | Size (kDa) | Purification yield (mg/L of transfected cells) | Relative yield (%) |
|---|---|---|---|
| inCT | 162.9 | 40.6±2.6 | 84.5 |
| inCT(AAA) | 162.1 | 50.4±3.8 | 100 |
| in2C21 | 162.6 | 33.5±1.8 | 66.5 |
| in2C22 | 162.9 | 1.8±0.4 | 4 |

The specific production yields of the CL domain variants are shown in Table 9 above. in2C22-GFP11-SBP2 had a very low yield, and in2C21-GFP11-SBP2 had a yield that was about 40% lower than that of wild-type inCT(AAA)-GFP11-SBP2, but showed a good yield of 30 mg or more per liter.

The cytosol-penetrating antibodies (in2C2N-GFP11-SBP2) fused with a cyclic peptide and GFP11-SBP2 that target membrane protein receptors overexpressed on the tumor cell surface are hereinafter referred to as in2C2N. For example, in2C21-GFP11-SBP2 is in2C21.

### Example 12. Evaluation of Physical Properties of Cytosol-Penetrating Antibody (in2C2N) Having Endosomal Escape Motif in Light Chain Constant Region CL Domain.

FIG. 14 shows the results of analyzing the physical properties of two antibodies (in2C21 and in2C22) by 12% SDS-PAGE under reducing or non-reducing conditions and size exclusion chromatography after purification together with the control antibody inCT (AAA) in the same manner as in Example 4. Specifically, it was confirmed through SDS-PAGE that both in2C21 and in2C22 had molecular weights of about 162 to 163 kDa under non-reducing conditions, similar to the control antibody, and under reducing conditions, and that under reducing conditions, the molecular weight of the heavy chain was about 55 kDa (antibody heavy chain: about 50 kDa, and GFP11-SBP2 fragment: about 5 kDa) and the molecular weight of the light chain was about 26.5 kDa (antibody light chain: about 25 kDa, and in4 cyclic peptide: 1.5 kDa). This shows that the expressed and purified cytosol-penetrating antibody to which the in4 cyclic peptide and GFP11-SBP2 have been fused exists as a monomer in solution and does not form a dimer or oligomer through an unnatural disulfide bond. In addition, when the mAU value at 280 nm was measured using size exclusion chromatography (Superdex 200 Increase 10/300GL, Cytiva), single peaks without oligomers or tailing were observed for in2C21 and in2C22, as in the control Trastuzumab.

### Example 13. Evaluation of Cytosol-Penetrating Ability of Cytosol-Penetrating Antibody (in2C21) Having Endosomal Escape Motif in Light Chain Constant Region CL Domain.

FIG. 15 shows the results of observing and quantifying GFP fluorescence generated by complementation of enhanced split green fluorescent protein using the live cell imaging device Lionheart Fx (Biotek), to evaluate the cytosol-penetrating ability of the CL domain variant in2C21.

Specifically, the transformed MDA-MB-231-SA-GFP1-10 cell line that stably expresses SA-GFP1-10 was treated with the antibody at concentrations of 100 nM and 500 nM in the same manner as in Example 7, and then images of living cells were analyzed in real time through the bright field channel and GFP channel of Lionheart Fx. Intracellular fluorescence intensity by the CH3 or CL domain variant was quantified using the Gen5 image analysis program (Biotek) in the same manner as in Example 7, and the results are graphically shown. The experimental results indicated that in2C21 exhibited a cell-based fluorescence intensity that is about 1.0 times that of inCT, i.e., the same level as that of the positive control. in2C21 showed a lower cytosol-penetrating ability than in2C11, which is thought to be because the CH3 domain variant has an avidity effect as a CH3-CH3 dimer, whereas the CL domain variant has mutations introduced into a monomer. Consequently, it was confirmed that, when the endosomal escape motif introduced into the CH3 domain is grafted to the structurally equivalent positions in the CL domain, the CL domain can have cytosol-penetrating ability. In addition, it was confirmed that the endosomal escape motif of the same composition and position has lower cytosol-penetrating ability when introduced into a CL domain monomer than when introduced into a CH3 domain dimer.

### Example 14. Expression and Purification of Cytosol-Penetrating Antibody (in2C4N-GFP11-SBP2) Having Endosomal Escape Motifs in Heavy Chain Constant Region CH3 Domain and Light Chain Constant Region CL domain.

To design a cytosol-penetrating antibody having high cytosol-penetrating ability conferred by the CH3 and CL domains, by introducing endosomal escape motifs into both the CH3 and CL domains, the CH3 domain variants and CL domain variants selected in Examples 1 to 13 above were combined. As shown in FIG. 18, cytosol-penetrating antibodies in which endosomal escape motifs were introduced into the CH3 and CL domains were named in2C4N.

To identify the biophysical properties of the CH3 and CL domain variants (in2C4N) and to evaluate the cytosol-penetrating ability thereof through an enhanced split green fluorescent protein complementation system, in2C4N-GFP11-SBP2 was constructed by fusing GFP11-SBP2 fused to the C-terminus of the antibody heavy chain. Similar to the construction of the above-described CH3 domain variants or CL domain variants, a cyclic peptide (in4, DGVRQCRGDCFDGPL) targeting integrin αvβ3/αvβ5, membrane protein receptors overexpressed on the surface of tumor cells/tissue, was fused to the N-terminus of the light chain to induce endocytosis mediated by the tumor cell/tissue-specific receptors.

Specifically, among the CH3 domain variants evaluated as described in Example 7 with reference to FIGS. 9a and 9b, four representative variants (in2C11, in2C17, in2C18, and in2C19) with improved cytosol-penetrating ability and two CL variants (in2C21, and in2C22) were combined to obtain eight CH3 and CL domain variants having endosomal escape motifs in both the CH3 and CL domains. The CH3 and CL domain variants were expressed and purified as full-length IgG monoclonal antibodies. The names of the CH3 and CL domain variants in2C4N are shown in Table 10 below.

**[Table 10]**

| GFP11-SBP2 fused antibodies | Combination | | | Size (kDa) | Purification yield (mg/L of transfected cells) | Relative yield (%) |
|---|---|---|---|---|---|---|
| | CH3 variants | CL variants | CH1 variants | | | |
| inCT | Wild type | Wild type | | 162.9 | 40.6±2.6 | 84.5 |
| inCT(AAA) | | | | 162.1 | 50.4±3.8 | 100 |
| in2C41 | in2C11 | | | 162.9 | 34.0±2.8 | 67 |
| in2C42 | in2C17 | in2C21 | | 163.1 | 34.5±7.8 | 68 |
| in2C43 | in2C18 | | Wild type | 163.1 | 3.1±1.5 | 6 |
| in2C44 | in2C19 | | | 163.5 | 21.5±7.7 | 43 |
| in2C45 | in2C11 | | | 163.1 | 3.1±1.5 | 6 |
| in2C46 | in2C17 | in2C22 | | 163.3 | 1.5±0.7 | 3 |
| in2C47 | in2C18 | | | 163.4 | ND | - |
| in2C48 | in2C19 | | | 163.5 | 0.4±0.3 | 1 |

Specifically, in2C41-GFP11-SBP2 was expressed using a heavy chain expression plasmid comprising CH1-hinge-CH2-CH3 having the VH of SEQ ID NO: 14 and the CH3 of in2C11 of SEQ ID NO: 4, and a light chain expression plasmid having the VL of SEQ ID NO: 16 and the CL of SEQ ID NO: 18. In the same way, the remaining antibodies were expressed using a heavy chain expression vector containing each CH3 domain variant and a light chain expression vector containing each CL domain variant. The expression of these antibodies in animal cells was performed in HEK293-F cells in the same manner as in Example 3.

Subsequently, the purification of the CH3 and CL domain variants and protein quantification were performed in the same manner as in Example 3. The specific production yields of the eight types of in2C4N-GFP11-SBP2 are shown in Table 13 above. Among the eight CH3 and CL domain combination antibodies, three antibodies, in2C41, in2C42, and in2C44, showed a good yield of 20 mg or more per liter.

The cytosol-penetrating antibodies (in2C4N-GFP11-SBP2) fused with a cyclic peptide and GFP11-SBP2 that target membrane protein receptors overexpressed on the tumor cell surface are hereinafter referred to as in2C4N. For example, in2C41-GFP11-SBP2 is in2C41.

### Example 15. Evaluation of Physical Properties of Cytosol-Penetrating Antibodies (in2C4N) Having Endosomal Escape Motifs in Heavy Chain Constant Region CH3 Domain and Light Chain Constant Region CL Domain.

FIG. 17 shows the results of analyzing eight CH3 and CL domain variants having endosomal escape motifs in the CH3 domain and CL domain by 12% SDS-PAGE under reducing or non-reducing conditions and size exclusion chromatography after purification together with the control antibody inCT (AAA). Specifically, it was confirmed that all the eight CH3 and CL domain variants had a molecular weight of about 162 to 163 kDa under non-reducing conditions, similar to the control antibody, and that under reducing conditions, the molecular weight of the heavy chain was about 55 kDa (antibody heavy chain: about 50 kDa, and GFP11-SBP2 fragment: about 5 kDa) and the molecular weight of the light chain was about 26.5 kDa (antibody light chain: about 25 kDa, and in4 cyclic peptide: 1.5 kDa). In the case of in2C43, in2C45, in2C46, and in2C47, the heavy chains produced double bands rather than single bands under reducing conditions. It was confirmed that the remaining four CH3 and CL domain variants, excluding the above four variants, exist as monomers in solution and do not form dimers or oligomers through unnatural disulfide bonds. The graph at the bottom of FIG. 17 shows the mAU values at 280 nm measured in the same manner as in Example 4 using size exclusion chromatography (Superdex 200 Increase 10/300GL, Cytiva) for three CH3 and CL domain variants (in2C41, in2C42, and in2C44) that showed a good yield of 10 mg or more per liter. Single peaks without oligomers and tailing were observed in the three variants, as in the controls Trastuzumab and inCT(AAA).

Therefore, additional evaluations were conducted on the three CH3-CL domain variants (in2C41, in2C42, and in2C44) that have a good production yield of 10 mg or more per liter (Table 10) and properties similar to the control wild-type antibody.

### Example 16. Evaluation of Non-Specific Binding of Cytosol-Penetrating Antibodies (in2C4N) Having Endosomal Escape Motif in Heavy Chain Constant Region CH3 Domain and Light Chain Constant Region CL Domain.

FIG. 18 shows the results of performing ELISA on four antigens, dsDNA, insulin, keyhole limpet hemocyanin (KLH) and cardiolipin, which allow the evaluation of the non-specific binding of antibodies, to evaluate the non-specific binding of three lead antibodies (in2C41, in2C42, and in2C44) having endosomal escape motifs in the CH3 and CL domains. The experiment was performed in the same manner as in Example 5. In the experiment, when the antibody at a concentration of 100 nM shows an absorbance of 0.05 or higher at 450 nm for at least one of the four antigens, it is determined to have non-specific binding. As a result of the experiment, in2C42 showed non-specific binding to the dsDNA antigen at concentrations of 100 nM and 500 nM, unlike the control group and the other two variants. Therefore, the other two variants excluding in2C42 were selected as candidates.

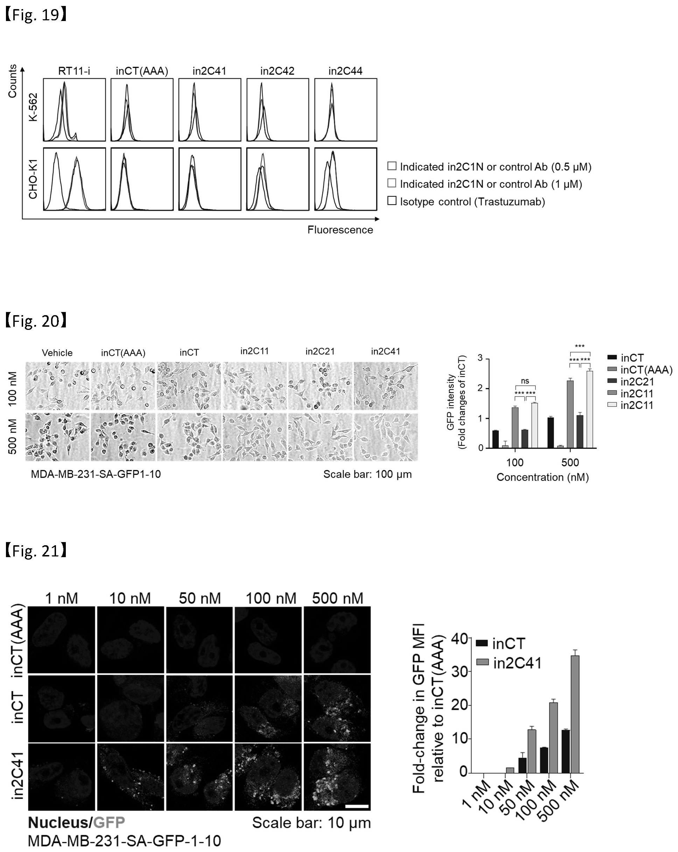

FIG. 19 shows the results of performing flow cytometry analysis to evaluate the non-specific binding of three lead antibodies (in2C41, in2C42, and in2C44) having endosomal escape motifs in the CH3 and CL domains to the cell membrane. The experiment was performed in the same manner as in Example 5. The experimental results indicated that in2C42 and in2C44 showed non-specific binding to the cell surfaces of K-562 and CHO-K1 compared to the wild-type negative control. Therefore, among the variants having endosomal escape motifs in the CH3 and CL domains, in2C41, which has good physical properties, was selected as the final CH3-CL variant.

### Example 17. Evaluation of Cytosol-Penetrating Ability of Cytosol-Penetrating Antibodies Having Endosomal Escape Motifs in Heavy Chain Constant Region CH3 Domain and Light Chain Constant Region CL Domain.

FIG. 20 shows the results of observing and quantifying GFP fluorescence generated by complementation of enhanced split green fluorescent protein using the live cell imaging device Lionheart Fx (Biotek), to evaluate the cytosol-penetrating ability of in2C41. In the same manner as in Example 7, MDA-MB-231-SA-GFP1-10 cells were treated with each of the cytosol-penetrating antibodies at a concentration of 500 nM for 12 hours, and then images of living cells were analyzed in real time through the bright field channel and GFP channel of Lionheart Fx. Intracellular fluorescence intensity by each of the CH3 and CL domain variants was quantified using the Gen5 image analysis program (Biotek) in the same manner as in Example 7, and the results are graphically shown.

When comparing the cytosol-penetrating abilities of in2C11, in2C21, and in2C41 used at a concentration of 100 nM or 500 nM to treat the MDA-MB-231-SA-GFP1-10 cells, in2C41 showed an about 2.6-fold higher fluorescence intensity compared to inCT, and similar to Example 14, in2C11 and in2C21 showed an about 2.2-fold higher fluorescence intensity and an about 1.1-fold higher fluorescence intensity, respectively, compared inCT. Thereby, it was confirmed that in2C41, which has endosomal escape motifs in the constant region CH3 domain and CL domain, has the highest cytosol-penetrating ability among the cytosol-penetration antibodies evaluated.

### Example 18. Evaluation of GFP Fluorescence Based on Concentration-Dependent Cytosol Penetration of Cytosol-Penetrating Antibody Having Endosomal Escape Motifs in Heavy Chain Constant Region CH3 Domain and Light Chain Constant Region CL Domain.

FIG. 21 shows the results of observing the concentration-dependent cytosol-penetrating ability of in2C41 by confocal microscopy using an enhanced split green fluorescent protein complementation system.

Specifically, the MDA-MB-231-SA-GFP1-10 cells constructed in Example 6 were cultured in an RPMI medium containing 10% FBS and 1% ABAM. A 12-mm-radius coverslip (Marienfeld, 01-115-20) was placed in each well of a 24-well plate (SPL, 30024), and each well was coated by dispensing 500 µL of poly-L-lysine solution (Sigma, P4707). After washing twice with triple distilled water, the plate was dried sufficiently at room temperature for at least 2 hours. 500 µL of 1 × 10⁵ MDA-MB-231-SA-GFP1-10 cells were dispensed into each well of the prepared plate and cultured for 24 hours at 37°C under 5% CO₂ and. After removing the supernatant, the cells were treated with each of the GFP11-SBP2-fused in2C41 and control antibodies (inCT(AAA), and inCT) at different concentrations (1, 10, 50, 100, and 500 nM) at 37°C for 12 hours. After removing the supernatant, the cells were washed twice with PBS (pH 7.4) and then acid-washed twice with low-pH glycine buffer (200 mM glycine, 150 mM NaCl, pH 2.5) for 30 seconds each. Next, the cells were washed twice with PBS and fixed with a 4% paraformaldehyde (Thermo Scientific^{™}, 28908) solution at room temperature for 10 minutes. After washing three times with PBS, the nuclei were stained (blue fluorescence) with Hoechst 33342 (Thermo Scientific^{™}, 62249) for 5 minutes. After washing three times with PBS, the cover glass in the well was removed with tweezers and mounted on a glass slide (Matsunami, HMA-APS-11) using mounting medium (Dako, S3023) for sampling. Each sample was observed using a confocal microscope (Leica, Stellaris 5).

In FIG. 21, the results of quantifying the intensity of GFP fluorescence observed from the cytosol as a fold-change relative to the negative control inCT (AAA) are graphically shown. As shown in FIG. 20, in2C41 showed an about 2.6-fold higher fluorescence intensity than inCT, and GFP fluorescence was observed even at 10 nM, a lower concentration than for the positive control inCT.

### Example 19. Quantitative Evaluation of Endosomal Escape Efficiency of Cytosol-Penetrating Antibody through Complementation of Enhanced Split Green Fluorescent Protein.

Table 12 below shows the concentration-dependent endosomal escape efficiencies of the cytosol-penetrating antibodies (inCT, and in2C41), measured using an enhanced split green fluorescent protein complementation system.

**[Table 12]**

| Assay system | | Split-GFP plus quantitative western blotting | | | | | |
|---|---|---|---|---|---|---|---|
| | | MDA-MB-231-SA-GFP1-10 cell | | | SW480-SA-GFP1-10 cell | | |
| CT concentration (µM) | | 0.1 | 0.5 | 1 | 0.1 | 0.5 | 1 |
| Endosomal escape efficiency (%) | inCT | 3.3 ± 0.4 | 5.0 ± 0.2 | 6.5 ± 0.4 | 2.6 ± 0.2 | 4.9 ± 0.2 | 6.0 ± 0.4 |
| | in2C41 | 8.4 ± 0.5 | 14.2 ± 0.4 | 18.7 ± 0.5 | 6.9 ± 0.1 | 12.9 ± 0.4 | 17.1 ± 0.3 |

Specifically, the endosomal escape efficiencies of the antibodies depending on their concentrations (0.1, 0.5, and 1 µM) were quantified using an enhanced split green fluorescent protein complementation system developed in a previous study (BBRC, 2015;467 (4) :771-777) and Western blotting.

It was confirmed that the endosomal escape efficiencies of the two cytosol-penetrating antibodies increased in proportion to the concentration. In particular, it was confirmed that the endosomal escape efficiency of the cytosol-penetrating antibody in2C41 developed in the present invention was about 3 times higher than that of the cytosol-penetrating antibody inCT used as a positive control.

### Example 20. Expression and Purification of HiBiT-Fused Antibody for Use with Split Nanoluciferase Luminescent Protein Complementation System.

For cytosol-penetrating antibodies that have the ability to penetrate the cytosol through the endosomal escape motif of the constant region, cytosol-penetrating ability and endosomal escape efficiency were evaluated using a split nanoluciferase luminescent protein complementation system, which is another cytosol-penetrating ability evaluation system (Nature Communications, 2021, 12(1):3721). The results were compared with the results of evaluating cytosol-penetrating ability through the enhanced split green fluorescent protein complementation system.

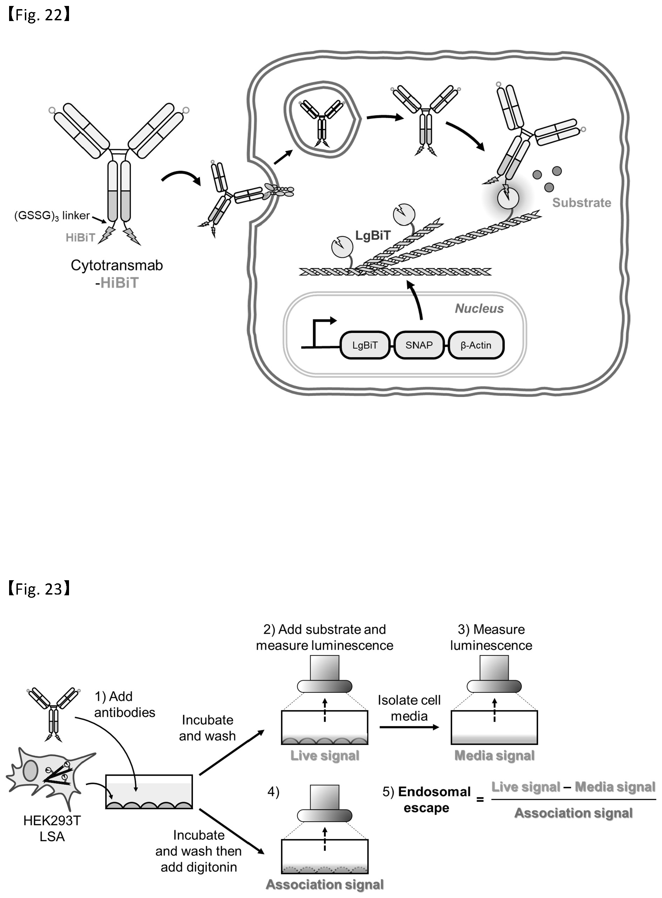

FIG. 22 is a schematic diagram illustrating the process in which bioluminescence by complementation of split nanoluciferase luminescent protein appears when a cytosol-penetrating antibody localizes in the cytosol. By measuring the luminescence that appears through complementation of HiBiT fused to the C-terminus of the heavy chain of the cytosol-penetrating antibody, which is expressed in the cytosolic actin, the endosomal escape efficiency of the cytosol-penetrating antibody can be quantified.

FIG. 23 shows a process of quantifying endosomal escape efficiency using a split nanoluciferase luminescent protein complementation system. A live signal, which is luminescence caused by an antibody delivered to the cytosol, is measured, the supernatant is removed from each well to exclude luminescence caused by LgBiT secreted outside the cell, a media signal is measured, and a cytosolic signal (hereinafter referred to as cytosolic delivery) value may be calculated by subtracting the media signal from the live signal. In addition, an association signal (hereinafter total cellular association) caused by the antibody delivered into the cytosol (cytosolic delivered antibody) through cell membrane permeabilization by treatment with digitonin and the antibody entrapped in the endosome (endosomal entrapped antibody) is measured. The endosomal escape efficiency can be expressed as a percentage by dividing the measured cytosolic delivery value by the total cellular association value.

To use the above-described system, HiBiT (VSGWRLFKKIS) was fused to the C-terminus of the heavy chain of a cytosol-penetrating antibody having the ability to penetrate the cytosol through the endosomal escape motif of the constant region.

Specifically, to construct a heavy chain expression vector for producing each of inCT-HiBiT, inCT(AAA)-HiBiT and in2C11-HiBiT, in2C21-HiBiT and in2C41-HiBiT as full-length IgG monoclonal antibodies, a DNA, which has a secretory signal peptide-encoding DNA fused to the 5' end and encodes a heavy chain comprising an antibody heavy chain variable region (VH, SEQ ID NO: 14) and a CH1-hinge-CH2-CH3 having a CH3 domain (SEQ ID NO: 1 or 4), was cloned into the pcDNA3.4 vector by NotI/HindIII. At this time, HiBiT was fused to the C-terminus of the heavy chain using three GSSG linkers. For the light chain, light chain DNA comprising the light chain variable region (VL, SEQ ID NO: 16) and light chain constant region (CL, SEQ ID NO: 17) constructed in Example 3 or light chain DNA comprising the light chain variable region (VL, SEQ ID NO: 16) and light chain constant region (CL, SEQ ID NO: 18) constructed in Example 12 was used.

In the case of inCT(AAA)-HiBiT, inCT-HiBiT, and in2C11-HiBiT, a heavy chain expression vector comprising the VH of SEQ ID NO: 14 and the CH3 domain of SEQ ID NO: 1 or 4 and a common light chain expression vector comprising the VL of SEQ ID NO: 16 and the CL domain of SEQ ID NO: 17 were transiently co-transfected, and the antibody was expressed and purified. In the case of in2C41-HiBiT, a heavy chain expression vector comprising the VH domain of SEQ ID NO: 14 and the CH3 domain of SEQ ID NO: 4 and a common light chain expression vector comprising the VL domain of SEQ ID NO: 16 and the CL domain of SEQ ID NO: 18 were transiently co-transfected, and the antibody was expressed and purified. The expression of these antibodies in animal cells was performed in HEK293-F cells in the same manner as in Example 3.

Subsequently, the purification of the HiBiT-fused cytosol-penetrating antibodies and protein quantification were performed in the same manner as in Example 3, and the production yields of these antibodies in animal cells are shown in Table 15 below. HiBiT-fused In2C41 having endosomal escape motifs in the constant region CH3 and CL domains showed a decreased yield of 52.5% relative to wild-type inCT(AAA)-HiBiT, but showed a good yield of 20 mg or more per liter.

**[Table 13]**

| HiBit fused antibodies | Combination | | | Size (kDa) | Purification yield (mg/L of transfected cells) | Relative yield (%) |
|---|---|---|---|---|---|---|
| | CH3 variants | CL variants | CH1 variants | | | |
| inCT | Wild type | Wild type | | 155.2 | 41.5±2.1 | 95 |
| inCT(AAA) | | | | 153.9 | 43.5±2.1 | 100 |
| in2C11 | in2C11 | Wild type | Wild type | 154.5 | 38.0±2.8 | 87 |
| in2C21 | Wild type | in2C21 | | 154.4 | 40.8±3.5 | 93 |
| in2C41 | in2C11 | in2C21 | | 155.0 | 21.8±1.7 | 52.5 |

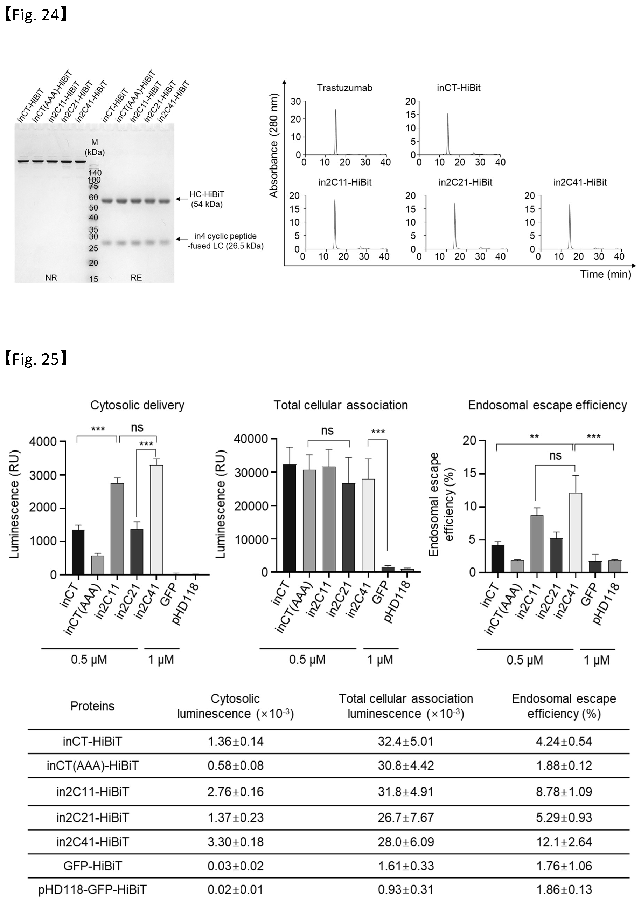

FIG. 24 shows the results of analyzing the HiBiT-fused cytosol-penetrating antibodies having endosomal escape motifs in the constant region CH3 and/or CL domain by 12% SDS-PAGE under reducing or non-reducing conditions and size exclusion chromatography after purification. Specifically, it was confirmed that all the three antibodies (in2C11-HiBiT, in2C21-HiBiT, and in2C41-HiBiT) had a molecular weight of about 154 kDa under non-reducing conditions, and that under reducing conditions, the molecular weight of the heavy chain was about 51 kDa (antibody heavy chain: about 51 kDa, and HiBiT fragment: about 3 kDa) and the molecular weight of the light chain was about 26.8 kDa (antibody light chain: about 25.3 kDa, and in4 cyclic peptide: 1.5 kDa). When the mAU value at 280 nm was measured using size exclusion chromatography (Superdex 200 Increase 10/300GL, Cytiva) in the same manner as in Example 3, single peaks without any special oligomers or tailing were observed for all the three antibodies (in2C11-HiBiT, in2C21-HiBiT, and in2C41-HiBiT), as in the controls Trastuzumab and inCT-HiBiT.

Thereby, it was confirmed that the HiBiT-fused cytosol-penetrating antibodies having endosomal escape motif in the constant regions have a good production yield of 20 mg or more per liter (Table 13) and have properties similar to the control wild-type antibody.

### Example 21. Evaluation of Endosomal Escape Efficiency of HiBiT-Fused Antibodies Using Split Nanoluciferase Luminescent Protein Complementation System.

FIG. 25 shows the results of quantifying the endosomal escape efficiency of HiBiT-fused cytosol-penetrating antibodies (in2C11-HiBiT, in2C21-HiBiT, and in2C41-HiBiT) having endosomal escape motifs in the constant regions at a concentration of 500 nM in HEK293T cells through a split nanoluciferase luminescent protein complementation system.

Specifically, the transformed HEK293T-LSA cell line that stably expresses LgBit-SNAP-Actin (LSA) was obtained from Monash University (Australia) and used. The HEK293T-LSA cell line was cultured in DMEM containing 10% FBS and 1% ABAM. 100 µL of 5 × 10³ cells were seeded into each well of a 96-well white plate (Greiner, 655098) and cultured for 24 hours at 37°C under 5% CO₂. After removing the supernatant, the cells were treated with each of positive control inCT-HiBiT, negative control inCT(AAA)-HiBiT, and three cytosol-penetrating antibodies (in2C11-HiBiT, in2C21-HiBiT, and in2C41-HiBiT) having endosomal escape motifs in the constant regions at a concentration of 500 nM at 37°C for 12 hours.

At this time, as other controls, GFP-HiBiT and pHD118-GFP-HiBiT, which have been reported to have endosomal escape efficiency in the split luciferase luminescent protein complementation system, were also evaluated. pHD118 is an endosomal escape peptide (EEP) reported to have pH-dependent endosomal escape ability (Nature Communications, 2021, 12(1):3721).

Next, the supernatant was removed to reduce interference caused by phenol red contained in the existing medium, and then each well was washed three times with DMEM (Cytiva, SH30585.02, 10% FBS) containing no phenol red. For the well for measuring the total cellular association signal, the cells were permeabilized by treatment with 75 µL of digitonin solution (Millipore, 300410-250MGCN, 0.01% w/v in DMSO) for 1 hour at 37°C, 1 hour before substrate treatment. The well for measuring cytosolic delivery were also washed three times with DMEM (Cytiva, SH30585.02, 10% FBS) containing no phenol red after removing the supernatant, and then 75 µL of fresh medium was added thereto. 25 µL of furimazine (Promega, N2012) substrate was added simultaneously to the well for measuring total cellular association signal that had undergone permeabilization with digitonin and the well for measuring cytosolic delivery (live signal and media signal), and then the wells were incubated on an orbital shaker at room temperature for 10 minutes. Then, the luminescence was measured with a plate reader Cytation 3 (Biotek, USA) to obtain the live signal and total cellular association signal. Next, 50 µL of the supernatant from each well where the live signal was measured was carefully transferred to a fresh plate, and then the luminescence was measured to obtain the media signal. The measured values were calculated as shown in FIG. 25, and the results of quantifying the endosomal escape efficiency are shown in FIG. 25.

In the HEK293-LSA cells, the endosomal escape efficiency of the conventional cytosol-penetrating antibody inCT was about 4.2%, and the endosomal escape efficiency of inCT (AAA) having no cytosol-penetrating ability was about 1.9%. The endosomal escape efficiency of in2C11 was about 8.8%, that of in2C21 was about 5.3%, and that of in2C41 was 12.1%. These values correspond to 2.0 times, 1.3 times, and 2.8 times the endosomal escape efficiency of inCT, respectively. GFP and pHD118-GFP showed low endosomal escape efficiencies of 1 to 2%, similar to the endosomal escape efficiencies reported in the literature.

Through the above experiment, the reliability of the evaluation system was confirmed by the fact that the results of quantifying cytosol-penetrating ability through the enhanced split green fluorescent protein complementation system were equivalent to the results of quantifying endosomal escape efficiency through the split nanoluciferase complementation system. In addition, all the five antibodies with which the HEK293-LSA were treated showed similar levels of total cellular association, which means that the five antibodies were associated with and internalized in the HEK293-LSA cells at similar levels. Thereby, it was confirmed that the increased endosomal escape efficiencies of in2C11, in2C21, and in2C41 cytosol-penetrating antibodies were due to increased cytosolic delivery of these antibodies rather than total cellular association.

### Example 22. Examination of Transport Route after Endocytosis of Cytosol-Penetrating Antibody Having Endosomal Escape Motifs in Heavy Chain Constant Region CH3 Domain and Light Chain Constant Region CL Domain.

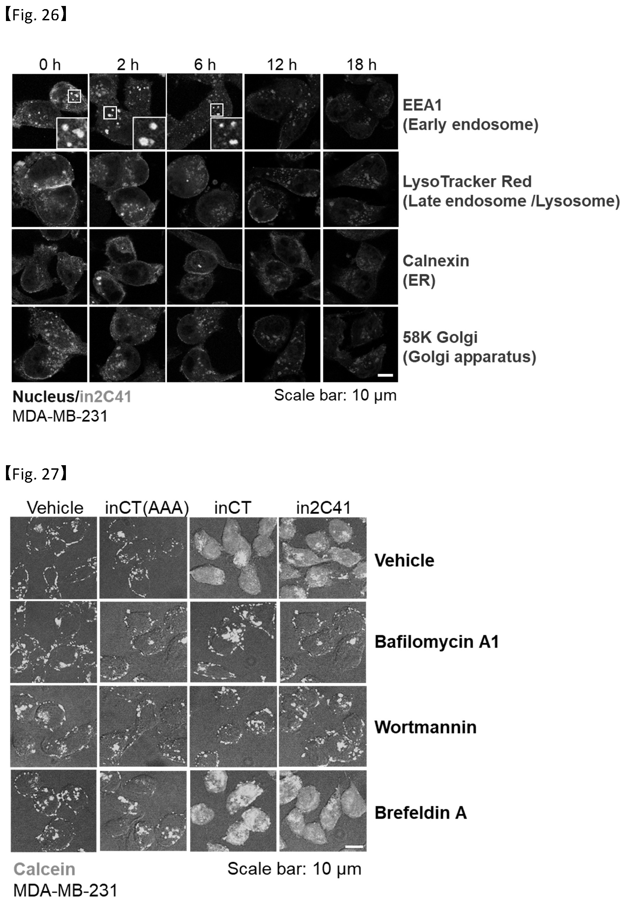

FIG. 26 shows the result of examining the overlap between in2C41 and several intracellular organelles (early endosome, late endosome/lysosome, endoplasmic reticulum, and Golgi apparatus) using a pulse-chase experiment and confocal microscopy, to observe the intracellular transport route of in2C41 that entered the cell.

Specifically, 500 µL of 1 × 10⁵ MDA-MB-231 cells were seeded into each well of a lysine-coated plate prepared in the same manner as in Example 18 and cultured for 24 hours at 37°C under 5% CO₂. The supernatant was removed, and the cells were treated with in2C41 at a concentration of 2 µM at 37°C for 30 minutes. Next, PBS washing and acid washing were performed, and the cells were incubated in an RPMI medium containing 10% FBS and 1% ABAM at 37°C for 0, 2, 6, 12, and 18 hours. After removing the supernatant, PBS washing and cell fixation were performed, and the cells were permeabilized by treatment with 1X PERM buffer, obtained by diluting permeabilization buffer (10X) (Invitrogen, 00-8333-56) diluted to 1X in 1% BSA (1% BSA in PBS) solution, at room temperature for 10 minutes. Then, the cells were treated with blocking solution (2% BSA in PBS) at room temperature for 2 hours to prevent non-specific binding of a staining antibody. The early endosome, endoplasmic reticulum, and Golgi apparatus were primarily labeled by treatment with anti-EEA1 mouse IgG (Santa Cruz, sc-53939), anti-calnexin mouse IgG (Santa Cruz, sc-70481), and anti-58K Golgi mouse IgG (Santa Cruz, sc-58770), respectively, at 25°C for 4 hours. Among the intracellular organelles, the late endosome/lysosome was treated with LysoTracker Red DND-99 (Invitrogen, L7528) diluted to 1 µM in medium at 37°C for 30 minutes before the cell fixation step, and then subjected to cell fixation, cell permeabilization, and blocking steps in the same manner as above. Subsequently, the intracellular organelles that have been primarily labeled with in2C41 and anti-mouse antibodies were secondarily labeled with an FITC (green fluorophore)-linked anti-human Fc antibody (Sigma, F9512) and an Alexa Fluor 555 (red fluorophore)-linked anti-human Fc antibody (Invitrogen, A212422). Then, in the same manner as in Example 18, the cells were washed with PBS, stained with nuclear staining, and sampled to be observed under a confocal microscope. Thereafter, PBS washing, nucleus staining, sampling, and confocal microscopic observation were performed in the same manner as in Example 18.

As shown in FIG. 26, it was confirmed that, in the cells cultured for 0 and 2 hours after treatment with in2C41 for 30 minutes, in2C41 did spread in the cytosol, and a portion thereof did overlap strongly with EEA1 and relatively weakly with LysoTracker. At and after 6 hours, the green fluorescence signal of in2C41 in the cytosol was found to gradually decrease. In particular, it was confirmed that in2C41 did not overlap with calnexin and the 58K Golgi at any time, suggesting that in2C41 moves directly from the later endosome to the cytosol without additional movement to the endoplasmic reticulum and Golgi apparatus.

### Example 23. Evaluation of Effect of Late Endosomal Acidification on Endosomal Escape of Cytosol-Penetrating Antibody Having Endosomal Escape Motifs in Heavy Chain Constant Region CH3 Domain and Light Chain Constant Region CL Domain.

FIG. 27 shows the results of observing the cytosol-penetrating ability of in2C41 by confocal microscopy using calcein in the presence or absence of pharmacological inhibitors.

Specifically, 500 µL of 1 × 10⁵ MDA-MB-231 cells were seeded into each well of a lysine-coated plate prepared in the same manner as in Example 18 and cultured for 24 hours under conditions of 5% CO₂ and 37°C. The pharmacological inhibitors used thereafter were bafilomycin A1, which inhibits acidification of endosomes by inhibiting ATPase hydrogen pump; wortmannin, which inhibits maturation from early endosomes to late endosomes; and brefeldin A, which inhibits transport from endosomes to the endoplasmic reticulum and Golgi. The cells were treated with bafilomycin A1, wortmannin, and brefeldin A diluted to 200 nM, 200 nM, and 7 µM, respectively, in serum-free RPMI medium, and then cultured at 37°C for 30 minutes. After removing the supernatant, the cells were washed twice with PBS and treated with 1 µM of each of in2C41 and control antibodies and 200 µM of membrane-impermeable green fluorescent calcein (Sigma, C0875) in medium at 37°C for 12 hours. Next, washing twice with PBS, fixation, sampling, and confocal microscopic observation were performed in the same manner in Example 18.

As shown in FIG. 27, in the cells treated with bafilomycin A1 and wortmannin, in2C41 was unable to escape from the endosome, so calcein remained trapped in the endosome, while in the cells treated with brefeldin A, calcein did spread throughout the cytosol. Thereby, it was confirmed that in2C41 escapes from the endosome in the acidic environment of the late endosome and moves directly into the cytosol without moving to the endoplasmic reticulum and Golgi apparatus, as described in Example 23.

### Example 24. Evaluation of pH-Dependent Cell Membrane-Binding Ability and Cell-Penetrating Ability of Cytosol-Penetrating Antibody Having Endosomal Escape Motifs in Heavy Chain Constant Region CH3 Domain and Light Chain Constant Region CL Domain.

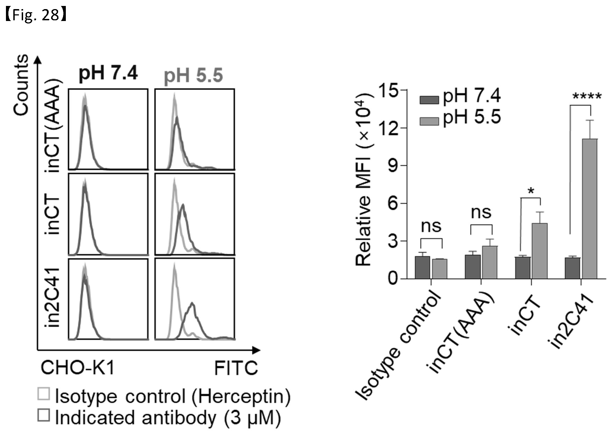

FIG. 28 shows the results of evaluating the pH-dependent cell membrane binding of in2C41 and control antibodies by flow cytometry analysis.

Specifically, 2 × 10⁵ CHO-K1 cells lacking the expression of integrin αvβ3/αvβ5 were prepared per sample and washed with each of pH 7.4 buffer (PBS, 2% FBS, 50 mM HEPES (Sigma, H3537)) and pH 5.5 buffer (PBS, 2% FBS, 50 mM MES (Sigma, H76039)). Then, the cells were treated with each of the in2C41 antibody and control antibodies diluted to a concentration of 3 µM in each pH buffer at 4°C for 1 hour. The cells were washed with each pH buffer and treated with FITC (green fluorophore)-linked anti-human Fc antibody (Sigma, F9512) diluted in each pH buffer for 30 minutes at 4°C. After washing with each pH buffer, the cells were analyzed using a flow cytometer (Agilent, Novocyte).

As shown in FIG. 28, it was confirmed that in2C41 specifically binds to the cell membrane at pH 5.5.

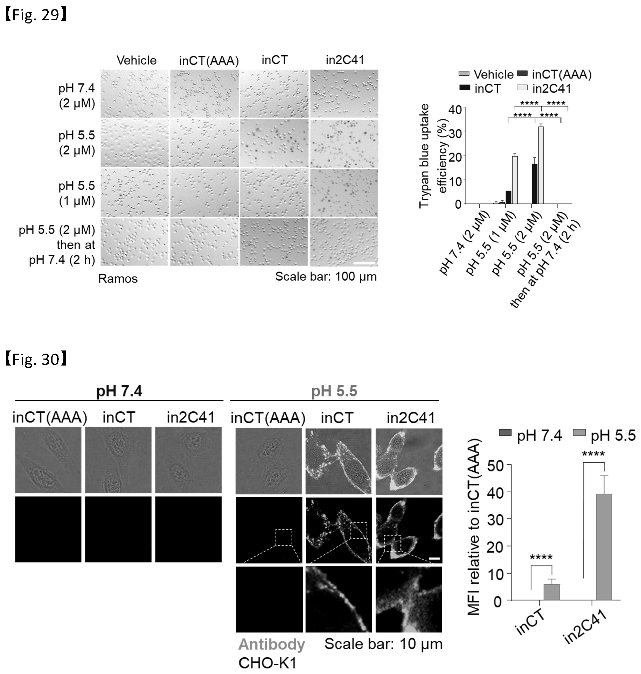

FIG. 29 shows the results of examining, by the live cell imaging device Lionheart Fx (Biotek), whether cells take up membrane-impermeable trypan blue through cell membrane permeabilization by in2C41 depending on pH and whether the cell membrane permeabilization is a transient and reversible phenomenon.

Specifically, in the same manner as in Example 9, Ramos cells were incubated with each of in2C41 or control antibodies at concentrations of 1 or 2 µM at each of pH 7.4 and pH 5.5 for 2 hours at 37°C. Here, to determine whether cell membrane permeabilization is transient and reversible, the cells incubated with the antibody (2 µM) at pH 5.5 were washed with PBS, and then incubated in RPMI at pH 7.4 for 2 hours. Subsequently, the cells were stained with trypan blue in the same manner as in Example 9 and then observed using a live cell imaging device.

In FIG. 29, the results of quantitatively comparing the number of cells that had taken up trypan blue are shown in a graph. Specifically, the number of cells that had taken up trypan blue out of the total number of cells was counted and expressed as a percentage. A total of 200 cells or more were counted and the mean value is shown in a graph. It was confirmed that, at pH 5.5, the cells incubated with in2C41 did take up trypan blue in proportion to the concentration. On the other hand, it was confirmed that, in the case of the cells incubated in the medium at pH 7.4 for 2 hours after incubation at pH 5.5, no uptake of trypan blue occurred, indicating that cell membrane permeabilization by in2C41 is a transient and reversible phenomenon.

FIG. 30 shows the results of confocal microscopy observation performed to examine whether in2C41 completely passes through the cell membrane and moves to the opposite side, depending on pH.

Specifically, 500 µL of 1 × 10⁵ CHO-K1 were seeded into each well of lysine-coated plates prepared in the same manner as in Example 18 and cultured for 24 hours at 37°C under 5% CO₂. In the same two buffer environments as in FIG. 30, the cells were treated with 5 µM of each of in2C41 and control antibodies and incubated at 37°C for 2 hours. Subsequently, PBS washing, fixation, cell permeabilization, and blocking processes were performed in the same manner as in Example 19, and the cells were treated with a FITC (green fluorescence)-linked anti-human Fc antibody (Sigma, F9512) for 1 hour at 25°C. Thereafter, the cells were washed twice with PBS and mounted on cover glass in the same manner as in Example 18.

As shown in FIG. 30, it was confirmed that, at pH 5.5, in2C41 can specifically localize in cells, and can pass through the membrane and move into the cytosol through cell membrane permeabilization created in the endosomal environment.

### Example 25. Evaluation of Thermal Stability of Cytosol-Penetrating Antibody Having Endosomal Escape Motifs in Heavy Chain Constant Region CH3 Domain and Light Chain Constant Region CL Domain.

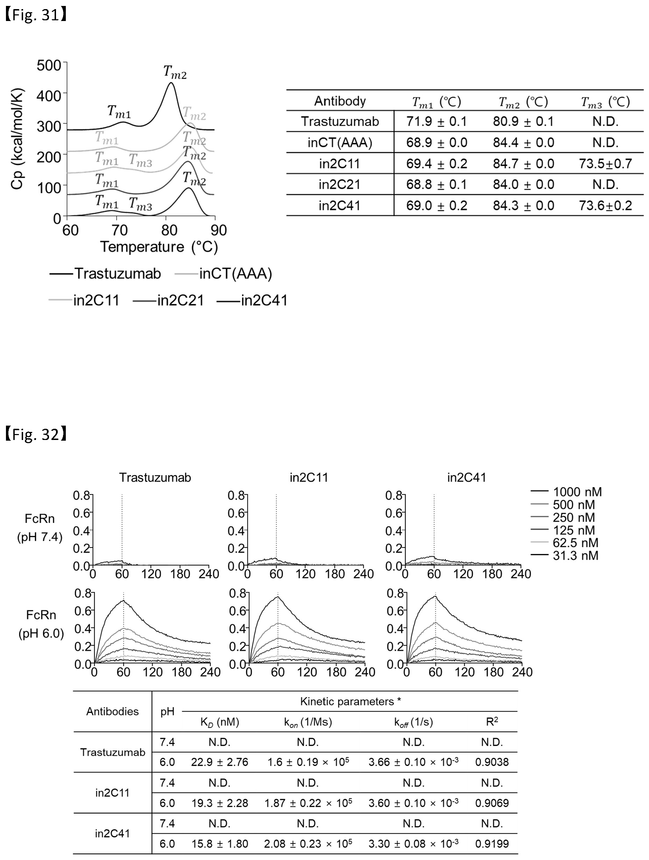

FIG. 31 shows the results of evaluating the thermal stability of cytosol-penetrating antibodies (in2C11, in2C21, and in2C41) having endosomal escape motifs in the antibody constant regions, their parent antibody inCT (AAA), and trastuzumab, an antibody approved for therapeutic uses, by differential scanning calorimetry.

Specifically, antibodies diluted to 1 mg/ml in histidine buffer (25 mM histidine, 150 mM NaCl, pH 6.5) were prepared and heated from 25°C to 90°C at a rate of 1°C/min on a MicroCal PEAQ-DSC (Malvern Panalytical, UK) instrument. Then, the melting temperature (Tm) of the antibody was calculated by subtracting the value measured only for the buffer under the same conditions.

As shown in FIG. 31, in2C21 having an endosomal escape motif in the CL domain was measured to have two melting points, Tₘ₁ (CH2 domain) and Tₘ₂ (Fab, CH3 domain), like inCT (AAA) and trastuzumab used as controls. In particular, the slight difference in melting point (Tₘ₂) from trastuzumab can be expected to be the difference caused by the VH and VL domains (Ionescu et al. 2008). On the other hand, the cytosol-penetrating antibodies (in2C11, and in2C41) having endosomal escape motifs in the CH3 domains were measured to have an additional melting point (Tₘ₃, CH3 domain) which is a temperature about 4°C higher than Tₘ₁. Thus, it was confirmed that the thermal stability was reduced by about 10°C due to the mutations introduced into the CH3 domain loops, similar to a previous study in which mutations were introduced into the CH3 domain loops to confer the ability to bind to a specific antigen (Lobner et al. 2017).

### Example 26. Examination of Retention of FcRn Binding Affinity of Cytosol-Penetrating Antibody Having Endosomal Escape Motifs in Heavy Chain Constant Region CH3 Domain and Light Chain Constant Region CL Domain.

FIG. 32 shows the results of performing biolayer interferometry using Octet Qke (Sartorius) to examine whether the cytosol-penetrating antibody (in2C41) having endosomal escape motifs in the CH3 and CL domains retains its binding affinity to FcRn, like wild-type IgG1 Fc.

Specifically, three antibodies, cytosol-penetrating antibodies (in2C11 and in2C41) having an endosomal escape motif in the antibody constant region, and trastuzumab, an antibody approved for therapeutic use, were each diluted to 50 µg/mL in 1X kinetics buffer (Sartorius, 18-1105) and fixed to 1.8 nm on a Fab2G biosensor (Sartorius, 18-5125). After stabilization in 100 mM PBS (pH 6.0 or pH 7.4) containing 0.05% Tween20, the binding affinity to recombinant human FcRn (SinoBiological, CT009-H08H) diluted in the same buffer to concentrations of 31.3, 62.5, 125, 250, 500, and 1,000 nM was measured. Based on the measured data, the affinity constant (*K*D) was obtained from a 1:1 Langmuir binding model (simultaneously *k*ₒₙ and *k*_{off}) and the ratio of the rate constant *k*_{off}/*k*ₒₙ.

The experimental results indicated that the cytosol-penetrating antibodies (in2C11 and in2C41) having endosomal escape motifs in the CH3 and CL domains exhibited binding ability similar to trastuzumab of wild-type IgG1 Fc. It was confirmed that, at pH 6.0, the binding affinity (*K*_{D}) to FcRn was similar at around 20 nM, and at pH 7.4, none of the antibodies bound to FcRn, suggesting that the endosomal escape motifs introduced into the CH3 and CL domains of in2C41 did not affect FcRn binding.

### Example 27. Evaluation of Pharmacokinetics of Cytosol-Penetrating Antibody Having Endosomal Escape Motifs in Heavy Chain Constant Region CH3 Domain and Light Chain Constant Region CL Domain.

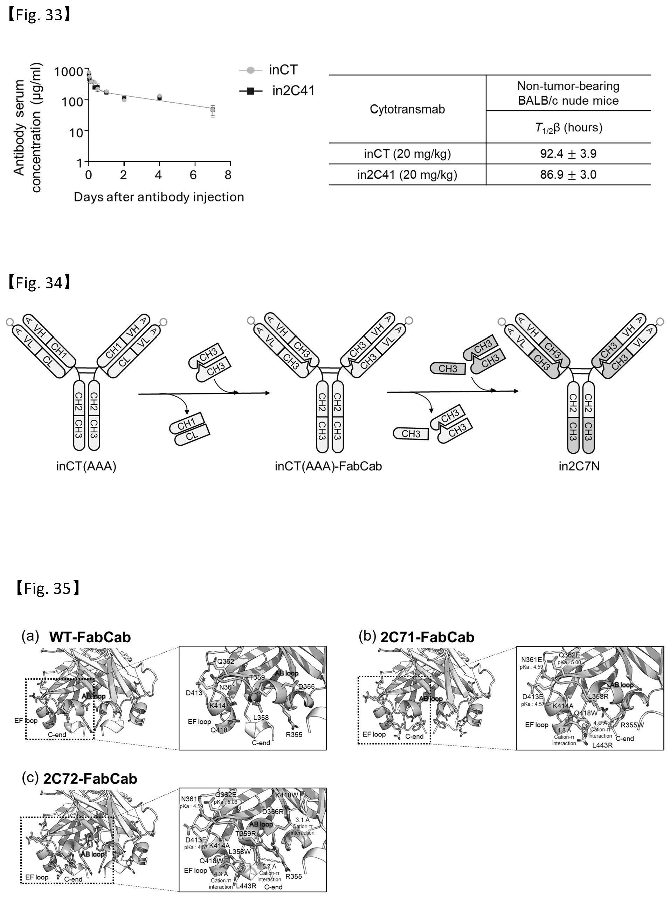

FIG. 33 shows the results of evaluating the pharmacokinetics of in2C41 in BALB/c nude mice.

Specifically, BALB/c nude mice were intravenously injected with each of the cytosol-penetrating antibody in2C41 and the control inCT at a dose of 20 mg/kg, and mouse blood was collected at each of 1, 4, 12, and 24 hours, and 2, 4, and 7 days. The collected blood was centrifuged at 12,000 rpm for 10 minutes at 4°C, and then only the supernatant plasma was stored at -80 °C. Subsequently, ELISA was performed to analyze the concentrations of in2C41 and inCT in the plasma. Specifically, anti-human Fab antibody (Sigma) was bound to each well of a 96-well half-area plate (Corning) at a concentration of 2.5 µg/ml for 1 hour at room temperature, and then the plate was washed 3 times with 0.1% PBST (PBS, pH 7.4, 0.1% Tween 20). After binding with 1% PBSB (PBS, pH 7.4, 1% BSA) for 1 hour, collected blood and purified antibody (for reference curve) were diluted in 1% PBSB and bound for 2 hours, and then the plate was washed 3 times with 0.1% PBST. Next, HRP-conjugated anti-human IgG Fc antibody (Sigma) as a detection antibody was bound for 1 hour, followed by washing 3 times with 0.1% PBST. The plate was incubated with TMB ELISA solution, the reaction was stopped with sulfuric acid solution, and then the absorbance at 450 nm was quantified.

As shown in FIG. 33, it was confirmed that in2C41 exhibited pharmacokinetics similar to the control antibody inCT (T_{1/2}β (inCT): 92.4 ± 3.9 h, T_{1/2}β (in2C41): 86.9 ± 3.0 h), indicating that the endosomal escape motifs introduced into the antibody constant regions did not affect the half-life of the antibody.

### Example 28. Design of Cytosol-Penetrating Antibody (in2C71) Having Endosomal Escape Motifs in FabCab Domain, in Which CH1-CL Pair of Antibody is Replaced with CH3-CH3 Pair, and CH3 Domain.

Following the Fcab (Fc with antigen-binding constant domains) technology of conferring antigen-binding ability to the CH3 domain, the FabCH3 technology of improving thermal stability by replacing the CH1-CL pair in the antibody Fab fragment with a CH3-CH3 pair was reported. As a fusion of the two technologies, the FabCab (Fab with antigen-binding constant domains) technology of conferring antigen-binding ability to the CH3 domain of FabCH3 was reported. Using this technique, cytosol-penetrating antibodies were constructed by replacing the CH1-CL pair of antibody Fab with a CH3-CH3 pair having an endosomal escape motif. Here, the FabCab domain variants in which the CH1-CL pair of antibody Fab is replaced with a CH3-CH3 pair having an endosomal escape motif were named in2C7N. In this case, when both CH3 replacing CH1 and CH3 replacing CL are of wild type, light chain homodimers or heavy chain oligomers may be produced during the protein assembly process. To prevent this, EW-RVT mutations for heterodimeric Fc pair formation were introduced into the CH3 pair (Korean Patent No. KR10-1522954).

FIG. 34 is a schematic diagram showing a FabCab domain variant (in2C7N) with enhanced cytosol-penetrating ability in which the CH1-CL pair is replaced with a heterodimeric CH3-CH3 pair having an endosomal escape motif. in2C7N was constructed by introducing an endosomal escape motif into the heterodimeric CH3 pair in inCT(AAA)-FabCab in which the CH1-CL pair of inCT(AAA) was substituted with an EW-RVT heterodimeric CH3 pair.

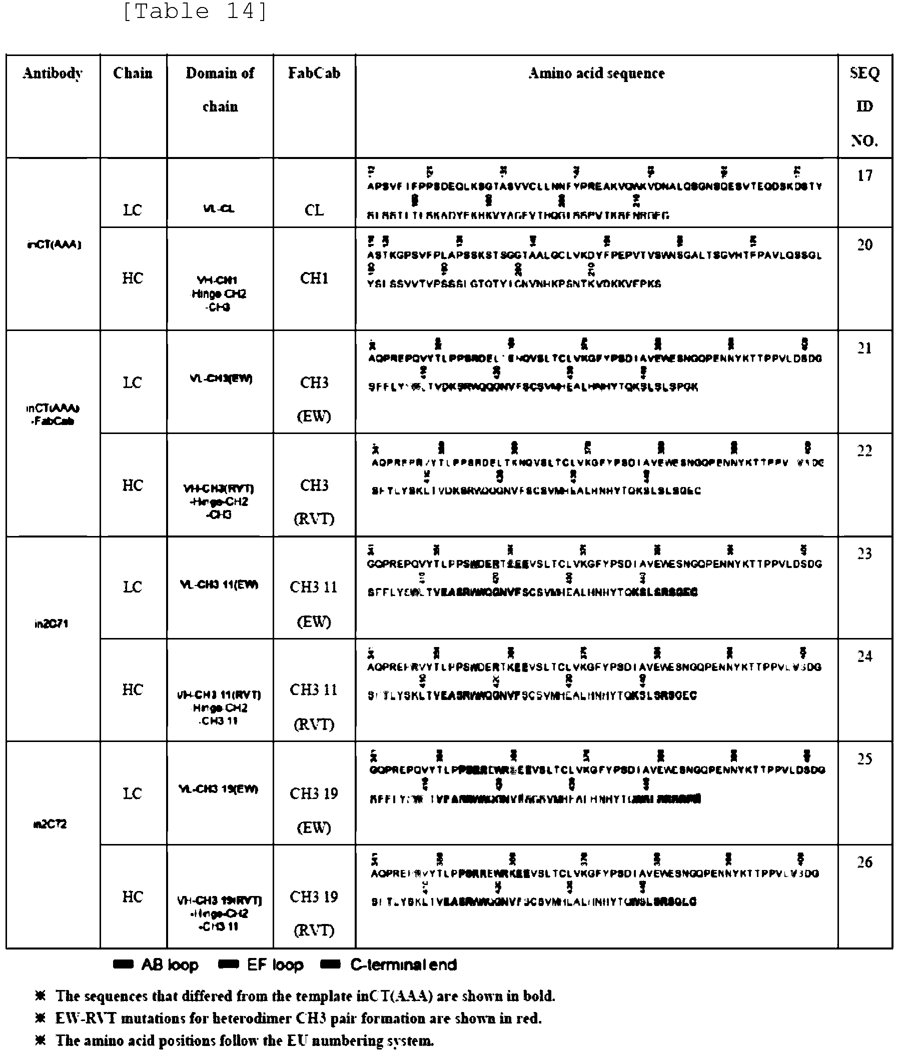

Specifically, FabCab domain variants were constructed using a heavy chain comprising the VH of SEQ ID NO: 14 and the heavy chain variable region (CH3(RVT)-hinge-CH2-CH3) of SEQ ID NO: 22, 24 or 26 having an RVT mutation introduced thereinto, and a light chain comprising the VL of SEQ ID NO: 16 and the CH3 domain of SEQ ID NO: 21, 23 or 25 having an EW mutation introduced thereinto. The constituent domains and amino acid sequences of the heavy chains and light chains of the FabCab domain variants (in2C7N) are shown in Table 16 above.

FIG. 35 shows the results of modeling, using AlphaFold2, the 3D structures of FabCab fragments in which the CH1-CL pair of the wild-type Fab domain is replaced with a CH3-CH3 pair having an endosomal escape motif and a mutation for heterodimer formation, and the results of analyzing the intramolecular interaction patterns of the antibodies using the PyMOL molecular visualizer. Thereby, it was confirmed that the 3D structures of the FabCab domain variants (in2C71 and in2C72) are similar to the folded state of the 3D structure of the wild-type IgG1 Fc in FIG. 2a.

**[Table 15]**

| FabCab variants | RMSD (Å) |
|---|---|
| FabCab Wild type | 0.189 |
| in2C71 | 0.172 |
| in2C72 | 0.141 |

To analyze the structural differences between the FabCab fragments of the FabCab domain variants (in2C71 and in2C72) and the wild-type Fc region, the RMSD of the FabCab CH3 domain heterodimer with respect to the CH3 domain homodimer (CH3-CH3 pair) was calculated, and the results are shown in Table 15 above. It was confirmed that the RMSD value of wild-type FabCab and wild-type IgG1 Fc having no endosomal escape motif was 0.189 Å, the RMSD values of in2C71 and in2C11 were 0.172 Å, and the RMSD value of in2C72 and in2C19 was 0.141 Å, indicating that all of the RMSD values were 0.2 Å or less, suggesting that they are very similar in structure to the wild type.

As shown in FIG. 35a, it was confirmed that the inCT(AAA) FabCab consisting of SEQ ID NOs: 21 and 22 folds into the same form as the wild type Fc of FIG. 2a.

In in2C71 consisting of SEQ ID NOs: 23 and 24, the endosomal escape motif of the CH3 domain of in2C11 were introduced into the FabCab CH3-CH3 pair of inCT(AAA)-FabCab. The heterodimeric CH3-CH3 pair is formed between the FabCab heavy chain and light chain by EW-RVT, similar to the inCT(AAA)-FabCab mentioned above. The endosomal escape motif consists of two tryptophan mutations (R355W and Q418W), two arginine mutations (L358R and L443R), one alanine mutation (K414A), and three glutamic acid mutations (N361E, Q3622, and D413E), which are present in the AB loop, EF loop, and C-terminal loop, like that in 2C11. As shown in FIG. 35b, the intramolecular interaction pattern of the endosomal escape motif was identical to that in 2C11 of FIG. 2b.

In in2C72 consisting of SEQ ID NOs: 25 and 26, the endosomal escape motif of the CH3 domain of in2C19 was introduced into the FabCab CH3-CH3 pair of inCT(AAA)-FabCab. The heterodimeric CH3-CH3 pair is formed between the FabCab heavy chain and light chain by EW-RVT, similar to inCT(AAA)-FabCab. The endosomal escape motif consists of three tryptophan mutations (L358W, Q418W, and K439W), three arginine mutations (D356R, T359R, and L443R), one alanine mutation (K414A), and three glutamic acid mutations (N361E, Q362E, and D413E), which are present in the AB loop, EF loop, and C-terminal loop, like that in 2C19. As shown in FIG. 35c, the intramolecular interaction pattern of the endosomal escape motif was identical to that in 2C19 of FIG. 2l.

### Example 29. Expression and Purification of Cytosol-Penetrating Antibodies (in2C7N-GFP11-SBP2) Having Endosomal Escape Motifs in FabCab Domain, in which CH1-CL Pair of Antibody Is Replaced with CH3-CH3 Pair, and CH3 Domain.

To identify the biophysical properties of in2C71, in which the endosomal escape motif of in2C11 is introduced into the FabCab domain, and in2C72, in which the endosomal escape motif of in2C19 is introduced into the FabCab domain, and to evaluate the endosomal escape ability through an enhanced split green fluorescent protein complementation system, in2C7N-GFP11-SBP2 in which GFP11-SBP2 is fused to the C-terminus of the antibody heavy chain was constructed. In addition, for comparison of the biophysical properties, the wild-type FabCab format antibody inCT(AAA)-FabCab-GFP11-SBP2 having no endosomal escape motif was also constructed.

As in the previous Example, a cyclic peptide (in4, DGVRQCRGDCFDGPL) targeting integrin αvβ3/αvβ5, membrane protein receptors overexpressed on the surface of tumor cells/tissue, was fused to the N-terminus of the light chain of the cytosol-penetrating antibody to induce endocytosis mediated by the tumor cell/tissue-specific receptors.

Specifically, to construct a light chain expression vector for producing inCT(AAA)-FabCab-GFP11-SBP2 as a full-length IgG monoclonal antibody, a DNA, which has a secretory signal peptide-encoding DNA fused to the 5' end and encodes a light chain comprising an antibody VL (SEQ ID NO: 16) and a CH3(EW) (SEQ ID NO: 23) into which an EW mutation for heterodimer formation has been introduced, was cloned into the pcDNA3.4 vector by NotI/HindIII. In addition, to construct a vector that expresses a heavy chain, a DNA, which has a secretory signal peptide-encoding DNA fused to the 5' end and encodes a heavy chain comprising an antibody VH (SEQ ID NO: 14) and a CH3(RVT) (SEQ ID NO: 24) into which an RVT mutation for heterodimer formation has been introduced, was cloned into the pcDNA3.4 vector by NotI/HindIII. In this case, to construct a GFP11-SBP2-fused cytosol-penetrating antibody, GFP11-(G₄S)₃-SBP2 was fused to the C-terminus of the heavy chain using with three GGGS linkers.

To construct a light chain expression vector for producing in2C71-GFP11-SBP2 as a full-length IgG monoclonal antibody, a DNA, in which a secretory signal peptide-encoding DNA fused to the 5' end and encodes a light chain comprising an antibody VL (SEQ ID NO: 16) and a CH3 11(EW) (SEQ ID NO: 25) into which an EW mutation for heterodimer formation and an endosomal escape motif have been introduced, was cloned into the pcDNA3.4 vector by NotI/HindIII. In addition, to construct a vector that expresses a heavy chain, a DNA, which has a secretory signal peptide-encoding DNA fused to the 5' end and encodes a heavy chain comprising an antibody VH (SEQ ID NO: 14) and a CH3 11 (RVT) (SEQ ID NO: 26) into which an RVT mutation for heterodimer formation and an endosomal escape motif have been introduced, was cloned into the pcDNA3.4 vector by NotI/HindIII. Likewise, to construct a GFP11-SBP2-fused cytosol-penetrating antibody, GFP11-(G4S)3-SBP2 was fused to the C-terminus of the heavy chain using three GGGS linkers.

To construct a light chain expression vector for producing in2C72-GFP11-SBP2 as a full-length IgG monoclonal antibody, a DNA, which has a secretory signal peptide-encoding DNA fused to the 5' end and encodes a light chain comprising an antibody VL (SEQ ID NO: 16) and a CH3 19(EW) (SEQ ID NO: 27) into which an EW mutation for heterodimer formation and an endosomal escape motif have been introduced, was cloned into the pcDNA3.4 vector by NotI/HindIII. In addition, to construct a vector that expresses a heavy chain, a DNA, which has a secretory signal peptide-encoding DNA fused to the 5' end and encodes a heavy chain comprising an antibody VH (SEQ ID NO: 14) and a CH3 19 (RVT) (SEQ ID NO: 28) into which an RVT mutation for heterodimer formation and an endosomal escape motif have been introduced, was cloned into the pcDNA3.4 vector by NotI/HindIII. Likewise, to construct a GFP11-SBP2-fused cytosol-penetrating antibody, GFP11-(G4S)3-SBP2 was fused to the C-terminus of the heavy chain using three GGGS linkers.

The three types of FabCab format cytosol-penetrating antibodies were purified in the same manner as in Example 3 after transiently transfecting HEK293-F cells with a light chain expression vector of VL-CH3(EW) comprising the VL of SEQ ID NO: 16 and the CH3(EW) of SEQ ID NO: 21, 23 or 25, and a heavy chain expression vector of VH-CH3(RVT)-hinge-CH2-CH3 comprising the VH of SEQ ID NO. 14 and the CH3(RVT) of SEQ ID NO. 22, 24 or 26.

**[Table 16]**

| GFP11-SBP2 fused antibodies | Size (kDa) | Purification yield (mg/L of transfected cells) | Relative yield (%) |
|---|---|---|---|
| inCT | 162.9 | 40.6±2.6 | 84.5 |
| inCT(AAA) | 162.1 | 50.4±3.8 | 100 |
| inCT(AAA)-FabCab | 166.0 | 11.9±2.1 | 23 |
| in2C71 | 166.9 | 12.5±1.4 | 25 |
| in2C72 | 167.8 | 0.34±0.01 | 0.7 |

Subsequently, the purification of the FabCab domain variants and protein quantification were performed in the same manner as in Example 3. The production yields of these variants in animal cells are shown in Table 16 above. in2C72-GFP11-SBP2 showed a significantly low yield of 0.34 mg per liter. inCT(AAA)-FabCab-GFP11-SBP2 and in2C71, in2C72-GFP11-SBP2 showed a significantly decreased yield of about 75% relative to the wild-type inCT(AAA)-GFP11-SBP2, but showed a good yield of 10 mg or more per liter.

The FabCab domain variants (in2C7N-GFP11-SBP2) fused with a cyclic peptide and GFP11-SBP2 that target membrane protein receptors overexpressed on the tumor cell surface are hereinafter referred to as in2C7N. For example, in2C71-GFP11-SBP2 is in2C71.

### Example 30. Evaluation of Physical Properties of Cytosol-Penetrating Antibody (in2C7N) Having Endosomal Escape Motifs in FabCab, in which CH1-CL Pair of Antibody Is Replaced with CH3-CH3 Pair, and CH3 Domain.

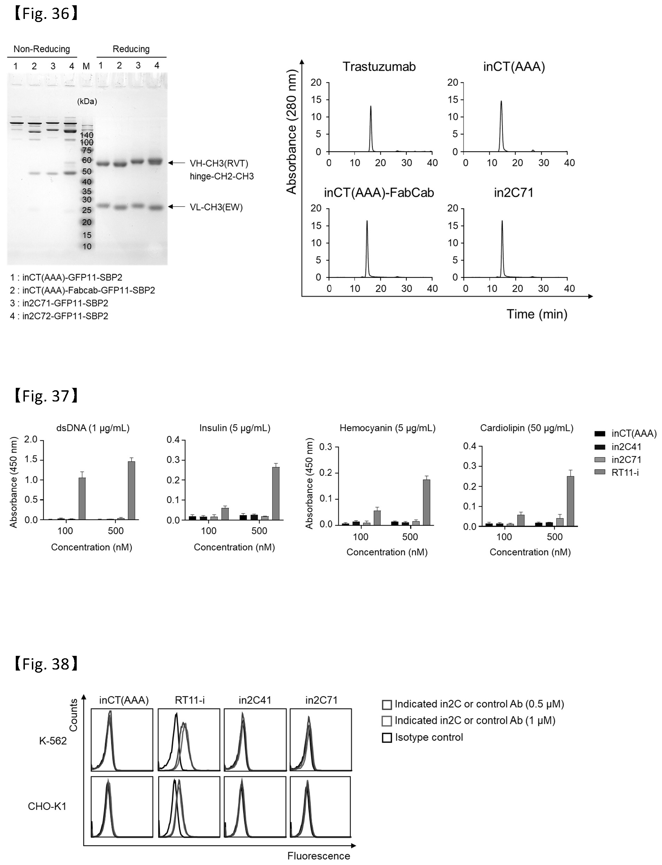

FIG. 36 shows the results of analyzing the physical properties of in2C71, in2C72, and inCT(AAA)-FabCab by 12% SDS-PAGE under reducing or non-reducing conditions and size exclusion chromatography after purification. Specifically, it was confirmed that all the three antibodies had a molecular weight of about 166 to 167 kDa under non-reducing conditions, and that under reducing conditions, the molecular weight of the heavy chain was about 56 kDa (antibody heavy chain: about 51 kDa, and GFP11-SBP2 fragment: about 5 kDa) and the molecular weight of the light chain was about 26.8 kDa (antibody light chain: about 25.3 kDa, and in4 cyclic peptide: 1.5 kDa). However, a band with a size of about 50 kDa was observed under non-reducing conditions, which was thought to be because the two light chains of VL-CH3(EW) formed some homodimers. When the mAU values at 280 nm of the samples were measured using size exclusion chromatography (Superdex 200 Increase 10/300GL, Cytiva) in the same manner as in Example 4, single peaks without any special oligomers or tailing were observed for both in2C71 and inCT(AAA)-FabCab, as in the controls Trastuzumab and inCT(AAA).

Thereby, it was confirmed that in2C71 having endosomal escape motifs in three CH3-CH3 pairs has a good production yield of 10 mg or more per liter (Table 16) and physical properties similar to the control wild-type antibody.

### Example 31. Evaluation of Non-Specific Binding of Cytosol-Penetrating Antibody Having Endosomal Escape Motifs in FabCab, in which CH1-CL Pair of Antibody Is Replaced with CH3-CH3 Pair, and CH3 Domain.

FIG. 37 shows the results of performing ELISA on four antigens, dsDNA, insulin, keyhole limpet hemocyanin (KLH) and cardiolipin, which allow the evaluation of the non-specific binding of the cytosol-penetrating antibody (in2C71) having endosomal escape motifs in three CH3-CH3 pairs. The experiment was performed in the same manner as in Example 5.

In the above experiment, when the antibody at a concentration of 100 nM shows an absorbance of 0.05 or higher at 450 nm for at least one of the four antigens, it is determined to have non-specific binding. It was confirmed that in2C71 does not have any special non-specific binding to the four antigens, similar to the negative control antibody.

FIG. 38 shows the results of performing flow cytometry analysis to evaluate the non-specific binding of the FabCab format cytosol-penetrating antibody in2C71 to the cell membrane. The experimental results indicated that in2C71 did not exhibit non-specific binding to the cell surfaces of K-562 and CHO-K1 cells, similar to the wild-type negative control.

### Example 32. Evaluation of Cytosol-Penetrating Ability of Cytosol-Penetrating Antibody (in2C71) Having Endosomal Escape Motifs in FabCab, in which CH1-CL Pair of Antibody Is Replaced with CH3-CH3 Pair, and CH3 Domain.

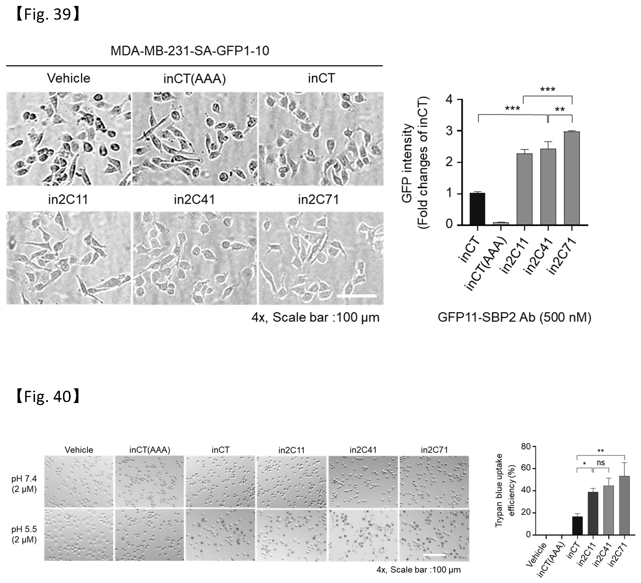

FIG. 39 shows the results of observing and quantifying GFP fluorescence generated by complementation of enhanced split green fluorescent protein using a live cell imaging device, Lionheart Fx (Biotek), to evaluate the cytosol-penetrating ability of the cytosol-penetrating antibodies (in2C41 and in2C71) having endosomal escape motifs in the antibody constant region. The experimental procedure and quantification of intracellular fluorescence intensity were performed in the same manner as in Example 7. The results of the experiment indicated that in2C71 exhibited an about 3-fold higher cell-based fluorescence intensity than inCT. Thereby, it was confirmed that in2C71, which has endosomal escape motifs in three CH3-CH3 pairs as a result of replacing the CH1-CL pair with a CH3-CH3 pairs, has cytosol-penetrating ability that is about 3-fold higher than in2C41 (about 2.5-fold higher than inCT) through the enhancement effect of multiple endosomal escape motifs.

### Example 33. Evaluation of pH-Dependent Interaction between Cytosol-Penetrating Antibody Having Endosomal Escape Motifs in Constant Region and Cell Membrane.

FIG. 40 shows the results of examining whether the Ramos cell line can take up membrane-impermeable trypan blue through pore formation caused by the cytosol-penetrating antibody having endosomal escape motifs in the constant region, at varying pHs using the live cell imaging device Lionheart Fx (Biotek). The experiment was performed in the same manner as in Example 9.

In FIG. 40, the results of quantitatively comparing the number of cells that had taken up trypan blue are shown as a graph. Specifically, the number of cells that had taken up trypan blue out of the total number of cells was counted and expressed as a percentage. A total of 200 cells or more were counted and the mean value is shown in the graph. It was confirmed that the cells treated with the cytosol-penetrating antibody having endosomal escape motifs in the constant region only took up trypan blue at pH 5.5. In particular, in2C41 and in2C71, which have high cytosol-penetrating ability, showed a high trypan blue uptake (%) of about 50% only at pH 5.5. Thereby, it was confirmed that the cytosol-penetrating antibodies (in2C41 and in2C71) having endosomal escape motifs in the constant region according to the present invention can interact with the cell membrane in a weakly acidic environment-specific manner through the glutamic acid mutations.

### Example 34. Design of Anti-α-Tubulin Cytosol-Penetrating Antibody (inTu41) in Full-Length IgG Form Targeting Intracellular α-Tubulin.

The novel high-efficiency cytosol-penetrating antibody having an endosomal escape motif in the constant region will ultimately localize in the cytosol in an increased amount, and can thus more effectively target proteins located in the cytosol.

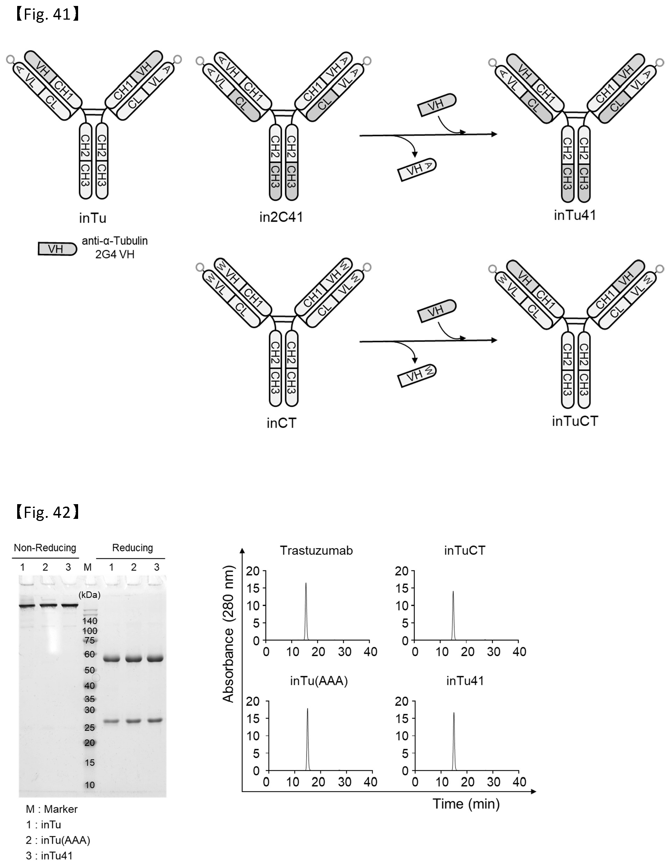

FIG. 41 is a schematic diagram illustrating the construction of an anti-α-tubulin cytosol-penetrating antibody (inTu41) in full-length IgG form to evaluate the activity of in2C41 having improved cytosol-penetrating ability. The antibody variable region sequence required for the construction of the anti-α-tubulin cytosol-penetrating antibody is shown in Table 17 below.

For the expression of an anti-α-tubulin cytosol-penetrating monoclonal antibody in full-length IgG form in animal cells, a DNA, which has a secretory signal peptide-encoding DNA fused to the 5' end and encodes a heavy chain comprising a heavy chain variable region VH (Protein Eng. Des. Sel., 2011, 24(12); 873-881, SEQ ID NO: 27), which specifically binds to cytoskeletal α-tubulin, and a heavy chain constant region (CH1-hinge-CH2-CH3) comprising the CH3 domain of in2C41 (SEQ ID NO: 4), was cloned into the pcDNA3.4 vector by NotI/HindIII. Also, a DNA, which has a secretory signal peptide-encoding DNA fused to the 5' end and encodes a light chain comprising a light chain variable region (VL, SEQ ID NO. 16) and the CL domain of in2C21 (SEQ ID NO: 18), was cloned into the pcDNA3.4 vector by NotI/HindIII. Thereafter, the animal expression vectors encoding the heavy and light chains were transiently co-transfected into HEK293-F cells, and the antibody was purified in the same manner as in Example 3.

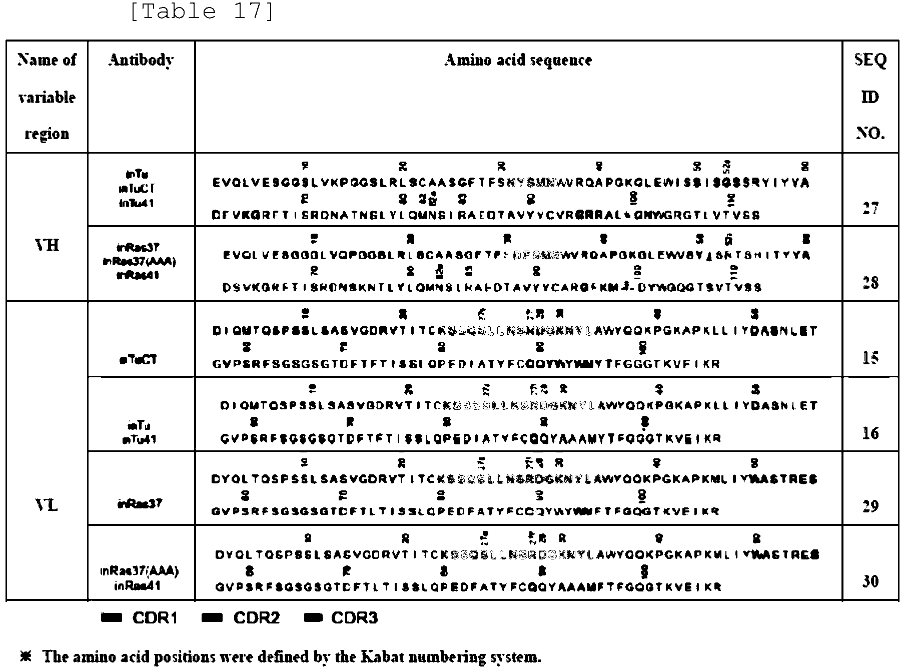

In addition, inTu, which has an anti-α-tubulin VH in a wild-type antibody having no cytosol-penetrating ability, was constructed and used as a control for the experiment, and an inCT-based anti-α-tubulin cytosol-penetrating antibody (inTuCT), which has been reported to penetrate the cytosol through the cell-penetrating light chain VL (SEQ ID NO: 15) and target intracellular α-tubulin through the anti-α-tubulin VH (SEQ ID NO: 27), was constructed and used as a control for the experiment.

To construct inTu, a DNA, which has a secretory signal peptide-encoding DNA fused to the 5' end and encodes a heavy chain having a heavy chain variable region VH (SEQ ID NO: 27), which specifically binds to cytoskeletal α-tubulin, and a wild-type heavy chain constant region (CH1-hinge-CH2-CH3), was cloned into the pcDNA3.4 vector by NotI/HindIII. In addition, a DNA, which has a secretory signal peptide-encoding DNA fused to the 5' end and encodes a light chain comprising a light chain variable region (VL, SEQ ID NO: 16) and a wild-type light chain constant region (CL, SEQ ID NO: 17), was cloned into the pcDNA3.4 vector by NotI/HindIII. Thereafter, the animal expression vectors encoding the heavy chain and light chain were transiently co-transfected into HEK293-F cells, and the antibody was purified in the same manner as in Example 3.

In the case of inTuCT, the animal expression vectors encoding the heavy chain of inTu and the light chain of inCT were transiently co-transfected into HEK293-F cells in the same manner, and the antibody was purified.

**[Table 18]**

| Anti-α-Tubulin antibodies | Domains for endosomal escape | Size (kDa) | Purification yield (mg/L of transfected cells) | Relative yield (%) |
|---|---|---|---|---|
| inTu | - | 150.1 | 25.0 | 100 |
| inTuCT | VL | 150.7 | 21.5 | 86 |
| inTu41 | CH3, CL | 150.9 | 23.1 | 92 |

Protein quantification of all the anti-α-tubulin antibodies in full-length IgG form was performed in the same manner as in Example 3. The production yields of inTu, inTuCT and inTu41 are shown in Table 18 above. inTuCT, which has cytosol-penetrating ability conferred by the light chain variable region VL, and inTu41, which has cytosol-penetrating ability conferred by the constant region CH3 and CL, both showed a good yield of 20 mg per or more liter, similar to that of inTu.

FIG. 42 is a graph showing the results of measuring the mAU values at 280 nm of the anti-α-tubulin antibodies in full-length IgG form and the control antibody using size exclusion chromatography (Superdex 200 Increase 10/300GL, Cytiva) in the same manner as in Example 4. Single peaks without oligomers or tailing were observed for all the three anti-α-tubulin antibodies, as in the control group Trastuzumab.

### Example 35. Examination of Whether Anti-α-Tubulin Cytosol-Penetrating Antibody (inTu41) in Full-Length IgG Form Overlaps with Cytoskeletal Protein α-Tubulin

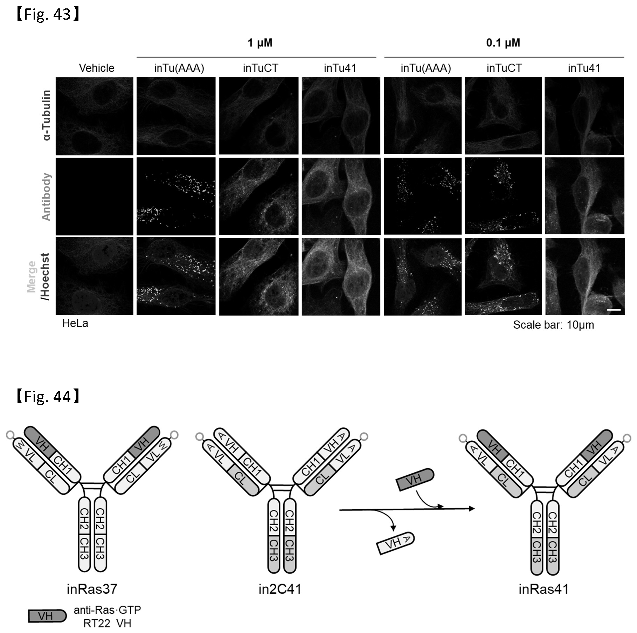

FIG. 43 shows the results of confocal microscopy performed to examine whether the anti-α-tubulin cytosol-penetrating antibody (inTu41) in full-length IgG form overlaps with the cytoskeletal protein α-tubulin localized in the cytosol.

Specifically, 500 µL of 1 × 10⁵ HeLa cells were seeded into each well of a lysine-coated plate prepared in the same manner as in Example 18, and cultured for 24 hours at 37°C under 5% CO₂. After removing the supernatant, the cells were treated with each of the anti-α-tubulin cytosol-penetrating antibody (inTu41) and the control antibody at a concentration of 0.1 or 1 µM for 12 hours at 37°C. Subsequently, PBS washing, acid washing, cell fixation, cell permeabilization, and blocking processes were performed in the same manner as in Example 23. Then, the cells were treated with anti-α-tubulin mouse antibody (Santa Cruz, sc-32293) diluted in blocking solution, as a primary antibody for α-tubulin staining, at 25°C for 1 hour. After washing three times with PBS, the intracellular antibody and anti-α-tubulin mouse antibody were reacted with FITC (green fluorescence)-linked anti-human Fc antibody (Sigma, F9512) and Alexa Fluor 647 (red fluorescence)-linked anti-mouse IgG antibody (Invitrogen, A21235), respectively, diluted in blocking solution, at 25°C for 1 hour. Next, PBS staining, nucleus staining, sampling, and confocal microscopic observation were performed in the same manner as in Example 18.

As shown in FIG. 43, in the case of inTu(AAA) having no cytosol-penetrating ability, a dot-shaped fluorescent signal in which the antibody was trapped in the endosome was observed regardless of the antibody concentration. On the other hand, in the cells treated with inTu41, inTu41 labeled with green fluorescence and α-tubulin labeled with red fluorescence in the cytosol overlapped at both concentrations. In particular, inTuCT, used as a positive control at a concentration of 0.1 µM, was observed to be trapped in the endosome, like inTu(AAA) used as a negative control, confirming that inTu41 developed in the present invention more effectively localizes in the cytosol than inTuCT.

### Example 36. Design of RAS-Targeting Cytosol-Penetrating Antibody (inRas41) in Full-Length IgG Form Targeting Intracellular Activated RAS.

An experiment was conducted to examine whether other proteins in the cytosol in addition to the cytoskeletal α-tubulin could be effectively targeted. FIG. 44 is a schematic diagram illustrating the construction of a RAS-targeting cytosol-penetrating antibody in full-length IgG form to confirm the activity of in2C41 with enhanced cytosol-penetrating ability.

The antibody variable region sequence required to construct the anti-RAS cytosol-penetrating penetrating antibody is shown in Table 19 above. For the expression of the anti-RAS cytosol-penetrating monoclonal antibody in full-length IgG form in animal cells, a DNA, which has a secretory signal peptide-encoding DNA fused to the 5' end and encodes a heavy chain comprising RT22 VH (Science Advances, 2020, 6(3):eaay2174, sequence number 28), which specifically binds to RAS, and CH1-hinge-CH2-CH3 comprising the CH3 domain of in2C41 (SEQ ID NO: 4), was cloned into the pcDNA3.4 vector by NotI/HindIII. Also, a DNA, which has a secretory signal peptide-encoding DNA fused to 5' end and encodes a light chain comprising a light chain variable region (VL, SEQ ID NO. 30) and the CL domain of in2C41 (SEQ ID NO: 18), was cloned into the pcDNA3.4 vector by NotI/HindIII. Thereafter, the animal expression vectors encoding the heavy chain and light chain were transiently co-transfected into HEK293-F cells, and the antibody was purified in the same manner as in Example 3.

In addition, an anti-RAS cytosol-penetrating antibody (inRas37), which has been reported to penetrate the cytosol through the cell-penetrating light chain VL (SEQ ID NO: 29) and target intracellular RAS through the anti-RAS RT22 VH (SEQ ID NO: 28), was constructed and used as a positive control for intracellular RAS targeting, and inRas37(AAA) containing an anti-RAS RT22 VH (SEQ ID NO: 28) in the wild-type VL (SEQ ID NO: 30) having no cytosol-penetrating ability was constructed and used as a negative control for cytosol-penetrating ability.

To construct inRas37 and inRas37(AAA), a DNA, which has a secretory signal peptide-encoding DNA fused to the 5' end and encodes a heavy chain comprising a heavy chain variable region VH (SEQ ID NO: 28), which specifically binds to RAS, and a wild-type heavy chain constant region (CH1-hinge-CH2-CH3), was cloned into the pcDNA3.4 vector by NotI/HindIII. Also, a DNA, which has a secretory signal peptide-encoding DNA fused to the 5' end and which encodes a light chain comprising a light chain variable region (VL, SEQ ID NO: 29 or 30) and a wild-type light chain constant region (CL, SEQ ID NO: 17), was cloned into the pcDNA3.4 vector by NotI/HindIII. Subsequently, the vectors were transiently co-transfected into HEK293-F cells, and the antibody was purified in the same manner as in Example 3.

Protein quantification of all the anti-RAS antibodies in full-length IgG form was performed in the same manner as in Example 3. The purification yields of inRas37, inRas37(AAA), and inRas41 are shown in Table 19 below. inRAS41, which has endosomal escape motif in the constant regions CH3 and CL, showed a decreased yield of 42% relative to inRAS37, but a good yield of about 27 mg per liter.

**[Table 19]**

| Anti-RAS antibodies | Domains for endosomal escape | Size (kDa) | Purification yield (mg/L of transfected cells) | Relative yield (%) |
|---|---|---|---|---|
| inRas37(AAA) | - | 150.2 | 64.5 | 100.0 |
| inRas37 | VL | 151.0 | 105.0 | 162.8 |
| inRas41 | CH3, CL | 151.0 | 27.5 | 42.6 |

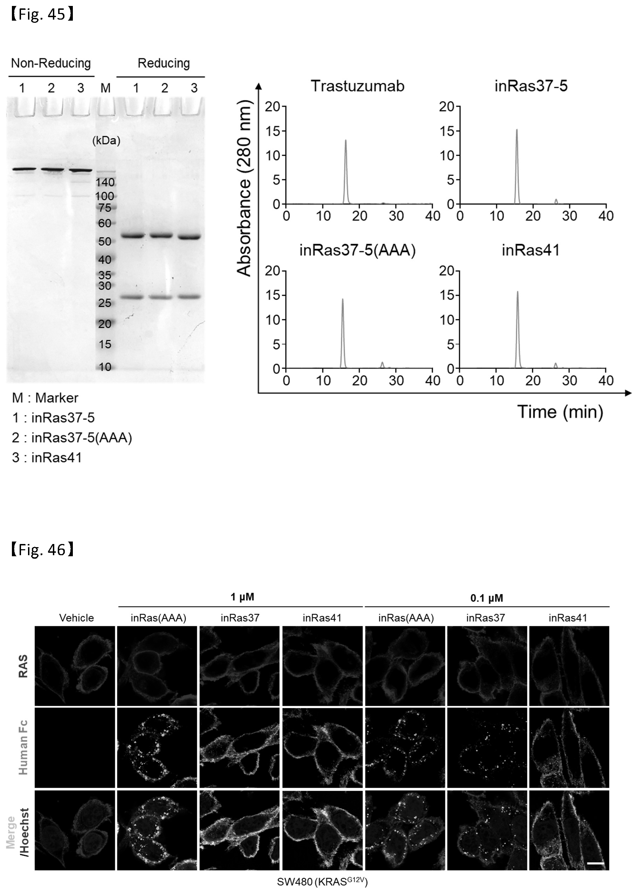

FIG. 45 shows the results of analyzing the physical properties of the anti-RAS cytosol-penetrating antibody (inRas41) having an endosomal escape motif in the constant region and the control antibody by 12% SDS-PAGE under reducing or non-reducing conditions and size exclusion chromatography after purification. Specifically, it was confirmed that all the three antibodies in Table 21 had a molecular weight of about 150 kDa under non-reducing conditions, and that under reducing conditions, the molecular weight of the heavy chain was about 49 kDa and the molecular weight of the light chain was about 26.5 kDa (antibody light chain: about 25 kDa, and in4 cyclic peptide: 1.5 kDa). When the mAU values at 280 nm were measured using size exclusion chromatography (Superdex 200 Increase 10/300GL, Cytiva) in the same manner as in Example 4, single peaks without oligomers or tailing were observed for all the three anti-RAS antibodies, as in the control Trastuzumab.

### Example 37. Examination of Whether Anti-RAS Cytosol-Penetrating Antibody in Full-Length IgG Form Overlaps with RAS.

FIG. 46 shows the results of examining whether the anti-RAS cytoplasmic penetrating antibody (inRas41) in full-length IgG form overlaps with the RAS located in the cytosol using a confocal microscope.

Specifically, 500 µL of 2 × 10⁵ SW480 cells were seeded into each well of a lysine-coated plate prepared in the same manner as in Example 18 and cultured for 24 hours at 37°C under 5% CO₂. After removing the supernatant, the cells were treated with each of an anti-α-Ras cytosol-penetrating antibody (inRas41) and a control antibody at a concentration of 0.1 or 1 µM at 37°C for 12 hours. Thereafter, PBS washing, acid washing, cell fixation, cell permeabilization, and blocking processes were performed in the same manner as Example 23. Next, the cells were treated with anti-Ras rabbit antibody (Abcam, ab108602) diluted in blocking solution, as a primary antibody for RAS staining, at 25°C for 4 hours. After washing three times with PBS, the intracellular antibody and the anti-Ras rabbit antibody were reacted with FITC (green fluorescence)-linked anti-human Fc antibody (Sigma, F9512) and Alexa Fluor 555 (red fluorescence)-linked anti-rabbit IgG antibody (Invitrogen, A21428), respectively, diluted in blocking solution, treated at 25°C for 1 hour. Next, PBS washing, nucleus staining, sampling, and confocal microscopic observation were performed in the same manner as in Example 18.

As shown in FIG. 46, in the case of inRas(AAA) having no cytosol-penetrating ability, a dot-shaped fluorescent signal was observed where the antibody was trapped in the endosome regardless of the antibody concentration. On the other hand, in the cells treated with inRas41, inRas41 labeled with green fluorescence and RAS labeled with red fluorescence in the cytosol overlapped at both concentrations. In particular, at a concentration of 0.1 µM, inRas37 used as a positive control was observed to be trapped in the endosome, like inRas(AAA) used as a negative control, suggesting that inRas41 developed in the present invention more effectively localizes in the cytosol than inRas37.

### Example 38. Design of Immunotoxin Having Cytosol-Penetrating Ability

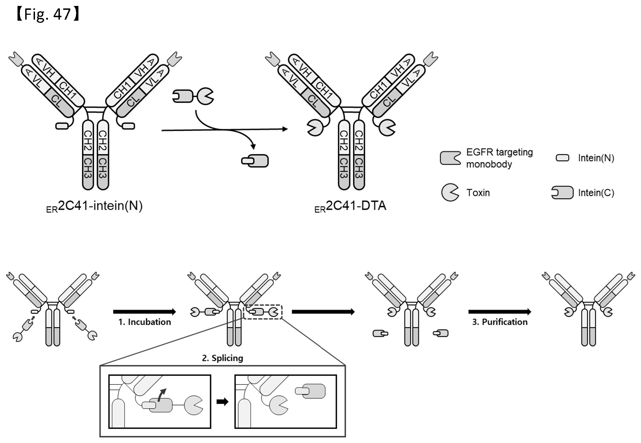

To evaluate whether the antibody having cytosol-penetrating ability in the antibody constant region can deliver an active protein into cells and whether it has the potential as a therapeutic drug, a cytosol-penetrating antibody-based immunotoxin was designed. FIG. 47 is a schematic diagram illustrating the process of fusing the catalytic domain (DTA) of the DT toxin derived from diphtheria (*Corynebacterium diphtheriae*) to an antibody by trans-splicing. The sequences of proteins required for the expression of the cytosol-penetrating antibody-based immunotoxin are shown in Table 20 below.

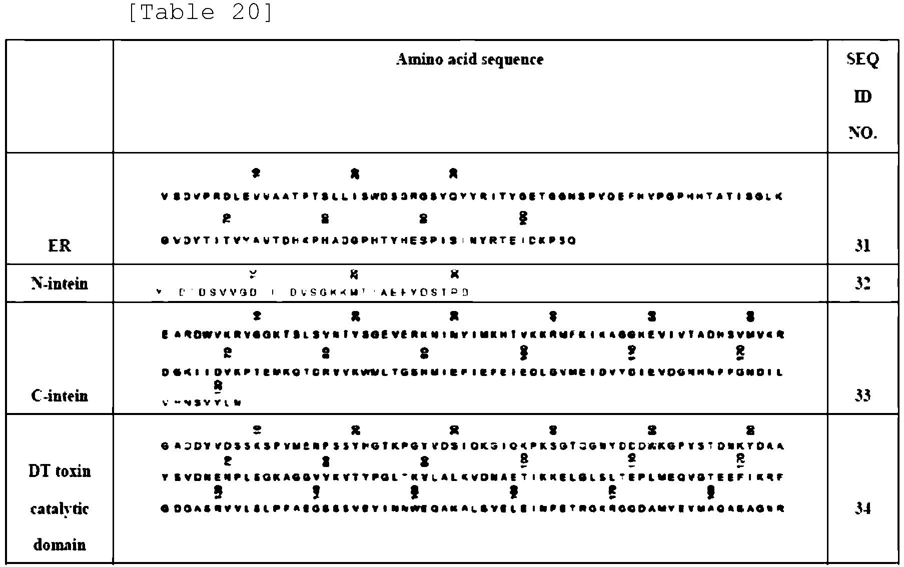

To enable splicing between the toxin to the antibody, N-intein was fused to the C-terminal of the light chain of each of _{ER}CT, _{ER}CT (AAA), and _{ER}2C41 antibodies, and C-intein was fused to the N-terminal of the toxin. Next, the fusion proteins were expressed in and from animal cells and *E. coli,* respectively, and then mixed to induce trans-splicing. Next, the mixture was purified again to obtain toxin-fused antibodies.

To produce antibodies capable of trans-splicing with a toxin, _{ER}CT-intein(N), _{ER}CT(AAA)-intein (N), and _{ER}2C41-intein(N) were constructed. For this purpose, a monobody targeting EGFR, a type of membrane protein receptor overexpressed on the surface of tumor cells/tissue, was fused to the N-terminus of the light chain via a (G₄S)₄ linker, and a cysteine-deficient N-intein required for trans-splicing was fused to the C-terminus of the light chain via a (G₄S)₂ linker. Specifically, to construct a light chain expression vector for producing a monoclonal antibody capable of trans-splicing, a DNA encoding a secretory signal peptide and a DNA encoding an EGFR-targeting monobody (SEQ ID NO: 31), which were fused to the 5' end, a DNA encoding a light chain comprising an antibody light chain variable region (VL, SEQ ID NO: 15 or 16) and an antibody light chain constant region (CL, SEQ ID NO: 17 or 18), and a DNA encoding N-intein (SEQ ID NO: 32), which was fused to the 3' end, were cloned into the pcDNA3.4 vector by NotI/HindIII. To construct a heavy chain expression vector for producing a monoclonal antibody, a DNA, which has a secretory signal peptide-encoding DNA fused to the 5' end and encodes an antibody heavy chain variable region (VH, SEQ ID NO. 13 or 14) and an antibody heavy chain constant region (CH1-hinge-CH2-CH3) containing an antibody CH3 domain (CH3, SEQ ID NO. 1 or 4), was cloned into the pcDNA3.4 vector by NotI/HindIII. The expression of these antibodies in animal cells was performed in HEK293-F cells in the same manner as in Example 3.

Intein(C)-DTA was constructed to produce a toxin that can be trans-spliced with antibodies. Cysteine-deficient C-intein was fused to the N-terminus of the catalytic domain of DT toxin via a (G₄S)₂ linker. Specifically, a DNA encoding the catalytic domain of DT toxin (SEQ ID NO: 34), which was fused to a DNA encoding C-intein (SEQ ID NO: 33) at the 5' end, and a DNA encoding eight histidines, which was fused to the 3' end, were cloned into the pET23m vector by NotI/NcoI. The vector was transformed into *E. coli* SoluBL21(DE3) (Genlantis), and the transformed *E. coli* was cultured in 2X TY medium at 37°C until the OD₆₀₀ reached 0.6. Then, 1 mM IPTG was added thereto, followed by incubation at 20°C for 24 hours. Thereafter, only the cell pellet was collected through centrifugation and resuspended in TBS (50mM Tris, 150mM NaCl), and the cells were disrupted using an ultrasonic homogenizer. Centrifugation was performed again, and the supernatant was filtered through a 0.45-µm filter and then purified using Ni-NTA resin (Thermo Scientific^{™}, 88221).

The purified antibody and toxin were mixed together in TBS (50 mM Tris-HCl, 150 mM NaCl, 1 mM EDTA, pH 7.0) at concentrations of 10 µM and 60 µM, respectively, and incubated at 18°C for 18 hours. After completion of the incubation, purification was performed using protein A resin in the same manner as in Example 3. The purified extracts were re-purified using Ni-NTA resin to obtain only toxin-fused antibodies. The toxin-fused antibodies were named _{ER}CT-DTA, _{ER}CT(AAA)-DTA, and _{ER}2C41-DTA.

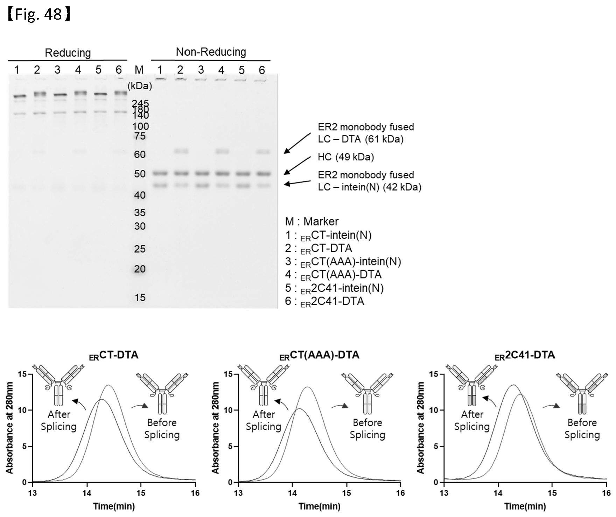

FIG. 48 shows the results of analyzing the antibodies before fusing with the toxin and the antibodies after fusing with the toxin by 12% SDS-PAGE and size exclusion chromatography. It can be confirmed that, after fusing with the toxin, a band corresponding to the light chain fused with the toxin appears at 60 kDa, and after fusing with the toxin, the peak in size exclusion chromatography shifted to the left. This suggests that at least one toxin was fused to the purified immunotoxin.

### Example 39. Evaluation of Cytotoxicity of Immunotoxin Having Cytosol-Penetrating Ability.

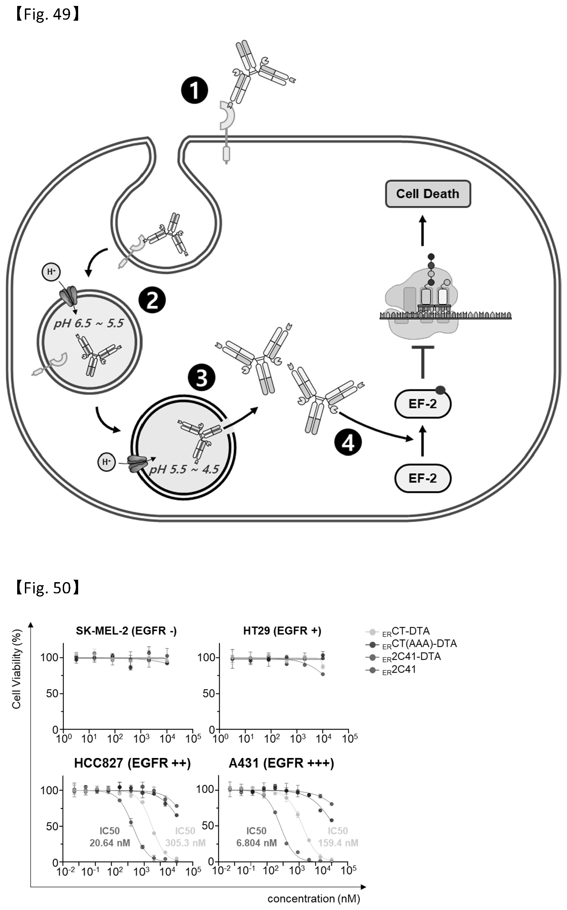

Cytotoxicity was evaluated to examine whether the toxin-fused cytosol-penetrating antibody could deliver an active protein such as a toxin into cell and to assess its potential as a therapeutic protein. FIG. 49 is a schematic diagram showing the process in which an immunotoxin having cytosol-penetrating ability exhibits cytotoxicity. An immunotoxin based on a cytosol-penetrating antibody fused with a monobody that target the cancer cell-overexpressed receptor EGFR binds to and are internalized into cells expressing EGFR, and it dissociates from EGFR in the endosomal weakly acidic environment, and then escapes from the endosome through the endosomal escape motif and moves into the cytosol. After reaching the cytosol, the catalytic domain of DT toxin fused to the antibody inhibits protein synthesis by ADP-ribosylating EF2, resulting in cell death. In order for DT toxin to exhibit cytotoxicity, DT toxin must move into the cytosol, and the catalytic domain of DT toxin alone cannot reach the cytosol. Therefore, if a toxin is fused with a cytosol-penetrating antibody, which exhibits cytosol-penetrating ability only when exposed to the endosomal weakly acidic environment after internalization into a target cell, it can induce target cell-specific cytotoxicity.

FIG. 50 shows the results of evaluating the cytotoxicity of immunotoxins with cytosol-penetrating ability to four types of cells with different EGFR expression levels to evaluate the target cell-specific cytotoxicity of the immunotoxins. SK-MEL-2 (EGFR-), a cell line that does not express EGFR, HT29 (EGFR+), a cell line that expresses EGFR as much as normal cells, HCC827 (EGFR++), a cell line that overexpresses EGFR, and A431 (EGFR+++), a cell line that highly overexpresses EGFR, were each cultured in an RPMI containing 10% FBS and 1% ABAM. 100 µL of 2 × 10³ cells were seeded into each well of a 96-well plate (SPL, 30096) and cultured at 37°C under 5% CO₂ until the cell density reached 60%. After removing the supernatant, each cytosol-penetrating antibody, fused or not fused with a toxin, was serially diluted 5-fold from 10 µM in RPMI containing 10% FBS and 1% ABAM, and the cells were treated with the dilution at 37°C for 72 hours. After removing the supernatant and washing with DPBS, 50 µL of crystal violet solution was added to each well and reacted at room temperature for 20 minutes. After washing 4 times with DBBS, the absorbance was measured at 570 nm, and cell viability was calculated by dividing the absorbance value of each well by the absorbance value of the well treated with only RPMI instead of the antibody.

As a result, it was confirmed that, in the cell lines (SK-MEL-2 and HT29) that do not express EGFR or express EGFR as much as normal cells, almost no cells died even at 10 µM, whereas in the cell lines (HCC827 and A431) that overexpress EGFR, high cytotoxicity was observed. In addition, compared to the previously developed _{ER}CT-based immunotoxin, the _{ER}2C41-based immunotoxin showed about 15- to 20-fold higher cytotoxicity in the EGFR-overexpressing cell lines. On the other hand, it was confirmed that the immunotoxin based on _{ER}CT(AAA) having no cytosol-penetrating ability, and the _{ER}2C41 antibody not fused with the toxin exhibited very weak cytotoxicity in the EGFR-overexpressing cell lines.

### Example 40. Evaluation of Efficacy of Immunotoxin with Cytosol-Penetration Ability in Mice

A mouse experiment was conducted to evaluate the *in vivo* efficacy of an immunotoxin with cytosol-penetrating ability. Cell line A431, which overexpresses EGFR, was exfoliated by trypsin treatment, and a cell suspension of 1 × 10⁸ cells/mL in DPBS was prepared and mixed with an equal amount of Matrigel (Corning, 354234). 100 µL (5 × 10⁶ cells) was injected subcutaneously into the flanks of 4-week-old female nude mice (Balb/c nude) using a 1-mL syringe. After 4 or 5 days, when the tumor volume reached 80 to 100 mm³, the mice were randomly divided into four groups, and (i) vehicle, (ii) _{ER}CT-DTA 20 mg/kg, (iii) _{ER}CT(AAA)-DTA 20 mg/kg, and (iv) _{ER}2C41-DTA 20 mg/kg were injected into the tail veins of the divided groups, respectively, at 3-day intervals. Tumor volume and mouse weight were measured every three days, and when the tumor volume in the vehicle-treated group reached 1,000 mm³, treatment was stopped, the tumor was extracted, and the mass thereof was measured.

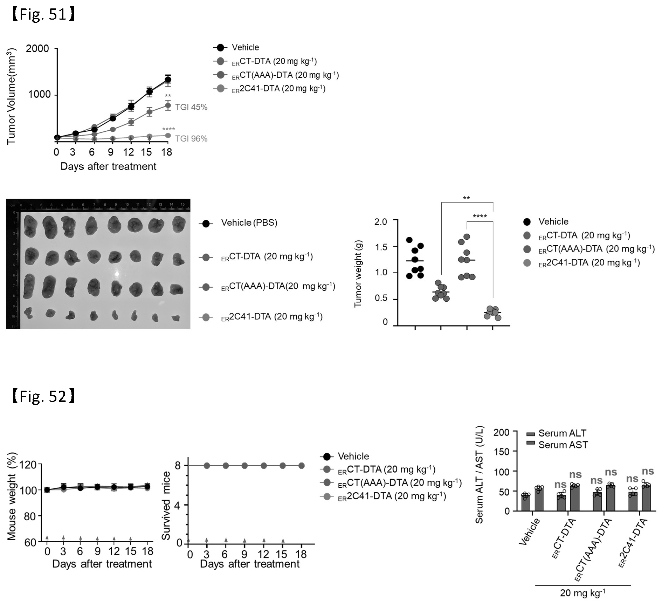

FIG. 51 shows the results of evaluating the volume of the tumor during 6 treatments and the weight of the tumor after the end of treatment. It was confirmed that, in the group treated with _{ER}CT(AAA)-DTA having no cytosol-penetrating ability, there was no tumor growth inhibitory effect, as in the vehicle-treated group, and _{ER}CT-DTA and _{ER}2C41-DTA exhibited tumor growth inhibitory effects of 45% and 96%, respectively. It was confirmed that, due to this difference, the 2C41-based immunotoxin with enhanced cytosol-penetrating ability exhibited a greater tumor growth inhibitory effect, as in Example 39. FIG. 52 shows the results of evaluating the survival rate and weight gain/loss of mice during treatment, and the results of evaluating serum ALT and AST levels after the end of treatment. During the period of six administrations, no mice died or showed a significant change in body weight. In addition, no significant changes in serum ALT and AST levels were observed in any of the experimental groups. Thereby, it was confirmed that the cytosol-penetrating antibody-based immunotoxin containing the endosomal escape motif introduced into the antibody constant region exhibits therapeutic effects against target cancer cells without side effects, and that the enhanced cytosol-penetrating ability can lead to enhanced therapeutic effects.

### Industrial Applicability

The high-efficiency IgG cytosol-penetrating antibody having an endosomal escape motif in the antibody constant region according to the present invention may be actively internalized in living cells, escape from the endosome through its endosomal escape ability, and localize in the cytosol. Thus, the IgG antibody may ultimately be distributed from outside the living cell into the cytosol without the help of any special external protein delivery system or substance.

In the case of previously developed IgG cytosol-penetrating antibodies (e.g., inCT, epCT65, ERCT, etc.) containing WYW endosomal escape motifs in VH and VL, there was a limitation in that the amount of antibody reaching the cytosol was small due to the low endosomal escape ability, so the effect of the antibody in the cytosol was not potent. As a new strategy to overcome this limitation, cytosol-penetrating antibodies containing an endosomal escape motif in the antibody constant region can localize in the cytosol with endosomal escape ability that is about 2 to 3 times higher than that of conventional cytosol-penetrating antibodies. Thereby, the amount of the cytosol-penetrating antibody distributed within the cytosol ultimately significantly increases.

In addition, when various payloads (e.g., proteins, fusion proteins, enzymes, toxins, siRNA, small molecule compounds, peptides, DNA, mRNA, etc.) are fused to the high-efficiency cytosol-penetrating antibody having an endosomal escape motif in the antibody constant region according to the present invention, the loaded payload may be delivered into the cytosol very efficiently and the effect of the payload may be expected.

In addition, the high-efficiency IgG cytosol-penetrating antibody having an endosomal escape motif in the antibody constant region according to the present invention overcomes the limitation that existing cytosol-penetrating antibodies must contain an endosomal escape motif in the VH/VL region. Also, the cytosol-penetrating antibody according to the present invention is highly versatile because it can deliver other antibodies into the cytosol while maintaining the original function (targeting ability for target proteins) of the other antibodies in the VH/VL regions.

In addition, if the VH and VL of the high-efficiency IgG cytosol-penetrating antibody having an endosome escape motif in the antibody constant region according to the present invention are engineered to specifically recognize a target protein in the cytosol, the antibody can actively move from outside the cell into the cytosol and exhibit the effect of inhibiting the activity of and/or activating the target protein in the cytosol.

Furthermore, the cytosol-penetrating antibody with an improved target cell-specific antibody constant region according to the present invention has high production yield and excellent biophysical properties, and retains thermal stability and serum half-life similar to existing wild-type antibodies. Thus, the cytosol-penetrating antibody according to the present invention is easy to develop into a foundational technology for therapeutic drugs and, unlike other non-specific cell-penetrating peptides, can penetrate the cytosol in a target cell/tissue-specific manner. In addition, the cytosol-penetrating antibody according to the present invention may be easily applied to other antibodies because it has a constant region obtained by improving the constant region that is common to general IgG antibodies. Moreover, the cytosol-penetrating antibody according to the present invention may be used as a carrier to deliver various types of active substances into the cytosol of cells, and exhibits strong cytosol-penetrating ability only for cells that overexpress a target receptor, and thus has almost no toxicity in normal cells with low target receptor expression levels even when a highly toxic payload is fused thereto, suggesting that it can be used in pharmaceutical compositions for the treatment and prevention of various diseases in the future.

### Sequence List Test

Electronic file attached

## Claims

1. A cytosol-penetrating antibody or a fragment thereof containing an endosomal escape motif in a heavy chain constant region and/or light chain constant region thereof.

2. The cytosol-penetrating antibody or fragment thereof according to claim 1, wherein the endosomal escape motif is contained in a CH3 domain of the heavy chain constant region and/or a CL domain of the light chain constant region.

3. The cytosol-penetrating antibody or fragment thereof according to claim 1, wherein the endosomal escape motif is located in an AB loop, EF loop and C-terminus of the heavy chain constant region CH3 domain of SEQ ID NO: 1.

4. The cytosol-penetrating antibody or fragment thereof according to claim 3, wherein the endosomal escape motif comprises the following endosomal escape motif in which amino acid residue numbers follow EU numbering:
AB loop: ³⁵³PS-X-X-E-X-X-K-X-X³⁶²
wherein X in the AB loop is selected from the group consisting of arginine (R), tryptophan (W), glutamic acid (E), alanine (A), asparagine (N), and glutamine (Q);
EF loop: ⁴¹¹TV-X-X-X-RW-XX-GNVF⁴²³
wherein X in the EF loop is selected from the group consisting of arginine (R), tryptophan (W), glutamic acid (E), alanine (A), asparagine (N), and glutamine (Q); and
C-terminus: ⁴³⁹X-SLS-X-SPGK⁴⁴⁷
wherein X in the C terminus is selected from the group consisting of arginine (R), tryptophan (W), glutamic acid (E), alanine (A), asparagine (N), and glutamine (Q).

5. The cytosol-penetrating antibody or fragment thereof according to claim 1, wherein the endosomal escape motif is located in an AB loop, EF loop and C-terminus of the light chain constant region CL domain of SEQ ID NO. 17.

6. The cytosol-penetrating antibody or fragment thereof according to claim 5, wherein the endosomal escape motif comprises the following endosomal escape motif in which amino acid residue numbers follow EU numbering:
AB loop: ¹²⁰PS-X-X-Q-X-X-S-X-X¹²⁹
wherein X in the AB loop is selected from the group consisting of arginine (R), tryptophan (W), glutamic acid (E), alanine (A), asparagine (N), and glutamine (Q);
EF loop: ¹⁸⁰TL-X-X-X-DY-XX-HKVY¹⁹²
wherein X in the EF loop is selected from the group consisting of arginine (R), tryptophan (W), glutamic acid (E), alanine (A), asparagine (N), and glutamine (Q); and
C-terminus: ²⁰⁷X-SFN-X-GEC²¹⁴
wherein X in the C-terminus is selected from the group consisting of arginine (R), tryptophan (W), glutamic acid (E), alanine (A), asparagine (N), and glutamine (Q).

7. The cytosol-penetrating antibody or fragment thereof according to claim 1, wherein the endosomal escape motif comprises arginine (R), tryptophan (W), and glutamic acid (E), wherein arginine (R), tryptophan (W), and glutamic acid (E) are structurally clustered.

8. The cytosol-penetrating antibody or fragment thereof according to claim 1, wherein the heavy chain constant region CH3 domain containing the endosomal escape motif comprises one or more sequences selected from the group consisting of SEQ ID NOs: 2 to 12.

9. The cytosol-penetrating antibody or fragment thereof according to claim 1, wherein the light chain constant region CL domain containing the endosomal escape motif comprises the sequence of SEQ ID NO: 18 or 19.

10. The cytosol-penetrating antibody or fragment thereof according to claim 1, wherein the endosomal escape motif is contained in IgG1, IgG2, IgG3, IgG4, IgM, IgE, IgA1, IgA2, IgD, or kappa (κ) and lambda (λ) light chains.

11. The cytosol-penetrating antibody or fragment thereof according to claim 1, comprising (1) or (2) below, or comprising (1) and (2) below:
(1) a CH1-CL pair comprising a heavy chain constant region CH1 domain and a light chain constant region CL domain containing the endosomal escape motif;
(2) a constant region CH3-CH3 pair containing the endosomal escape motif in the heavy chain constant region CH3 domain; and/or
(3) a constant region CH3-CH3 pair in which a constant region CH1-CL pair is replaced with the constant region CH3-CH3 pair of (2) and which contains the endosomal escape motif in the heavy chain constant region CH3 domain.

12. The cytosol-penetrating antibody or fragment thereof according to claim 11, wherein the heavy chain constant region CH1 and light chain constant region CL pair is replaced with a CH3-CH3 pair containing the endosomal escape motif, so that six CH3 domains contain the endosomal escape motif, and the CH3-CH3 pair replacing the CH1-CL pair is a heterodimer.

13. The cytosol-penetrating antibody or fragment thereof according to claim 12, wherein the antibody comprising the CH3-CH3 pair replacing the CH1-CL pair comprises one or more sequences selected from the group consisting of SEQ ID NOs: 23 to 26.

14. A polynucleotide encoding the cytosol-penetrating antibody or fragment thereof according to any one of claims 1 to 13.

15. A recombinant expression vector comprising the polynucleotide of claim 14.

16. An isolated host cell comprising the recombinant expression vector of claim 15.

17. A composition for delivering an active substance into cytosol, comprising the cytosol-penetrating antibody or fragment thereof according to any one of claims 1 to 13.

18. The composition according to claim 17, wherein the active substance is one or more selected from the group consisting of peptides, proteins, toxins, antibodies, antibody fragments, RNA, siRNA, DNA, small molecule drugs, nanoparticles, and liposomes.
